# EUROPÄISCHE PATENTSCHRIFT

(11) **EP 2 780 325 B1**
(45) Veröffentlichungstag und Bekanntmachung des Hinweises auf die Patenterteilung: **03.02.2016**
(21) Anmeldenummer: 12774933.1
(22) Anmeldetag: 18.10.2012
(51) Int. Cl.: C07D 221/20, C07D 491/20, C07D 495/10, C07D 519/00, C07D 491/107, C07F 5/02, C07F 9/6561, C07F 9/6568, C09K 11/06, H01L 51/50

(54) **SPIRO-DIHYDROACRIDINDERIVATE UND IHRE VERWENDUNG ALS MATERIALIEN FÜR ORGANISCHE ELEKTROLUMINESZENZVORRICHTUNGEN**
SPIRO-DIHYDROACRIDINE DERIVATIVES AND THEIR USE AS MATERIALS FOR ORGANIC ELECTROLUMINESCENT DEVICES
DÉRIVÉS SPIRO-DIHYDROACRIDINE ET LEUR UTILISATION EN TANT QUE MATÉRIAUX POUR DES DISPOSITIFS ÉLECTROLUMINESCENTS ORGANIQUES

(30) Priorität: 17.11.2011 EP 11009127
(43) Veröffentlichungstag der Anmeldung: 24.09.2014
(73) Patentinhaber: Merck Patent GmbH, 64293 Darmstadt (DE)
(72) Erfinder: STOESSEL, Philipp, 60487 Frankfurt am Main (DE); MONTENEGRO, Elvira, 69469 Weinheim (DE); BREUNING, Esther, 64372 Ober-Ramstadt (DE)
(86) Internationale Anmeldenummer: PCT/EP2012/004362
(87) Internationale Veröffentlichungsnummer: WO 2013/083216

(56) Entgegenhaltungen:
- US-A1- 2010 019 658
- DATABASE CA [Online] CHEMICAL ABSTRACTS SERVICE, COLUMBUS, OHIO, US; 2011, JE, JONG TAE ET AL: "Spiro compound as an electroluminescent material for organic electroluminescent device", XP002690464, gefunden im STN Database accession no. 2011:1305143 -& KR 2011 111 093 A (SFC LTD., S. KOREA) 10. Oktober 2011 (2011-10-10)
- DATABASE CA [Online] CHEMICAL ABSTRACTS SERVICE, COLUMBUS, OHIO, US; 2011, JE, JONG TAE ET AL: "Heterocyclic compound as an electroluminescent material for organic electroluminescent device", XP002690465, gefunden im STN Database accession no. 2011:1332787 -& KR 2011 113 469 A (SFC LTD., S. KOREA) 17. Oktober 2011 (2011-10-17)
- DATABASE CA [Online] CHEMICAL ABSTRACTS SERVICE, COLUMBUS, OHIO, US; 2011, JE, JONG TAE ET AL: "Spiro compound as an electroluminescent material for OLED device", XP002690466, gefunden im STN Database accession no. 2011:1289396 -& KR 2011 110 508 A (SFC LTD., S. KOREA) 7. Oktober 2011 (2011-10-07)
- DATABASE CA [Online] CHEMICAL ABSTRACTS SERVICE, COLUMBUS, OHIO, US; 2000, NAGO, HIRONOBU ET AL: "Photochromic chromene having spiro rings, photochromic materials, and photochromic optical materials", XP002690467, gefunden im STN Database accession no. 2000:873310 -& JP 2000 344761 A (TOKUYAMA CORP., JAPAN) 12. Dezember 2000 (2000-12-12)
- DATABASE CA [Online] CHEMICAL ABSTRACTS SERVICE, COLUMBUS, OHIO, US; 2011, JE, JONG TAE ET AL: "Spiro compound as an electroluminescent material for organic electroluminescent element", XP002690468, gefunden im STN Database accession no. 2011:1419577 -& KR 2011 120 075 A (SFC LTD., S. KOREA) 3. November 2011 (2011-11-03)
- DATABASE CA [Online] CHEMICAL ABSTRACTS SERVICE, COLUMBUS, OHIO, US; 2002, ENOKIDA, TOSHIO ET AL: "Spiro compound for organic electroluminescent material and component", XP002690469, gefunden im STN Database accession no. 2002:708993 -& JP 2002 265938 A (TOYO INK MFG. CO., LTD., JAPAN) 18. September 2002 (2002-09-18)
- DATABASE CA [Online] CHEMICAL ABSTRACTS SERVICE, COLUMBUS, OHIO, US; 2003, LI, BIN ET AL: "Bispirocyclic ring derivatives and organic electroluminescent devices using them", XP002690470, gefunden im STN Database accession no. 2003:74514 -& CN 1 338 499 A (QINGHUA UNIV., PEOP. REP. CHINA; TSINGHUA UNIV.) 6. März 2002 (2002-03-06)

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer. Außerdem ist es wünschenswert, dass sich die verwendeten Materialien in hoher Ausbeute und Reinheit synthetisieren lassen.

Die Eigenschaften von phosphoreszierenden OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Auch für fluoreszierende OLEDs gibt es bei diesen Materialien sowie für Emitter und Matrixmaterialien noch Verbesserungsbedarf.

Gemäß dem Stand der Technik werden unter anderem Carbazolderivate, z. B. gemäß WO 2005/039246, US 2005/0069729, JP 2004/288381, EP 1205527 oder WO 2008/086851, Indolocarbazolderivate, z. B. gemäß WO 2007/063754 oder WO 2008/056746, Indenocarbazolderivate, z. B. gemäß WO 2010/136109, oder Dihydroacridinderivate, z. B. gemäß US 2010/0019658, als Matrixmaterialien für phosphoreszierende Emitter in organischen Elektrolumineszenzvorrichtungen eingesetzt. Hier sind weitere Verbesserungen wünschenswert, ebenso wie in Bezug auf die Effizienz, die Lebensdauer und die thermische Stabilität der Materialien.

Aufgabe der vorliegenden Erfindung ist die Bereitstellung von Verbindungen, welche sich für den Einsatz in einer fluoreszierenden oder phosphoreszierenden OLED eignen, insbesondere als Matrixmaterial oder als Lochtransport-/ Elektronenblockiermaterial bzw. Exzitonenblockiermaterial, aber auch als Lochblockiermaterial, Matrix für fluoreszierende Emitter oder als fluoreszierender Emitter. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit bei der Herstellung von OLEDs zu ermöglichen.

Überraschend wurde gefunden, dass bestimmte, unten näher beschriebene Verbindungen diese Aufgabe lösen und zu Verbesserungen der organischen Elektrolumineszenzvorrichtung führen. Dabei betreffen die Verbesserungen insbesondere die Lebensdauer, die Effizienz und/oder die Betriebsspannung. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung gemäß Formel (1), (2) oder (3) 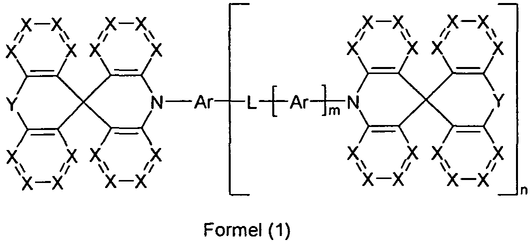 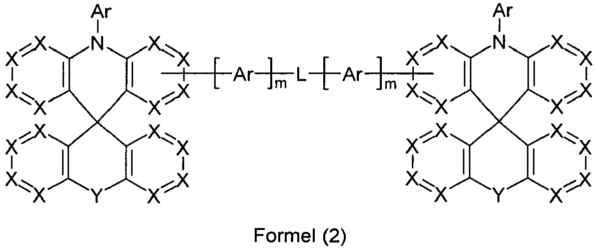 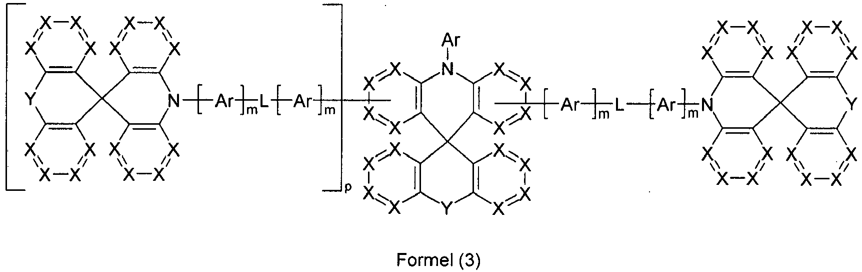 wobei für die verwendeten Symbole und Indizes gilt:
- Y: ist bei jedem Auftreten gleich oder verschieden O oder S;
- X: ist bei jedem Auftreten gleich oder verschieden CR² oder N; oder zwei benachbarte X stehen für S, O oder NR², so dass ein Fünfring entsteht; oder zwei benachbarte X stehen für eine Gruppe der folgenden Formel (4) oder (5), 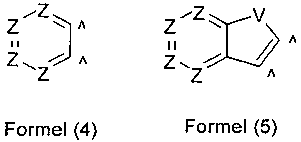 wobei ^ die entsprechenden benachbarten Gruppen X in der Formel (1), (2) oder (3) andeutet;
dabei steht X für C, wenn an diese Gruppe X in Formel (2) bzw. (3) eine Gruppe Ar bzw. L gebunden ist;
- V: ist bei jedem Auftreten gleich oder verschieden C(R²)₂, NR², O oder S;
- Z: ist bei jedem Auftreten gleich oder verschieden CR² oder N;
- Ar: ist bei jedem Auftreten gleich oder verschieden ein aromatisches oder heteroaromatisches Ringsystem mit 5 bis 40 aromatischen Ringatomen, das mit einem oder mehreren Resten R³ substituiert sein kann; dabei können die Gruppe Ar und die benachbarte Gruppe X, die in diesem Fall für C steht, auch durch eine Einfachbindung oder eine bivalente Gruppe, ausgewählt aus C(R³)₂, NR³, O oder S, miteinander verbrückt sein;
- L: ist bei jedem Auftreten gleich oder verschieden eine Einfachbindung oder eine bivalente Gruppe;
- R¹, R², R³: ist bei jedem Auftreten gleich oder verschieden ausgewählt aus der Gruppe bestehend aus H, D, F, Cl, Br, I, CN, NO₂, N(Ar¹)₂, N(R⁴)₂, C(=O)Ar¹, C(=O)R⁴, P(=O)(Ar¹)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R⁴ substituiert sein kann, wobei eine oder mehrere nicht-benachbarte CH₂-Gruppen durch R⁴C=CR⁴, C=C, Si(R⁴)₂, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60, aromatischen Ringatomen, das jeweils mit einem oder mehreren Resten R⁴ substituiert sein kann, einer Aryloxy- oder Heteroaryloxygruppe mit 5 bis 60 aromatischen Ringatomen, die mit einem oder mehreren Resten R⁴ substituiert sein kann, wobei optional zwei oder mehr benachbarte Substituenten R¹ bzw. R³ ein monocyclisches oder polycyclisches, aliphatisches Ringsystem bzw. R² ein monocyclisches oder polycyclisches, aliphatisches, aromatisches oder heteroaromatisches Ringsystem bilden können, das mit einem oder mehreren Resten R⁴ substituiert sein kann;
- R⁴: ist bei jedem Auftreten gleich oder verschieden ausgewählt aus der Gruppe bestehend aus H, D, F, Cl, Br, I, CN, NO₂, N(Ar¹)₂, N(R⁵)₂, C(=O)Ar¹, C(=O)R⁵, P(=O)(Ar¹)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R⁵ substituiert sein kann, wobei eine oder mehrere nicht-benachbarte CH₂-Gruppen durch R⁵C=CR⁵, C=C, Si(R⁵)₂, C=O, C=S, C=NR⁵, P(=O)(R⁵), SO, SO₂, NR⁵, O, S oder CONR⁵ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R⁵ substituiert sein kann, einer Aryloxy- oder Heteroaryloxygruppe mit 5 bis 60 aromatischen Ringatomen, die mit einem oder mehreren Resten R⁵ substituiert sein kann, oder einer Kombination dieser Systeme, wobei optional zwei oder mehr benachbarte Substituenten R⁴ ein monocyclisches oder polycyclisches, aliphatisches Ringsystem bilden können, das mit einem oder mehreren Resten R⁵ substituiert sein kann;
- Ar¹: ist bei jedem Auftreten gleich oder verschieden ein aromatisches oder heteroaromatisches Ringsystem mit 5-30 aromatischen Ringatomen, das mit einem oder mehreren nicht-aromatischen Resten R⁵ substituiert sein kann; dabei können zwei Reste Ar¹, welche an dasselbe N-Atom oder P-Atom binden, auch durch eine Einfachbindung oder eine Brücke, ausgewählt aus N(R⁵), C(R⁵)₂, O oder S, miteinander verbrückt sein;
- R⁵: ist bei jedem Auftreten gleich oder verschieden ausgewählt aus der Gruppe bestehend aus H, D, F, CN, einem aliphatischen Kohlenwasserstoffrest mit 1 bis 20 C-Atomen, einem aromatischen oder heteroaromatischen Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R⁵ miteinander ein mono- oder polycyclisches, aliphatisches Ringsystem bilden können;
- m: ist bei jedem Auftreten gleich oder verschieden 0 oder 1;
- n: ist 0, 1, 2, 3, 4 oder 5;
- p: ist 0 oder 1.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme. Wie oben beschrieben, können die Gruppe Ar und eine benachbarte Gruppe X auch miteinander verbrückt sein. Dabei wird unter einer "benach-barten Gruppe X" eine Gruppe X verstanden, die von der Gruppe Ar durch ein Stickstoff- und ein Kohlenstoffatom getrennt ist, wie im Folgenden schematisch dargestellt, wobei diese Gruppe X für C steht, wenn dieses X und die Gruppe Ar verbrückt sind:

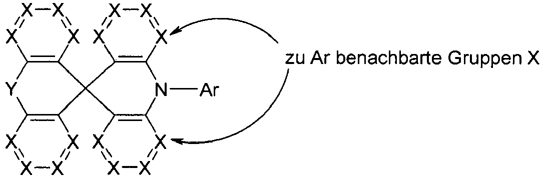

Die Ringbildung ist im Folgenden exemplarisch an Verbindungen der Formel (1) mit n = 0 dargestellt, wobei die Ringbildung ganz analog mit den anderen erfindungsgemäßen Verbindungen erfolgen kann: 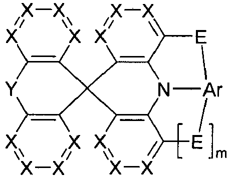 wobei E für eine Einfachbindung, C(R³)₂, NR³, O oder S steht und die weiteren verwendeten Symbole und Indizes die oben genannten Bedeutungen aufweisen.

In einer bevorzugten Ausführungsform der Erfindung steht Y für O.

Bevorzugt sind weiterhin alle Gruppen Y gleich gewählt.

In einer weiteren bevorzugten Ausführungsform der Erfindung steht X gleich oder verschieden bei jedem Auftreten für CR² oder N, wobei maximal eine Gruppe X pro Cyclus für N steht; oder zwei benachbarte Gruppen X stehen für eine Gruppe der Formel (4) oder (5), insbesondere Formel (5), wobei Z gleich oder verschieden bei jedem Auftreten für CR² steht und V gleich oder verschieden bei jedem Auftreten für NR² oder C(R²)₂ steht.

Besonders bevorzugt steht X gleich oder verschieden bei jedem Auftreten für CR².

Bevorzugt sind weiterhin Verbindungen der oben genannten Formel (1) sowie der folgenden Verbindungen (2a) oder (3a),

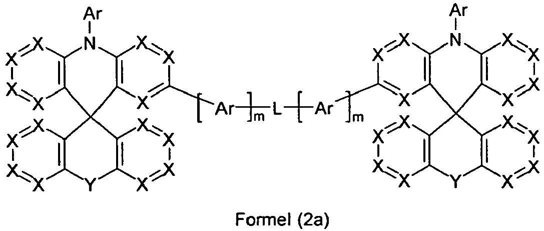

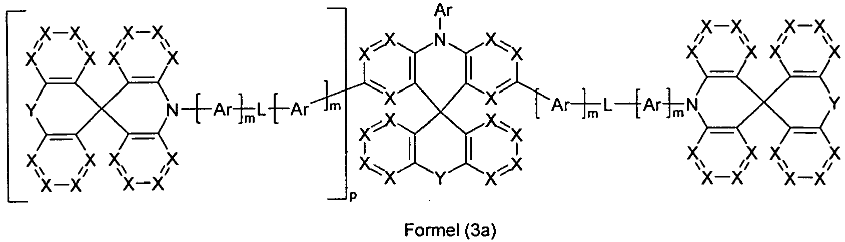

Besonders bevorzugt sind Verbindungen der oben genannten Formel (1), insbesondere mit n = 0.

In einer bevorzugten Ausführungsform der Erfindung steht die Gruppe L gleich oder verschieden bei jedem Auftreten für eine geradkettige Alkylen-, Alkyliden-, Alkylenoxy- oder Thioalkylenoxygruppe mit 1 bis 10 C-Atomen oder eine verzweigte oder cyclische Alkylen-, Alkyliden-, Alkylenoxy- oder Thioalkylenoxygruppe mit 3 bis 40 C-Atomen oder eine Alkenylen- oder Alkinylengruppe mit 2 bis 40 C-Atomen, die mit jeweils einem oder mehreren Resten R⁴ substituiert sein kann, wobei eine oder mehrere nicht benachbarte CH₂-Gruppen durch -R⁴C=CR⁴-, -C=C-, Si(R⁴)₂, C=O, C=NR⁴, P(=O)R⁴, S=O, SO₂, -O-, -S- oder -CONR⁴- ersetzt sein können und wobei ein oder mehrere H-Atome durch D, F, Cl, Br, I, CN oder NO₂ ersetzt sein können oder P(R⁴), P(=O)(R⁴), N(Ar¹); oder L ist eine Einfachbindung.

In einer besonders bevorzugten Ausführungsform der Erfindung steht die Gruppe L für eine Einfachbindung.

Bevorzugte Ausführungsformen der Verbindungen gemäß Formel (1), (2) und (3) sind die Verbindungen der folgenden Formeln (6), (7) und (8), 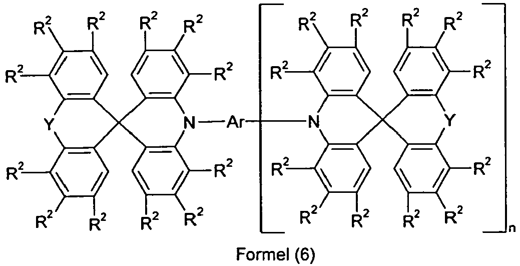 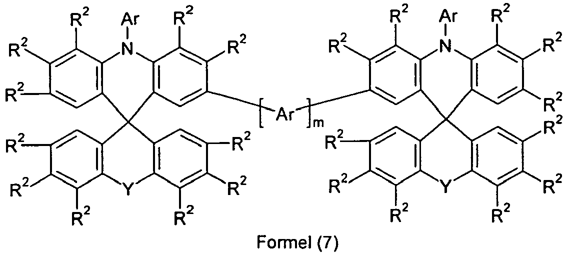 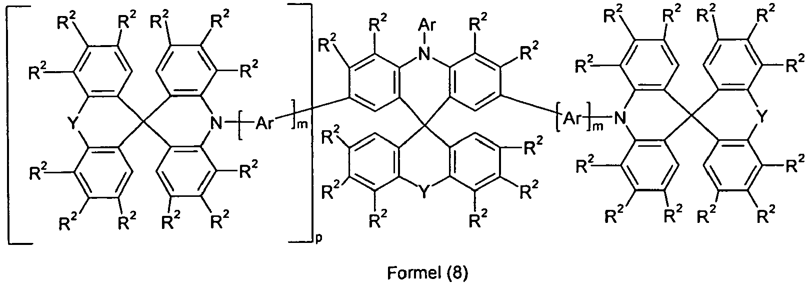 wobei die verwendeten Symbole und Indizes die oben genannten Bedeutungen aufweisen und Y bevorzugt für O steht.

Weiterhin können Gruppen der Formel (4) oder (5) ankondensiert sein, wie im Folgenden exemplarisch durch die Formeln (9), (10), (11) und (12) mit ankondensierten Gruppen der Formel (5) dargestellt,

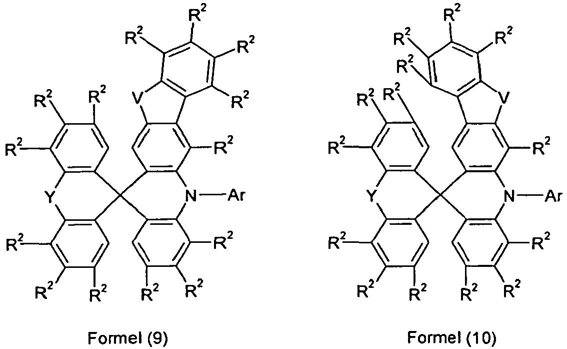

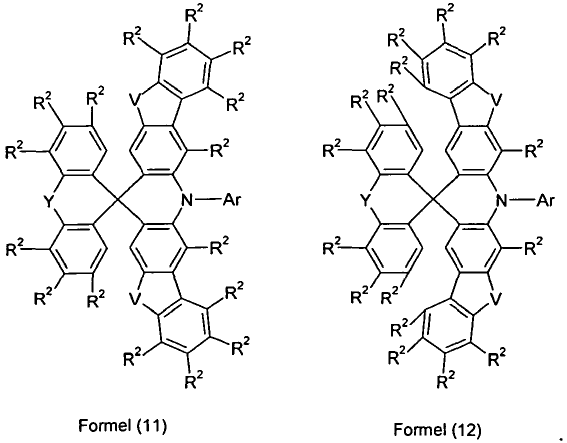

Besonders bevorzugt sind die Verbindungen der folgenden Formeln (6a) bis (12a), 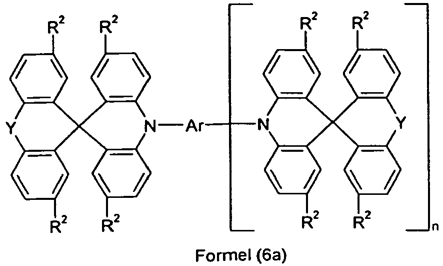 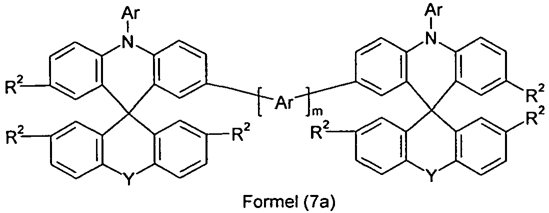 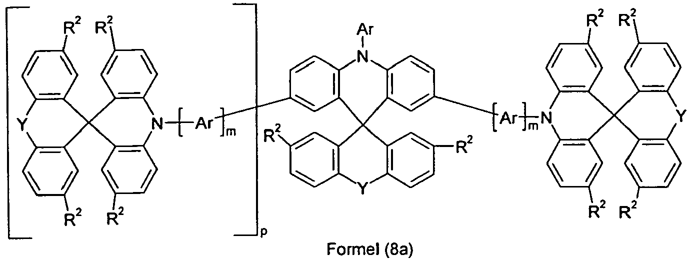 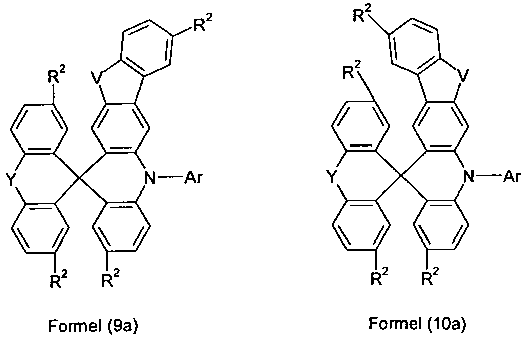 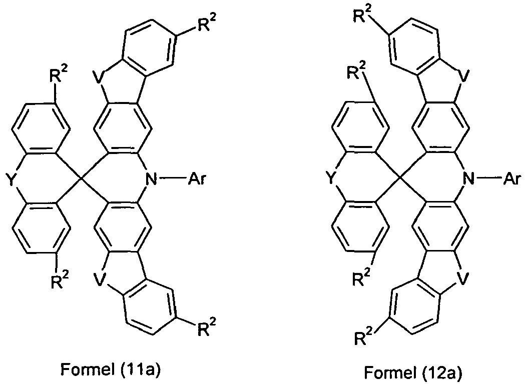 wobei die verwendeten Symbole und Indizes die oben genannten Bedeutungen aufweisen und Y bevorzugt für O steht.

Ganz besonders bevorzugt sind die Verbindungen der folgenden Formeln (6b) bis (12b), 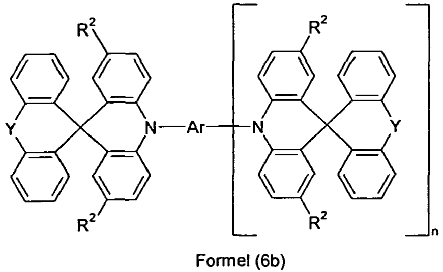 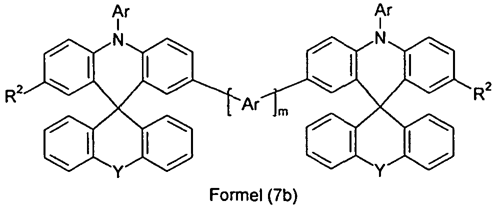 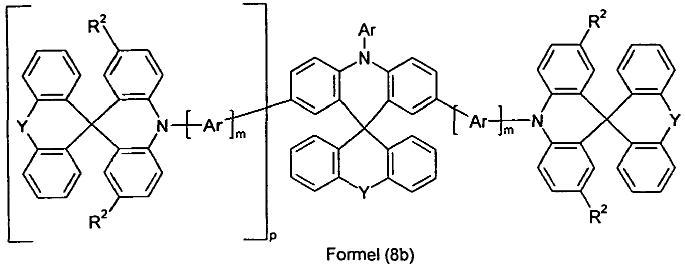 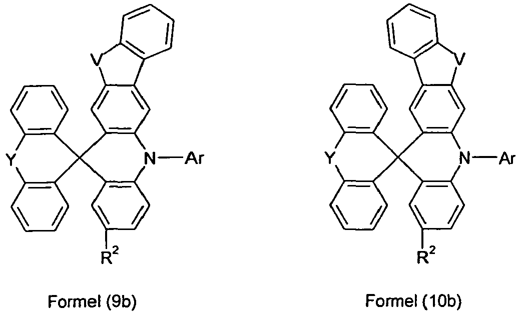 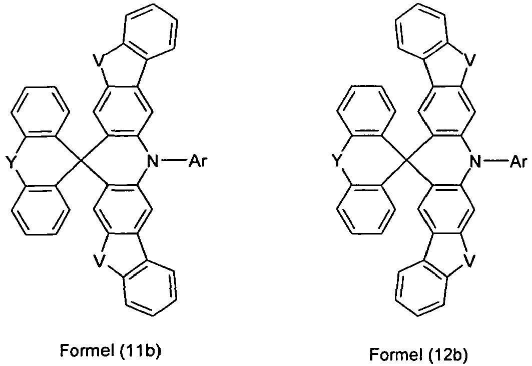 wobei die verwendeten Symbole und Indizes die oben genannten Bedeutungen aufweisen und Y bevorzugt für O steht.

Insbesondere bevorzugt sind die Verbindungen der Formel (6b), in denen der Index n = 0 oder 1 ist.

In einer bevorzugten Ausführungsform der Erfindung sind R¹, R² und R³ in den oben genannten Formeln gleich oder verschieden bei jedem Auftreten ausgewählt aus der Gruppe bestehend aus H, D, F, Cl, Br, CN, N(Ar¹)₂, C(=O)Ar¹, einer geradkettigen Alkyl- oder Alkoxygruppe mit 1 bis 10 C-Atomen, einer verzweigten oder cyclischen Alkyl- oder Alkoxygruppe mit 3 bis 10 C-Atomen, einer Alkenyl- oder Alkinylgruppe mit 2 bis 10 C-Atomen, die jeweils mit einem oder mehreren Resten R⁴ substituiert sein kann, wobei eine oder mehrere nicht-benachbarte CH₂-Gruppen durch O ersetzt sein können und wobei ein oder mehrere H-Atome durch D oder F ersetzt sein können, und einem aromatischen oder heteroaromatischen Ringsystem mit 5 bis 30 aromatischen Ringatomen, das jeweils mit einem oder mehreren Resten R⁴ substituiert sein kann.

In einer besonders bevorzugten Ausführungsform der Erfindung sind R¹, R² und R³ in den oben genannten Formeln gleich oder verschieden bei jedem Auftreten ausgewählt aus der Gruppe bestehend aus H, D, F, Cl, Br, CN, einer geradkettigen Alkylgruppe mit 1 bis 5 C-Atomen, einer verzweigten oder cyclischen Alkylgruppe mit 3 bis 10 C-Atomen, wobei ein oder mehrere H-Atome durch D oder F ersetzt sein können, und einem aromatischen oder heteroaromatischen Ringsystem mit 5 bis 18 aromatischen Ringatomen, das jeweils mit einem oder mehreren Resten R⁴ substituiert sein kann.

Bevorzugt umfasst R² keine Heteroarylgruppe mit 5 Ringatomen, keine Alkenylgruppe und keine Alkinylgruppe.

R² ist bevorzugt gleich oder verschieden bei jedem Auftreten ausgewählt aus der Gruppe bestehend aus H, D, F, Cl, Br, CN, einer geradkettigen Alkylgruppe mit 1 bis 5 C-Atomen, einer verzweigten oder cyclischen Alkylgruppe mit 3 bis 10 C-Atomen, wobei ein oder mehrere H-Atome durch D oder F ersetzt sein können, und einem aromatischen Ringsystem mit 5 bis 18 aromatischen Ringatomen, das jeweils mit einem oder mehreren Resten R⁴ substituiert sein kann, wobei R⁴ bei jedem Auftreten gleich oder verschieden ausgewählt ist aus der Gruppe bestehend aus H, D, F, Cl, Br, I, CN, NO₂, N(Ar¹)₂, N(R⁵)₂, C(=O)Ar¹, C(=O)R⁵, P(=O)(Ar¹)₂, einer geradkettigen Alkyl-, Alkoxy- oder Thioalkylgruppe mit 1 bis 5 C-Atomen, einer verzweigten oder cyclischen Alkyl-, Alkoxy- oder Thioalkylgruppe mit 3 bis 10 C-Atomen, wobei ein oder mehrere H-Atome durch D, F, Cl, Br oder CN ersetzt sein können, und einem aromatischen oder heteroaromatischen Ringsystem mit 5 bis 18 aromatischen Ringatomen, das jeweils mit einem oder mehreren Resten R⁵ substituiert sein kann.

Dabei haben für Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Bevorzugte Gruppen Ar sind ausgewählt aus aromatischen oder heteroaromatischen Ringsystemen mit 5 bis 24 aromatischen Ringatomen, welche jeweils durch einen oder mehrere Reste R³ substituiert sein können. Besonders bevorzugte Gruppen Ar sind ausgewählt aus Benzol, ortho-, meta- oder para-Biphenyl, ortho-, meta-, para- oder verzweigtes Terphenyl, ortho-, meta- para- oder verzweigtes Quaterphenyl, 1- oder 2-Naphthyl, Pyrrol, Furan, Thiophen, Indol, Benzofuran, Benzothiophen, Carbazol, Dibenzofuran, Dibenzothiophen, Pyridin, Pyrimidin, Pyrazin, Pyridazin, Triazin, Anthracen, Phenanthren, Pyren, Benzanthracen oder Kombinationen aus zwei oder drei dieser Gruppen, welche jeweils mit einem oder mehreren Resten R³ substituiert sein können.

Wenn R¹, R² bzw. R³ für ein aromatisches oder heteroaromatisches Ringsystem stehen, ist dieses gleich oder verschieden bei jedem Auftreten bevorzugt aus denselben Gruppen ausgewählt, wie oben als bevorzugte Gruppen für Ar angegeben.

Wenn die Verbindungen der Formel (1), (2) oder (3) bzw. die bevorzugten Ausführungsformen als Elektronentransportmaterial verwendet werden, ist es bevorzugt, wenn mindestens einer der Reste R¹, R² und/oder Ar für ein elektronenarmes heteroaromatisches Ringsystem steht. Elektronenarme Heteroaromaten sind erfindungsgemäß Fünfringheteroaromaten mit mindestens zwei Heteroatomen oder Sechsringheteroaromaten, an die jeweils noch ein oder mehrere aromatische oder heteroaromatische Gruppen ankondensiert sein können, zum Beispiel substituierte oder unsubstituierte Imidazole, Pyrazole, Thiazole, Oxazole, Oxadiazole, Triazole, Pyridine, Pyrazine, Pyrimidine, Pyridazine, Triazine, Benzimidazole, etc., insbesondere solche, wie sie im Folgenden aufgeführt sind.

Wenn die erfindungsgemäße Verbindung als Matrixmaterial für einen phosphoreszierenden Emitter oder als Elektronentransportmaterial eingesetzt wird, ist bevorzugt mindestens ein Substituent R¹, R² und/oder R³ oder eine monovalente Gruppe Ar eine elektronenarme Gruppe, insbesondere ausgewählt aus Strukturen gemäß den folgenden Formeln (13) bis (16) für R¹ bis R³ oder den Formeln (13), (15) oder (16) für Ar, 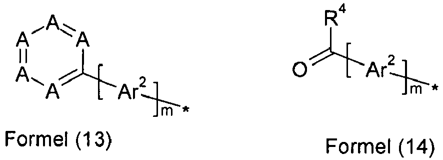 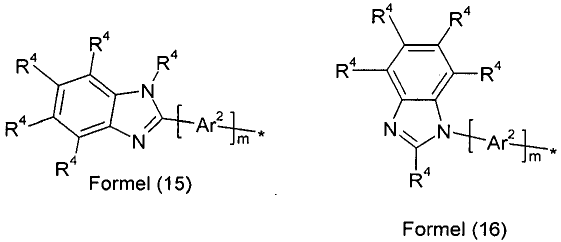 und/oder mindestens eine bivalente bzw. trivalente Gruppe Ar steht bevorzugt für eine Gruppe der folgenden Formeln (17) bis (19), 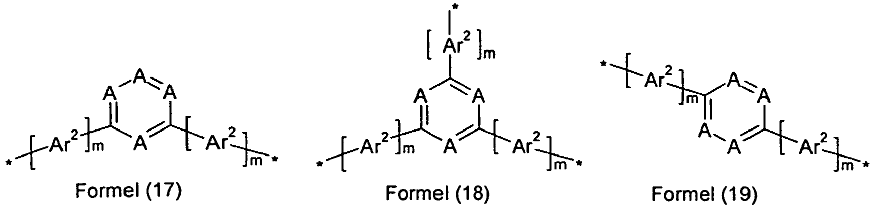 wobei R³, R⁴ und m die oben genannte Bedeutung haben, * die Position der Bindung der Gruppe gemäß Formel (13) bis (19) andeutet und weiterhin gilt:
- A: ist bei jedem Auftreten gleich oder verschieden CR⁴ oder N, mit der Maßgabe, dass eine, zwei oder drei Gruppen A für N stehen;
- Ar²: ist gleich oder verschieden bei jedem Auftreten ein bivalentes aromatisches oder heteroaromatisches Ringsystem mit 5 bis 16 C-Atomen, welches durch einen oder mehrere Reste R⁴ substituiert sein kann;
wobei R⁴ durch R³ zu ersetzen ist, wenn die Gruppen der Formeln (13), (15) und (16) für Ar stehen, sowie in den Fällen der Formeln (17) bis (19).

In einer besonders bevorzugten Ausführungsform der Erfindung steht mindestens ein Substituent R¹, R², R³ oder Ar für eine Gruppe der oben genannten Formel (13), und/oder mindestens eine Gruppe Ar steht für eine Gruppe der oben genannten Formeln (17) bis (19), wobei jeweils zwei oder drei Symbole A für N stehen und die anderen Symbole A für CR⁴ stehen, wobei R³ durch R⁴ zu ersetzen ist, wenn die Gruppen der Formeln (13), (15) und (16) für Ar stehen, sowie in den Fällen der Formeln (17) bis (19).

Besonders bevorzugte Gruppen R¹, R², R³ oder Ar sind daher die Gruppen der folgenden Formeln (20) bis (26), und besonders bevorzugte Gruppen Ar sind die Gruppen der folgenden Formeln (27) bis (34), 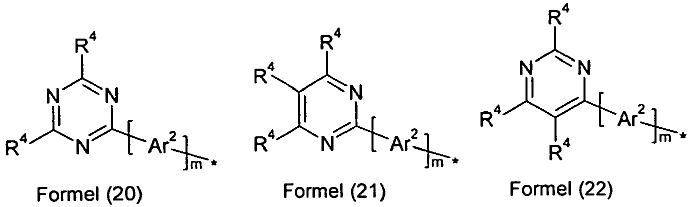 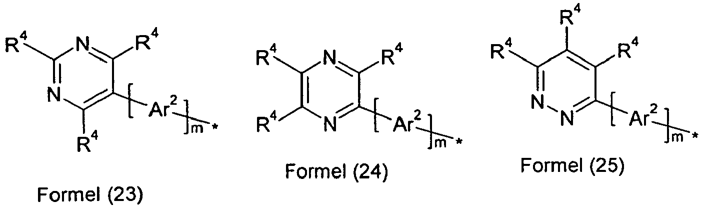 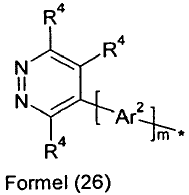 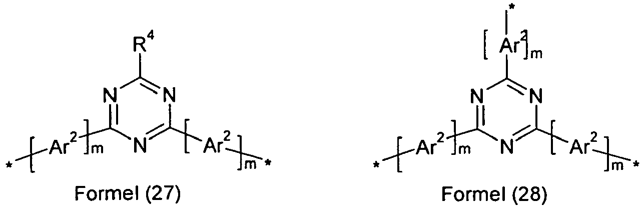 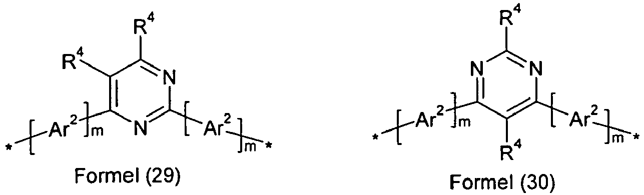 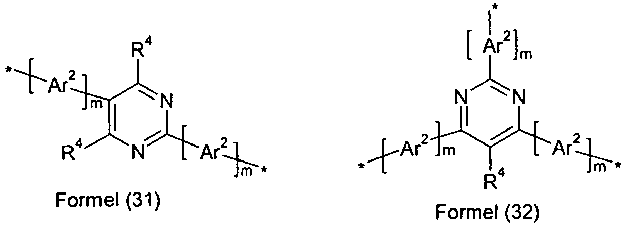 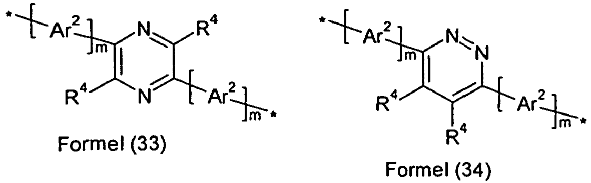 wobei die verwendeten Symbole und Indizes die oben genannten Bedeutungen aufweisen, und wobei R⁴ durch R³ zu ersetzen ist, wenn die Gruppen der Formeln (20) bis (26) für Ar stehen, sowie in den Fällen der Formeln (27) bis (34).

Wenn R¹, R², R³ oder Ar für eine Gruppe der Formel (20) steht, dann steht R³ bzw. R⁴ in dieser Gruppe bevorzugt für ein aromatisches oder heteroaromatisches Ringsystem mit 5 bis 24 aromatischen Ringatomen, welches durch einen oder mehrere Reste R⁵ substituiert sein kann, insbesondere für Phenyl, ortho-, meta- oder para-Biphenyl, ortho-, meta-, para- oder verzweigtes Terphenyl oder ortho-, meta-, para- oder verzweigtes Quaterphenyl.

Wenn R¹, R², R³ oder Ar für eine Gruppe der Formel (21) bis (26) steht, dann steht R³ bzw. R⁴ in diesen Gruppen bevorzugt gleich oder verschieden bei jedem Auftreten für H, D oder ein aromatisches oder heteroaromatisches Ringsystem mit 5 bis 24 aromatischen Ringatomen, welches durch einen oder mehrere Reste R⁵ substituiert sein kann, insbesondere für H oder Phenyl, ortho-, meta- oder para-Biphenyl, ortho-, meta-, para- oder verzweigtes Terphenyl oder ortho-, meta-, para- oder verzweigtes Quaterphenyl.

Wenn Ar für eine Gruppe der Formel (27) bis (34) steht, dann steht R³ in diesen Gruppen bevorzugt gleich oder verschieden bei jedem Auftreten für H, D oder ein aromatisches oder heteroaromatisches Ringsystem mit 5 bis 24 aromatischen Ringatomen, welches durch einen oder mehrere Reste R⁵ substituiert sein kann, insbesondere für H oder Phenyl, ortho-, meta- oder para-Biphenyl, ortho-, meta-, para- oder verzweigtes Terphenyl oder ortho-, meta-, para- oder verzweigtes Quaterphenyl.

Wenn die erfindungsgemäße Verbindung als Matrixmaterial für einen phosphoreszierenden Emitter, als Lochtransportmaterial oder als Elektronen- bzw. Exzitonenblockiermaterial eingesetzt wird, ist mindestens ein Substituent R¹, R², R³ oder Ar bevorzugt ausgewählt aus der Gruppe bestehend aus Triarylaminderivaten, Carbazolderivaten, Indenocarbazolderivaten, Indolocarbazolderivaten, Azacarbazolderivaten, Indolderivaten, Furanderivaten, Benzofuranderivaten, Dibenzofuranderivaten, Thiophenderivaten, Benzothiophenderivaten oder Dibenzothiophenderivaten, welche jeweils durch einen oder mehrere Reste R³ bzw. R⁴ substituiert sein können, oder mindestens ein Substituent R¹, R² oder R³ steht für -NAr₂. Diese Gruppen sind bevorzugt ausgewählt aus den Gruppen der folgenden Formeln (35) bis (49), 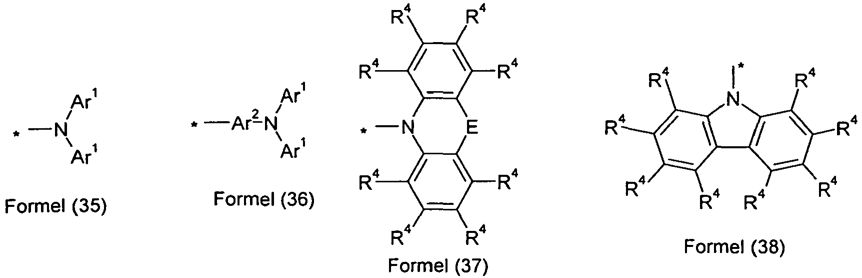 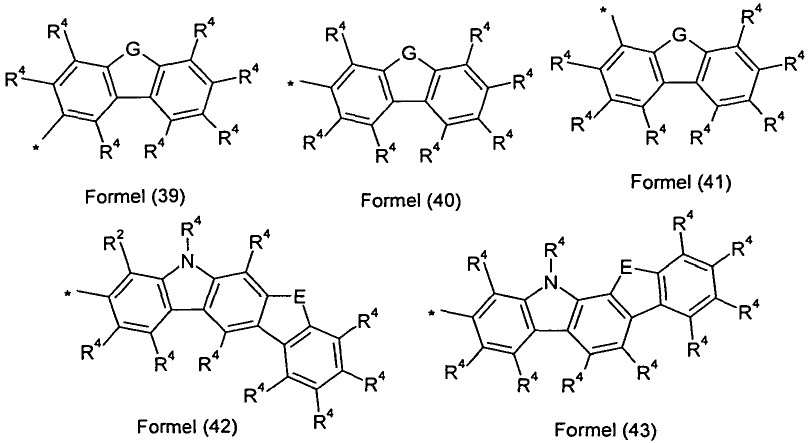 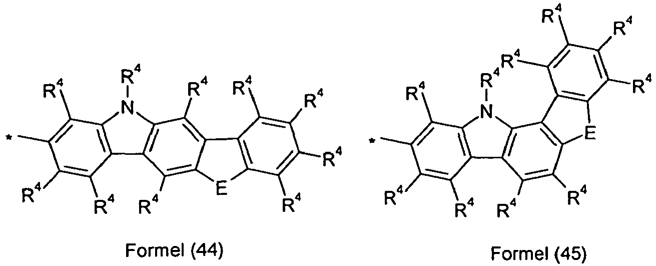 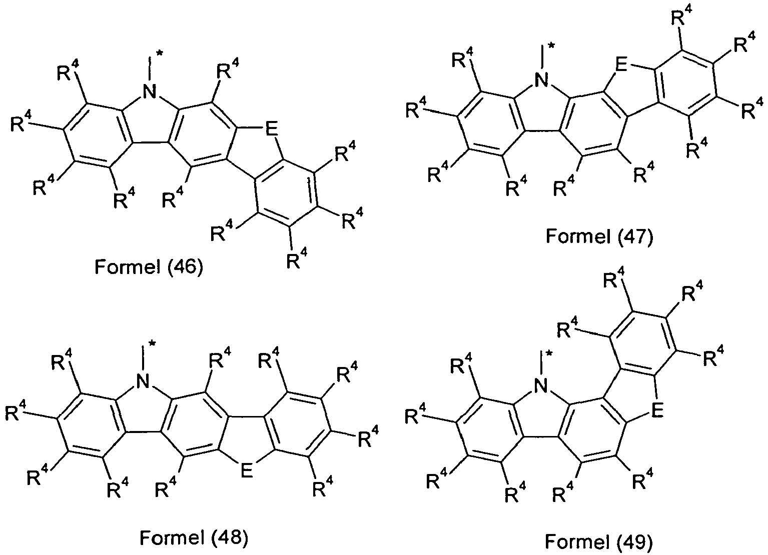 wobei die verwendeten Symbole die oben genannten Bedeutungen aufweisen und weiterhin gilt:
- E: ist ausgewählt aus der Gruppe bestehend aus C(R⁴)₂, NR⁴, O oder S;
- G: ist ausgewählt aus der Gruppe bestehend aus NR⁴, O oder S, und
R⁴ durch R³ zu ersetzen ist, wenn die Gruppen der Formeln (35) bis (49) für Ar stehen.

In einer weiteren bevorzugten Ausführungsform der Erfindung stehen in den erfindungsgemäßen Verbindungen die Symbole R¹, R² bzw. R³, die nicht für eine Gruppe der oben aufgeführten Formeln (13) bis (49) stehen, für H oder D.

Die oben genannten bevorzugten Ausführungsformen können beliebig miteinander kombiniert werden. In einer besonders bevorzugten Ausführungsform der Erfindung treten die oben genannten Bevorzugungen gleichzeitig auf.

Wenn die Verbindungen der Formel (1), (2) oder (3) bzw. die bevorzugten Ausführungsformen als Matrixmaterial für einen phosphoreszierenden Emitter verwendet werden, ist es bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, wenn die Reste R¹, R², R³ und Ar keine kondensierte Aryl- bzw. Heteroarylgruppe enthalten, in der zwei oder mehr Sechsringe direkt aneinander ankondensiert sind, und wenn zwei benachbarte Gruppen X nicht für eine Gruppe der Formel (4) stehen. Besonders bevorzugt enhält die Verbindung der Formel (1), (2) oder (3) überhaupt keine kondensierten Aryl- oder Heteroarylgruppen, in denen Sechsringe direkt aneinander ankondensiert sind.

Wenn die Verbindungen der Formel (1), (2) oder (3) bzw. die bevorzugten Ausführungsformen als Matrixmaterial für einen fluoreszierenden Emitter oder als fluoreszierender Emitter verwendet werden, ist es bevorzugt, wenn mindestens einer der Reste R¹, R² und/oder R³ eine Gruppe enthält, die ausgewählt ist aus Naphthalin, Anthracen, Phenanthren, Pyren und/oder Benzanthracen, welches jeweils noch durch die oben genannten Gruppen substituiert sein kann, und/oder wenn mindestens an einem der aromatischen Ringe zwei benachbarte Gruppen X für eine Gruppe der Formel (4) stehen.

Wenn die Verbindungen der Formel (1), (2) oder (3) bzw. die bevorzugten Ausführungsformen als Lochtransportmaterial in einer lochtransportierenden, lochinjizierenden oder anderen Schicht eingesetzt werden, ist es bevorzugt, wenn mindestens einer der Reste R¹, R² und/oder R³ eine Gruppe enthält, die ausgewählt ist aus aromatischen Ringsystemen mit 6 bis 24 aromatischen Ringatomen. Weiterhin ist es in diesem Fall bevorzugt, dass Y gleich O ist.

Beispiele für bevorzugte Verbindungen gemäß den oben aufgeführten Ausführungsformen sind die Verbindungen der folgenden Strukturen.

| | |
|---|---|
| 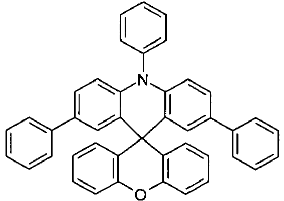 | 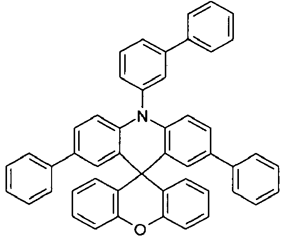 |
| 1 | 2 |
| 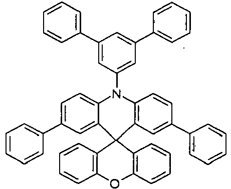 | 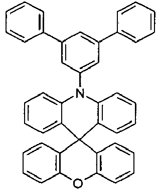 |
| 3 | 4 |
| 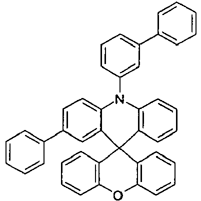 | 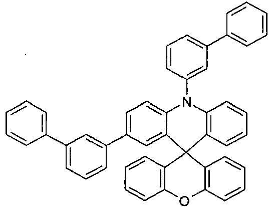 |
| 5 | 6 |
| 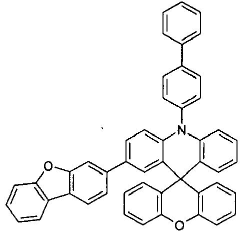 | 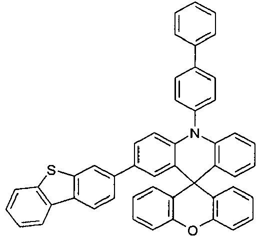 |
| 7 | 8 |
| 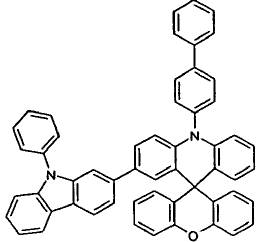 | 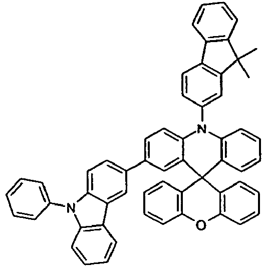 |
| 9 | 10 |
| 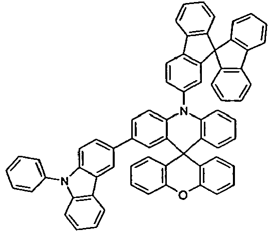 | 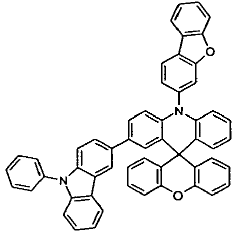 |
| 11 | 12 |
| 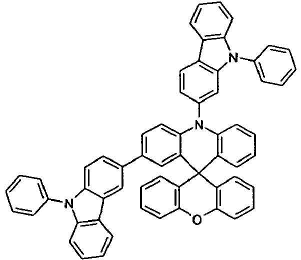 | 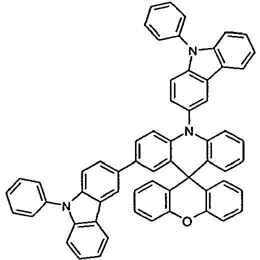 |
| 13 | 14 |
| 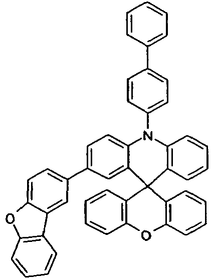 | 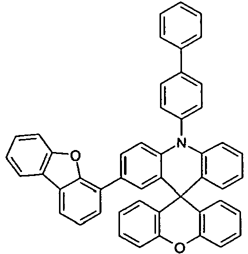 |
| 15 | 16 |
| 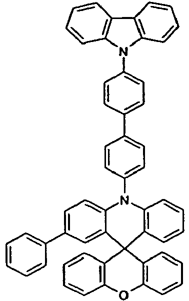 | 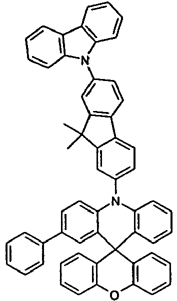 |
| 17 | 18 |
| 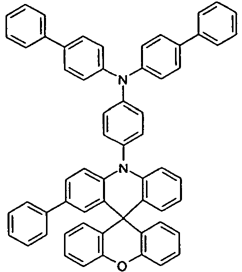 | 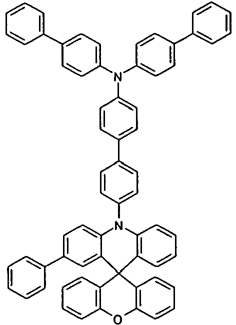 |
| 19 | 20 |
| 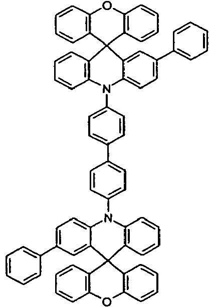 | 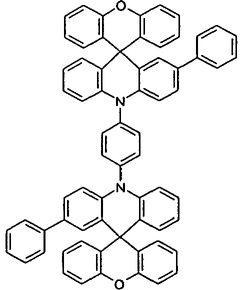 |
| 21 | 22 |
| 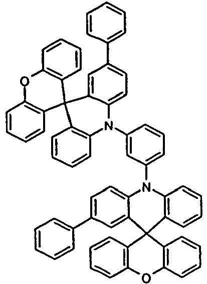 | 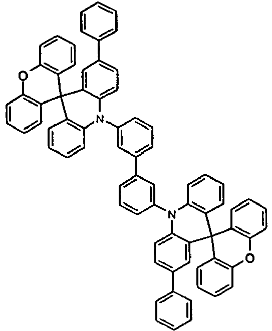 |
| 23 | 24 |
| 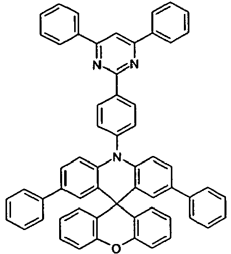 | 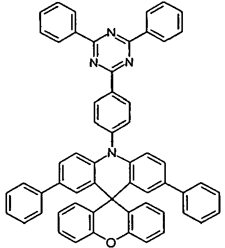 |
| 25 | 26 |
| 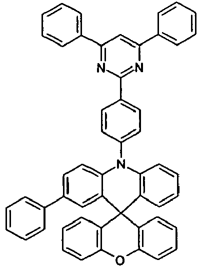 | 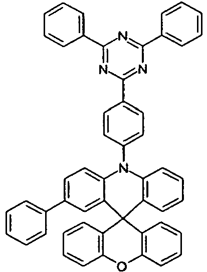 |
| 27 | 28 |
| 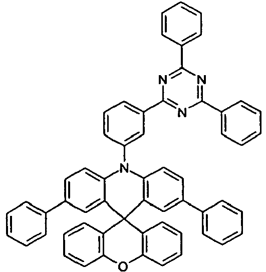 | 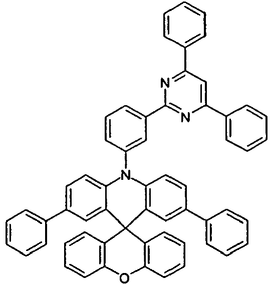 |
| 29 | 30 |
| 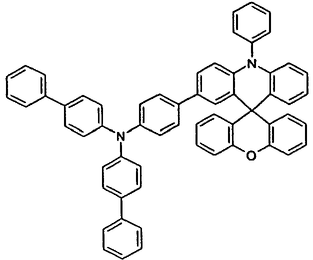 | 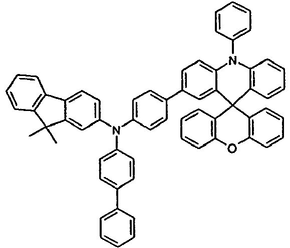 |
| 31 | 32 |
| 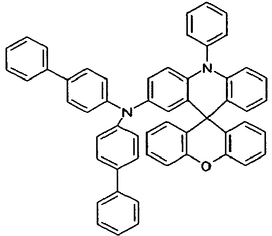 | 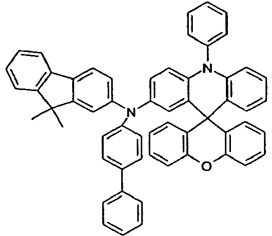 |
| 33 | 34 |
| 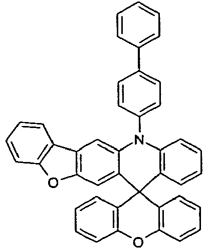 | 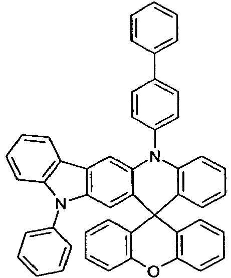 |
| 35 | 36 |
| 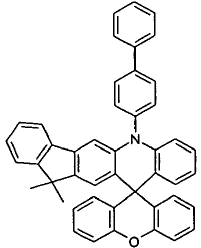 | 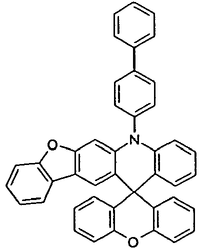 |
| 37 | 38 |
| 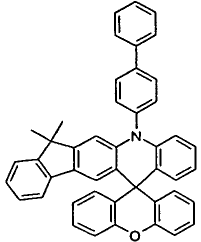 | 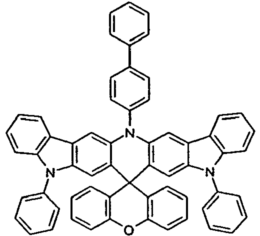 |
| 39 | 40 |
| 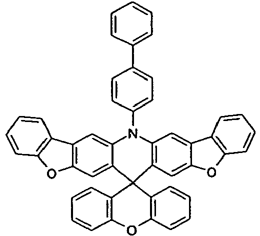 | 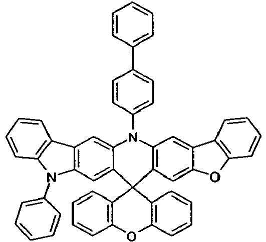 |
| 41 | 42 |
| 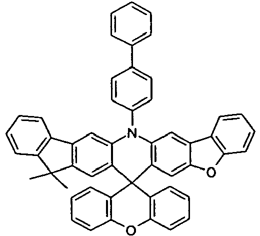 | 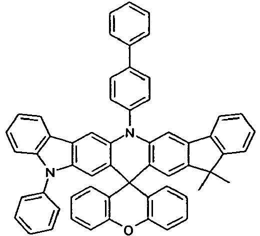 |
| 43 | 44 |
| 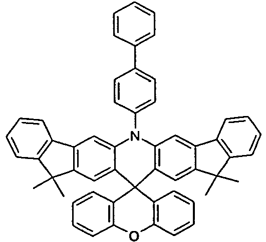 | 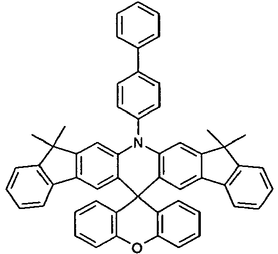 |
| 45 | 46 |
| 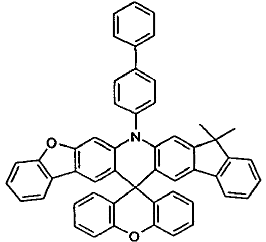 | 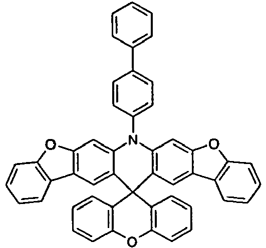 |
| 47 | 48 |
| 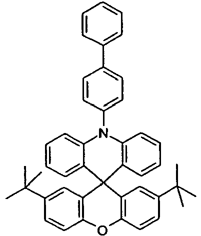 | 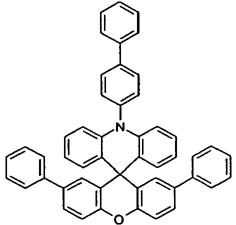 |
| 49 | 50 |
| 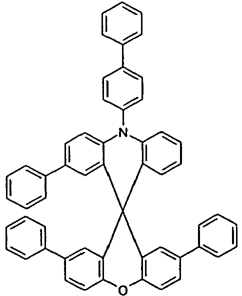 | 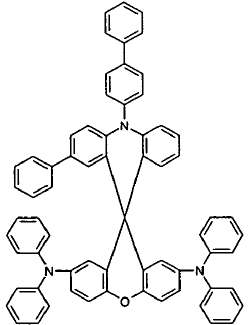 |
| 51 | 52 |
| 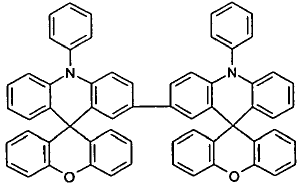 | 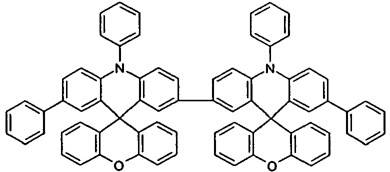 |
| 53 | 54 |
| 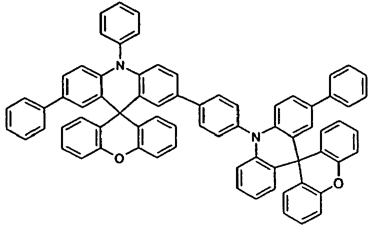 | 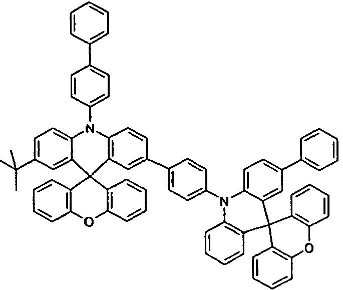 |
| 55 | 56 |
| 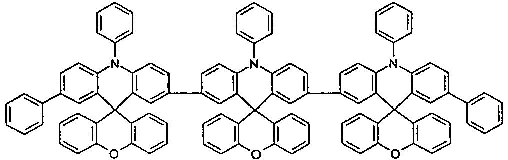 | |
| 57 | |
| 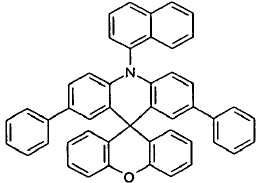 | 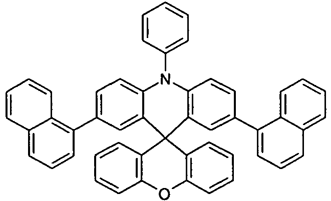 |
| 58 | 59 |
| 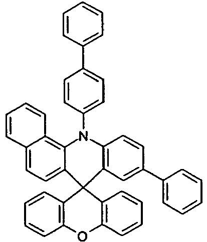 | 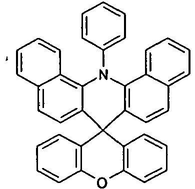 |
| 60 | 61 |
| 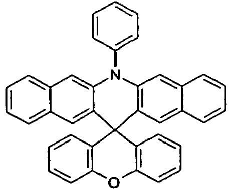 | 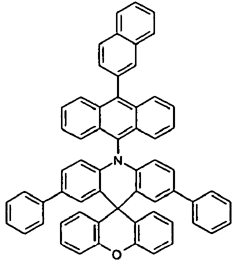 |
| 62 | 63 |
| 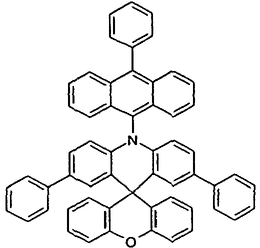 | 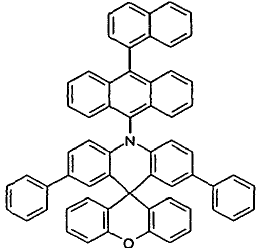 |
| 64 | 65 |
| 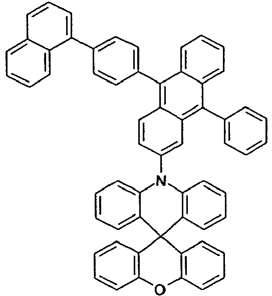 | 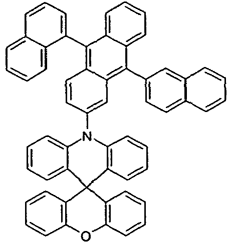 |
| 66 | 67 |
| 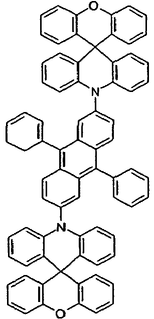 | 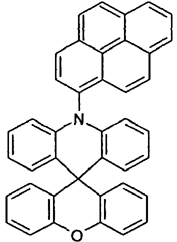 |
| 68 | 69 |
| 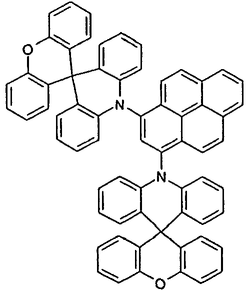 | 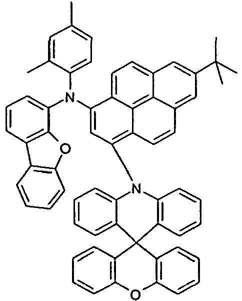 |
| 70 | 71 |
| 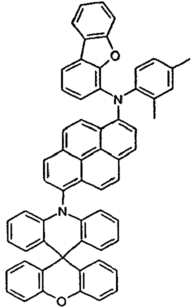 | 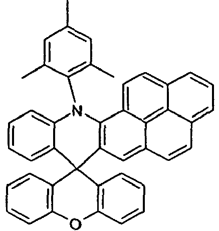 |
| 72 | 73 |
| 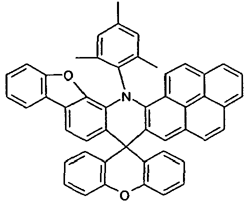 | 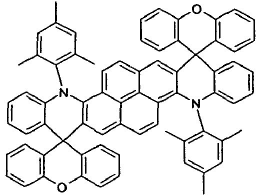 |
| 74 | 75 |
| 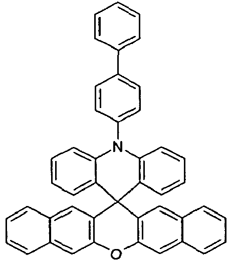 | 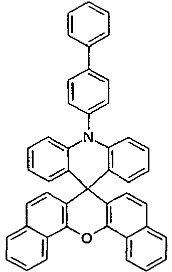 |
| 76 | 77 |
| 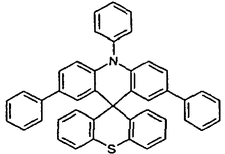 | 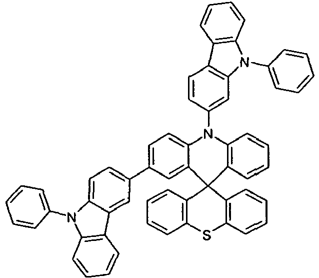 |
| 78 | 79 |
| 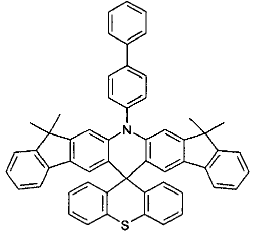 | 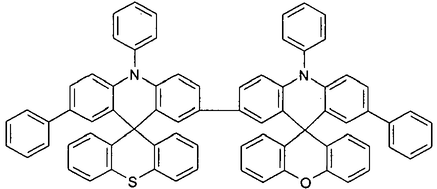 |
| 80 | 81 |
| | 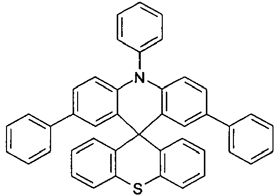 |
| | 89 |
| 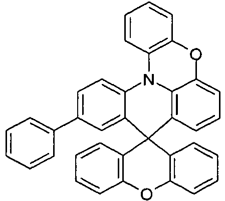 | 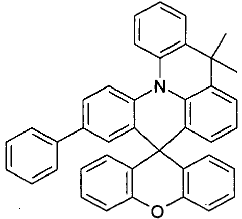 |
| 92 | 93 |
| 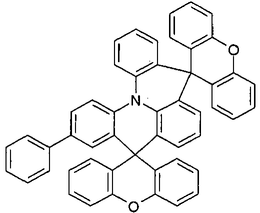 | 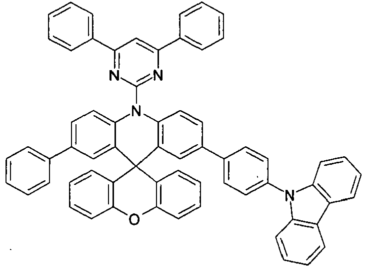 |
| 94 | 95 |
| 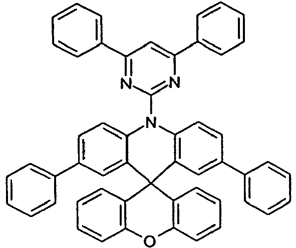 | 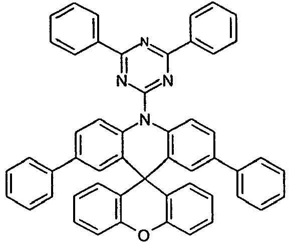 |
| 96 | 97 |
| 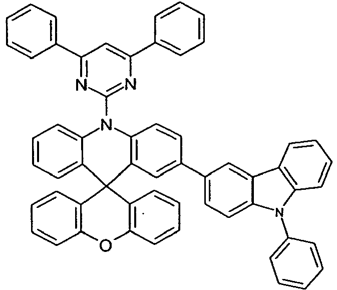 | 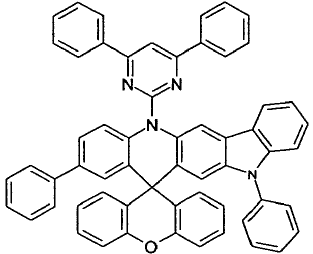 |
| 98 | 99 |
| 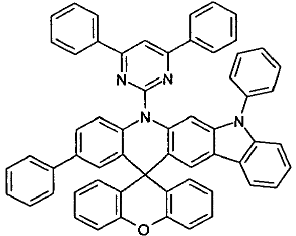 | 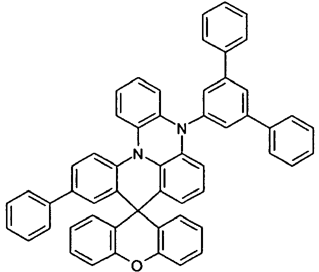 |
| 100 | 101 |

Die erfindungsgemäßen Verbindungen können nach den in Schema 1 bis 4 skizzierten Wegen dargestellt werden.

Ausgehend von einem 1,2-Dihalogen-substituierten Aromaten bzw. Heteroaromaten führt eine Buchwald- bzw. Ullmann-Kupplung mit einem sekundären Amin zu einem o-Halogen-funktionalisierten tertiären Amin. Dieses kann nach Metallierung mit einem heterocyclischen Keton zu einem Triarylmethanol gekuppelt werden, welches säurekatalysiert zur erfindungsgemäßen Spiroverbindung (A) cyclisiert (Schema 1). Die Spiroverbindung (A) kann regioselektiv - bevorzugt in p-Position zum N-Atom-mono- oder dibromiert (gegebenenfalls auch tri-, tetra-, etc. bromiert unter Einbeziehung weiterer aktiver Positionen) werden, die so entstandenen Bromide (B) und (C) können anschließend nach dem Fachmann geläufigen Methoden (C-C-Kuplung wie Suzuki-, Negishi-, Yamamoto-, Grignard-Cross-, Stille-, Heck-Kupplung, etc.; C-N-Kupplung wie Buchwald- oder Ullmann-Kupplung, Sililyierung, Phosphanylierung, Boranylierung, Poly-Kondensation, etc.) weiter umgesetzt werden. Neben der regioselektiven Bromierung ist selbsverständlich auch die Umsetzung mit anderen Elektrophilen z. B. in Friedel-Krafts-Alkylierung oder - Acylierung, Sulfonierung, Nitrierung, etc. möglich.

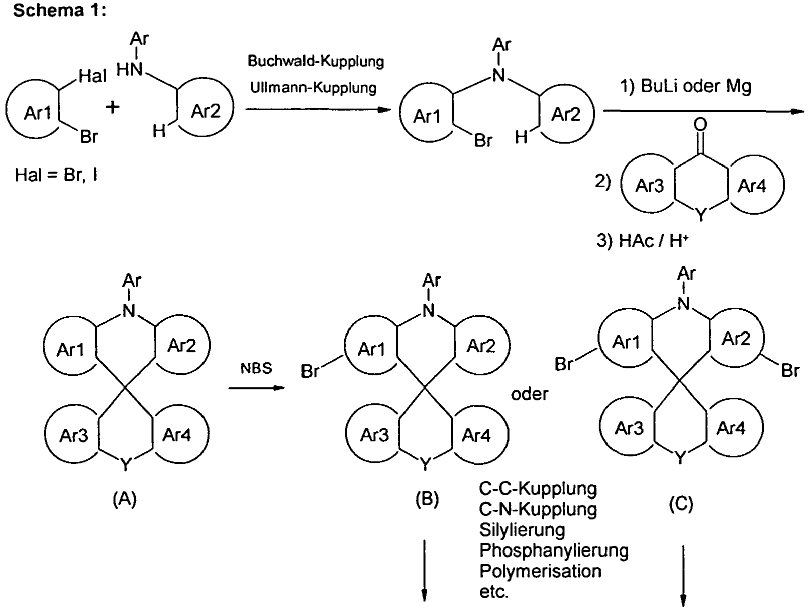

Trägt das sekundäre Amin zu Beginn der Sequenz eine Schutzgruppe PG (z. B. N-Acyl, N-Boc, N-Benzyl, N-Sulfenyl, N-Silyl, etc.), so kann nach Aufbau der Spiroverbindung (D) durch Schutzgruppenabspaltung das sekundäre Spiroamin (E) erhalten werden, das nach dem Fachmann geläufigen Methoden der C-N-Kupplung (Buchwald- oder Ullmann-Kupplung, etc.) weiter umgesetzt werden kann (Schema 2). Auf diesem Wege sind erfindungsgemäße Verbindungen, enthaltend mehrere der Einheiten (E), durch Kupplung mit mehrfach funktionalisierten Aromaten zugänglich. Diese können dann über den in Schema 1 beschriebenen Weg der Umsetzung mit Elektrophilen weiter funktionalisiert werden.

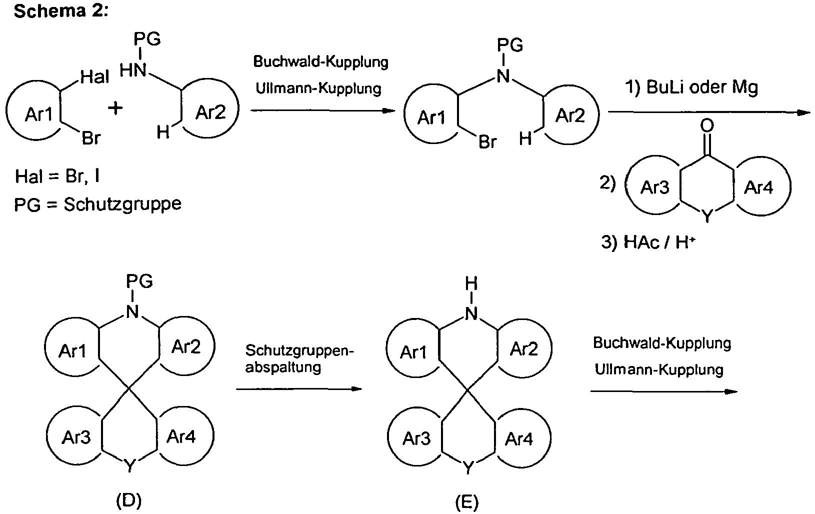

Das Bromid (B) eignet sich außerdem zum Aufbau erweiterter erfindungsgemäßer kondensierter Heterocyclen (Schema 3). Suzuki-Kupplung von (B) mit o-YH-funktionalisierten Aromaten bzw. Heteroaromaten führt zu (F), das zu (G) cyclisiert werden kann. Analog führt eine Buchwald- oder Ullmann-Kupplung von (B) zu (H), das zu (I) cyclisiert werden kann.

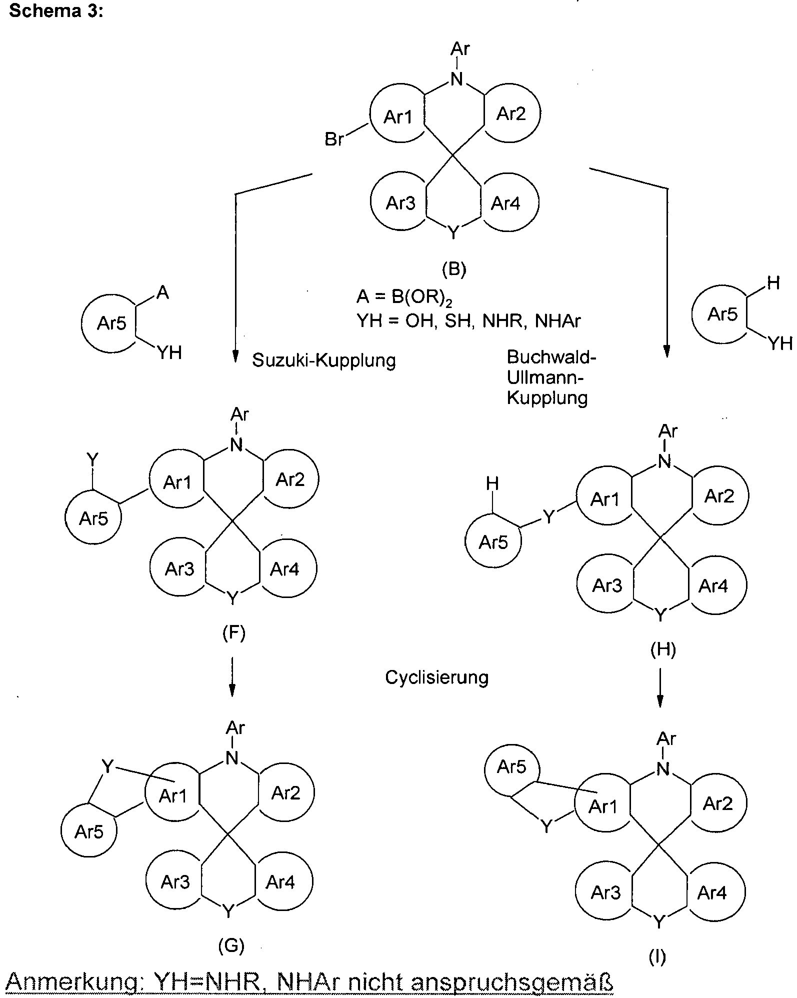

Das Spiroamin (E) eignet sich weiterhin zum Aufbau erweiterter erfindungsgemäßer kondensierter Heterocyclen (K) (Schema 4). C-N-Kupplung von (E) mit o-Halogen-funktionalisierten Aromaten, die YH-Funktionen tragen, und anschließende Cyclisierung des Intermediats (J) führt zu erweiterten Heterocyclen (K).

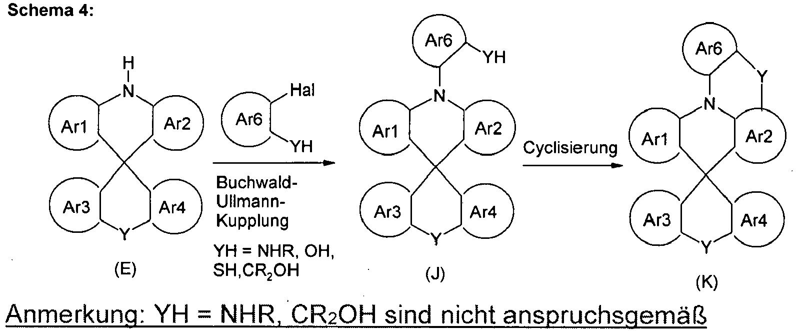

Die in Schema 3 und 4 gezeigten Umsetzungen sind selbstverständlich auch analog mit dem Dibromid (C) möglich. Auf diese Weise können nach dem Fachmann geläufigen Methoden kondensierte Heterocyclen enthaltend Fluorene, Dibenzofurane, Thiodibenzofurane, Carbazole, etc. erhalten werden.

Ein weiterer Gegenstand der vorliegenden Erfindung ist ein Verfahren zur Herstellung einer Verbindung gemäß Formel (1), (2) oder (3), umfassend die Reaktionsschritte:
a) Aufbau des halogenierten Grundkörpers; und
b) Einführung von Substituenten über eine übergangsmetallkatalysierte Kupplungsreaktion.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, oder mit reaktiven, polymerisierbaren Gruppen, wie Olefinen, Styrolen, Acrylaten oder Oxetanen, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten erfindungsgemäßen Verbindungen, wobei an einer oder mehreren Positionen statt Substituenten eine oder mehrere Bindungen der erfindungsgemäßen Verbindung zum Polymer, Oligomer oder Dendrimer vorhanden sind. Je nach Verknüpfung der erfindungsgemäßen Verbindung bildet diese eine Seitenkette des Oligomers oder Polymers oder ist in der Hauptkette verknüpft oder bildet den Kern eines Dendrimers. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Homopolymere oder Copolymere, wobei die Einheiten gemäß Formel (1) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/22026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten. Außerdem können die Polymere entweder einpolymerisiert oder als Blend eingemischt TriplettEmitter enthalten. Gerade die Kombination von den erfindungsgemäßen Oligomere, Polymeren oder Dendrimeren mit Triplett-Emittern führt zu besonders guten Ergebnissen.

Weiterhin können die erfindungsgemäßen Verbindungen auch weiter funktionalisiert werden und so zu erweiterten Strukturen umgesetzt werden. Hier ist als Beispiel die Umsetzung mit Arylboronsäuren gemäß Suzuki oder mit primären oder sekundären Aminen gemäß Hartwig-Buchwald zu nennen. So können die erfindungsgemäßen Verbindungen auch direkt an phosphoreszierende Metallkomplexe oder auch an andere Metallkomplexe gebunden werden.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich bei der erfindungsgemäßen organischen Elektrolumineszenzvorrichtung auch um eine Tandem-OLED handeln, insbesondere auch für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1), (2) oder (3) bzw. die oben ausgeführten bevorzugten Ausführungsformen als Matrixmaterial für fluoreszierende oder phosphoreszierende Emitter und/oder als fluoreszierenden Emitter, insbesondere als blau fluoreszierenden Emitter, und/oder in einer elektronenblockierenden bzw. exzitonenblockierenden Schicht und/oder in einer Lochtransportschicht und/oder in einer Lochblockierschicht und/oder in einer Elektronentransportschicht, je nach genauer Substitution.

In einer weiteren Ausführungsform der Erfindung enthält die organische Elektrolumineszenzvorrichtung die erfindungsgemäße Verbindung in einer optischen Auskopplungsschicht. Unter einer optischen Auskopplungsschicht wird dabei eine Schicht verstanden, die nicht zwischen der Anode und der Kathode liegt, sondern die außerhalb der eigentlichen Vorrichtung auf eine Elektrode aufgebracht wird, beispielsweise zwischen einer Elektrode und einem Substrat, um die optische Auskopplung zu verbessern.

In einer bevorzugten Ausführungsform der Erfindung wird die erfindungsgemäße Verbindung als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die erfindungsgemäße Verbindung als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Besonders geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder der nicht offen gelegten Anmeldung EP 11007693.2 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte CarbazolDerivate, z. B. gemäß WO 2011/042107, WO 2011/060867, WO 2011/088877 und der nicht offen gelegten Anmeldung EP 11003232.3, oder Triphenylenderivate, z. B. gemäß WO 2012/048781. Ebenso kann ein weiterer phosphoreszierender Emitter, welcher kürzerwellig als der eigentliche Emitter emittiert, als Co-Host in der Mischung vorhanden sein.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 2005/033244, WO 2005/019373, US 2005/0258742, WO 2010/086089, WO 2011/157339 und WO 2012/007086 entnommen werden. Weiterhin eignen sich beispielsweise die in den nicht offen gelegten Anmeldungen EP 11004545.7, EP 11005252.9 und EP 11006562.0 offenbarten Metallkomplexe. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Bevorzugt ist der Einsatz der Verbindung gemäß Formel (1), (2) oder (3) in einer Lochtransport- bzw. Lochinjektions- bzw. Elektronenblockier- bzw. Exzitonenblockierschicht.

In einer bevorzugten Ausführungsform der Erfindung werden die Verbindungen gemäß Formel (1), (2) oder (3) als Lochtransportmaterial eingesetzt. Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Die Lochtransportschicht kann direkt an die Emissionsschicht angrenzen.

Wenn die Verbindungen gemäß Formel (1), (2) oder (3) als Lochtransportmaterial oder als Lochinjektionsmaterial verwendet werden, kann es bevorzugt sein, wenn sie mit Elektronenakzeptor-Verbindungen dotiert sind, beispielsweise mit F₄-TCNQ oder mit Verbindungen, wie sie in EP 1476881 oder EP 1596445 beschrieben werden.

Als Dotanden sind prinzipiell alle Verbindungen geeignet, welche Elektronenakzeptorverbindungen darstellen und die Leitfähigkeit der organischen Schicht durch Oxidation des Hosts erhöhen können. Der Fachmann kann im Rahmen seines allgemeinen Fachwissens ohne größeren Aufwand weitere geeignete Verbindungen identifizieren und sie in Kombination mit den erfindungsgemäßen Verbindungen einsetzen. Insbesondere geeignet als Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

In einer weiteren bevorzugten Ausführungsform der Erfindung wird eine Verbindung gemäß Formel (1), (2) oder (3) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

Wird die Verbindung gemäß Formel (1), (2) oder (3) als Lochtransportmaterial in einer Lochtransportschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen in der Lochtransportschicht eingesetzt werden.

In nochmals einer weiteren bevorzugten Ausführungsform der Erfindung wird die erfindungsgemäße Verbindung als Elektronentransportmaterial in einer Elektronentransport- oder Elektroneninjektionsschicht eingesetzt. Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. LiQ (Lithiumhydroxychinolinat).

In nochmals einer weiteren bevorzugten Ausführungsform der Erfindung wird die erfindungsgemäße Verbindung in einer Lochblockierschicht eingesetzt. Unter einer Lochblockierschicht wird eine Schicht verstanden, die auf Kathodenseite direkt an eine emittierende Schicht angrenzt.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen, eingesetzt als Matrixmaterial für fluoreszierende oder phosphoreszierende Emitter, führen zu sehr hohen Effizienzen sowie zu langen Lebensdauern. Dies gilt insbesondere, wenn die Verbindungen als Matrixmaterial für einen phosphoreszierenden Emitter eingesetzt werden.
2. Die erfindungsgemäßen Verbindungen eignen sich nicht nur als Matrix für rot phosphoreszierende Verbindungen, sondern auch für grün und gegebenenfalls auch für blau phosphoreszierende Verbindungen.
3. Die erfindungsgemäßen Verbindungen eignen sich sehr gut zur Verwendung in einer Lochtransportschicht oder Elektronenblockierschicht und führen dort zu sehr guten Effizienzen und Lebensdauern der elektronischen Vorrichtungen.
4. Die erfindungsgemäßen Verbindungen lassen sich in sehr hoher Ausbeute und sehr hoher Reinheit herstellen, wodurch eine aufwändige Reinigung, die immer auch mit Materialverlusten verbunden ist, entfallen kann oder zumindest nur in erheblich geringerem Ausmaß erforderlich ist.
5. Die erfindungsgemäßen Verbindungen weisen eine hohe thermische Stabilität auf, was nicht nur bei der Herstellung der OLEDs durch Vakuumverdampfung, sondern auch bei der Aufreinigung durch Sublimationsverfahren Vorteile bietet.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern in eckigen Klammern sind die entsprechenden CAS-Nummern.

### Beispiel 1: (2-Bromphenyl)-phenyl-(2,4,6-trimethylphenyl)-amin, Synthon S1

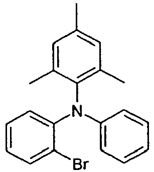

Ein Gemisch aus 211.3 g (1 mol) Mesityl-phenyl-amin [23592-67-8], 311.3 g (1.1 mol) 1-Brom-2-iod-benzol [583-55-1] und 1500 ml Toluol wird mit 50 ml (50 mmol) Tri-tert-butlyphosphin (1 M in Toluol), 5.6 g (25 mmol) Palladium(II)acetat und nach 5 min. Rühren mit 115.3 g (1.2 mol) Natriumtert-butylat versetzt und dann 16 h unter Rückfluss erhitzt. Nach Erkalten gibt man unter Rühren 500 ml Wasser zu, trennt die wässrige Phase ab, wäscht die organische Phase zweimal mit je 500 ml gesättigter Kochsalzlösung, trocknet über Magnesiumsulfat, entfernt das Toluol im Vakuum und kristallisiert das verbleibende Öl aus ca. 1000 ml 1-Butanol um. Ausbeute: 224.0 g (611 mmol), 61 %; Reinheit: das Produkt enthält nach NMR ca. 15 % (2-lodphenyl)-phenyl-(2,4,6-trimethylphenyl)-amin, es wird im Folgenden ohne weitere Reinigung umgesetzt.

Analog können folgende Verbindungen erhalten werden:

| Bsp. | sekundäres Amin | Produkt | Ausbeute |
|---|---|---|---|
| 2 | 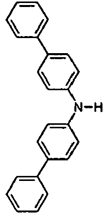 | 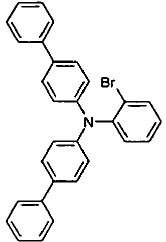 | 68% |
| | [102113-98-4] | S2 | |
| 3 | 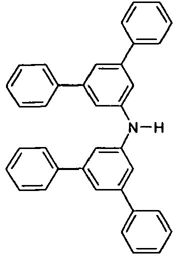 | 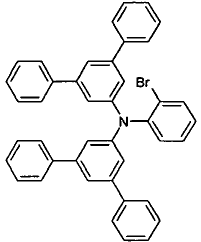 | 56% |
| | [1290039-78-9] | S3 | |
| 4 | 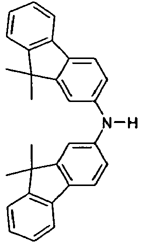 | 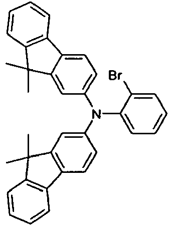 | 58% |
| | [500717-23-7] | S4 | |
| 5 | 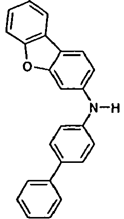 | 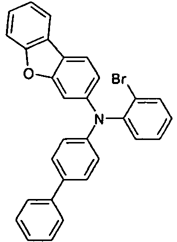 | 64% |
| | [1290039-85-8] | S5 | |
| 19 | 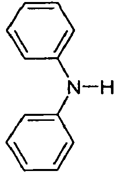 | 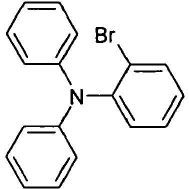 | 69% |
| | 122-39-4 | S6 | |
| 20 | 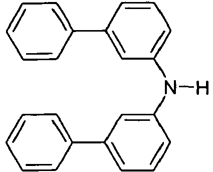 | 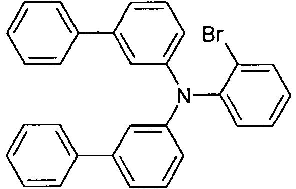 | 66% |
| | 169224-65-1 | S7 | |
| 21 | 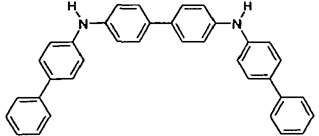 | 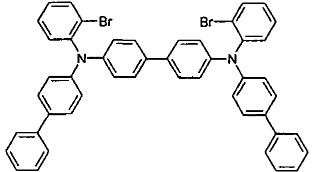 | 56% |
| | 531-91-9 | S8 | |
| 22 | 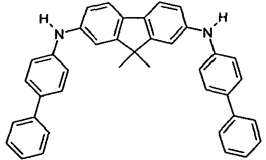 | 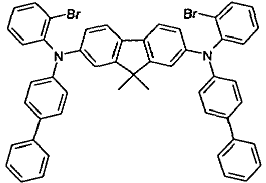 | 57% |
| | 354987-86-3 | S9 | |

### Beispiel 6: 10-Mesityl-spiro[acridin-9(10H),9'-xanthen]

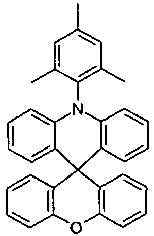

Eine auf-78 °C gekühlte, gut gerührte Suspension von 91.6 g (250 mmol) (2-Bromphenyl)-phenyl-(2,4,6-trimethylphenyl)-amin (S1) in 750 ml THF wird tropfenweise mit 100 ml (250 mmol) n-Butyllithium (2.5 M in n-Hexan) versetzt und 30 min. nachgerührt. Anschließend tropft man eine Suspension von 49.1 g (250 mmol) Xanthon [90-47-1] zu, rührt 15 min. nach und lässt die Reaktionsmischung dann langsam auf Raumtemperatur erwärmen. Man entfernt das Lösungsmittel komplett im Vakuum, nimmt den öligen Rückstand in 700 ml Eisessig auf und rührt die Suspension 4 h bei 70 °C. Nach Erkalten saugt man vom Feststoff ab, wäscht diesen zweimal mit je 100 ml Eisessig, zweimal mit je 100 ml Ethanol, trocknet dann im Vakuum und kristallisiert das Rohprodukt einmal aus ca. 200 ml Dioxan um. Ausbeute: 84.8 g (182 mmol), 73 %; Reinheit nach NMR ca. 99.5 %.

Analog können folgende Verbindungen erhalten werden:

| Bsp. | sekundäres Amin | Produkt | Ausbeute |
|---|---|---|---|
| 7 | 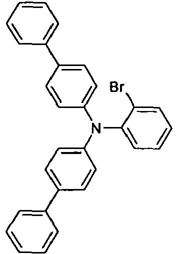 | 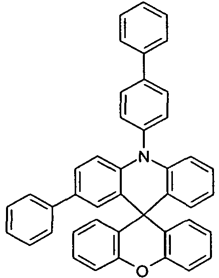 | 64 % |
| | S2 | | |
| 8 | 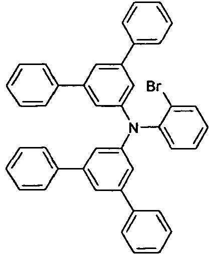 | 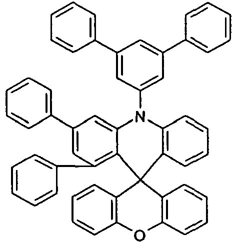 | 71 % |
| | S3 | | |
| 9 | 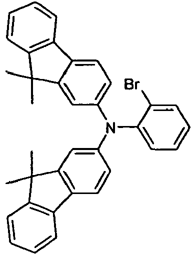 | 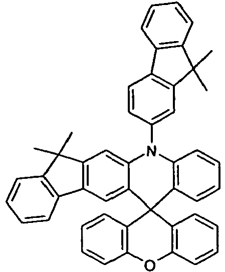 | 38 % |
| | S4 | dreimalige Umkristallisation aus Dioxan | |
| 10 | 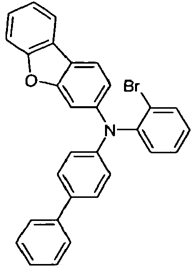 | 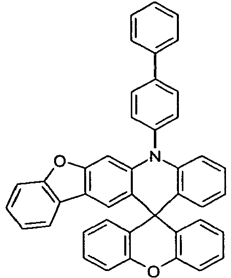 | 23 % |
| | S5 | fünfmalige Umkristallisation aus Dioxan | |
| 23 | 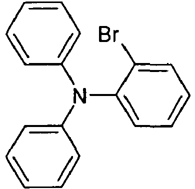 | 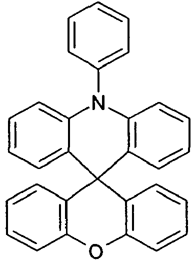 | 76% |
| | S6 | | |
| 24 | 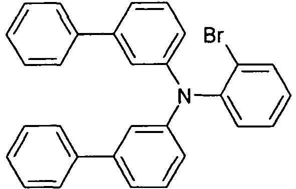 | 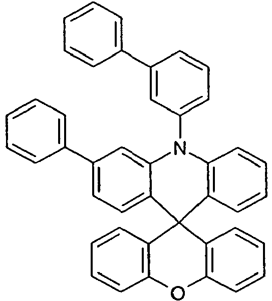 | 54 % |
| | S7 | | |
| 25 | 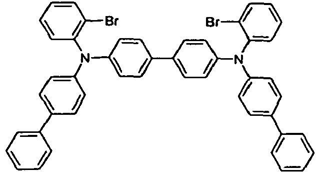 | 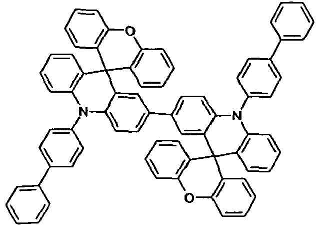 | 19% |
| | S8 | | |
| 26 | 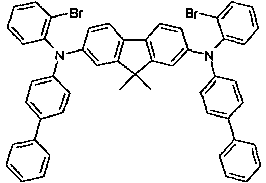 | 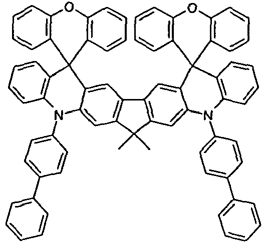 | 26 % |
| | S9 | | |
| 27 | 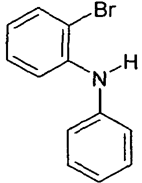 | 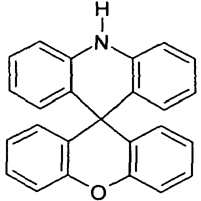 | 48% |
| | 61613-22-7 Einsatz von 500 mmol n-BuLi | | |
| 28 | 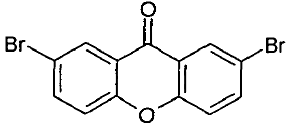 | 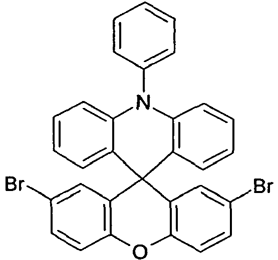 | 73 % |
| | 40102-85-0 | | |
| | 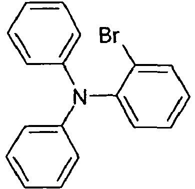 | | |
| | S6 | | |
| 29 | 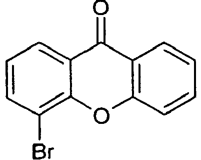 | 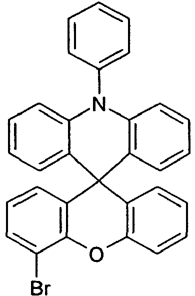 | 71% |
| | 861548-92-7 | | |
| | 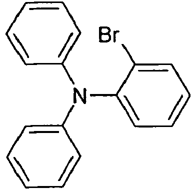 | | |
| | S6 | | |
| 30 | 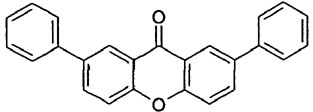 | 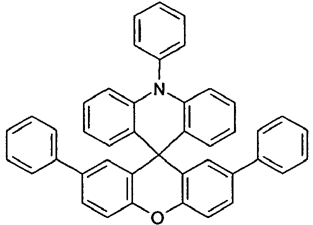 | 78% |
| | 1316277-14-1 | | |
| | 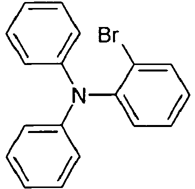 | | |
| | S6 | | |
| 31 | 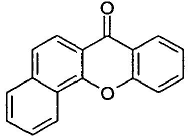 | 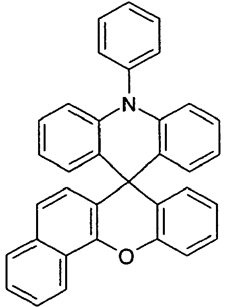 | 72% |
| | 63154-69-8 | | |
| | 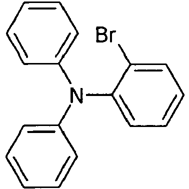 | | |
| | S6 | | |
| 32 | 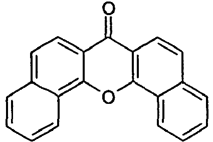 | 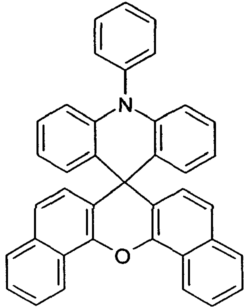 | 76% |
| | 3264-24-2 | | |
| | 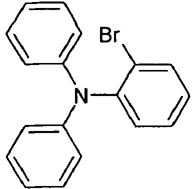 S6 | | |
| 33 | 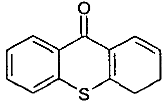 | 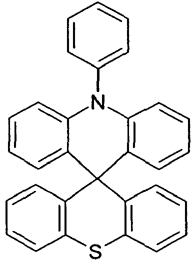 | 68 % |
| | 492-22-8 | | |
| | 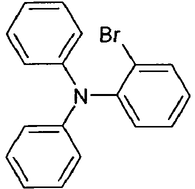 | | |
| | S6 | | |
| 34 | 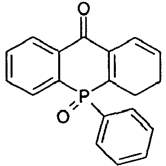 | 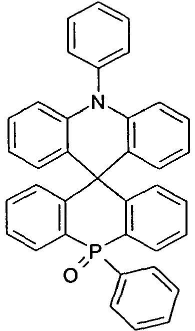 | 37% |
| | 54086-38-3 | | |
| | 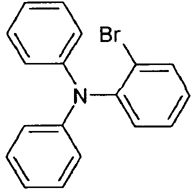 | | |
| | S6 | | |
| 35 | 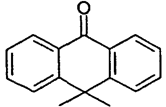 | 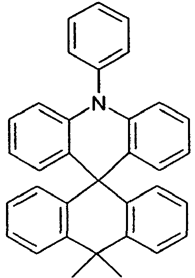 | 53 % |
| | 5447-86-9 | | |
| | 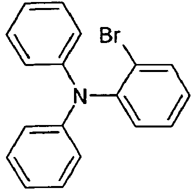 | | |
| | S6 | | |
| 36 | 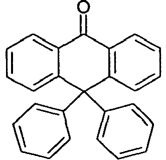 | 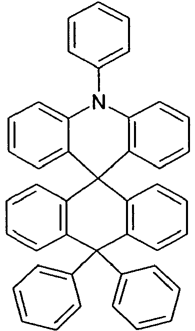 | 59% |
| | 3216-03-3 | | |
| | 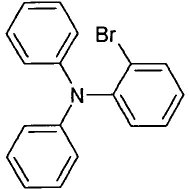 | | |
| | S6 | | |
| 37 | 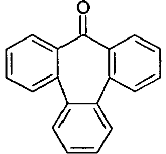 | 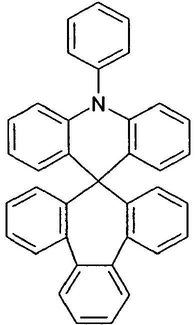 | 43 % |
| | 68089-73-6 | | |
| | 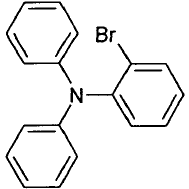 | | |
| | S6 | | |

### Beispiel 11: 3,6-Dibrom-10-mesityl-spiro[acridin-9(10H),9'-xanthen]

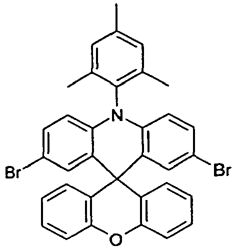

Eine auf 35 °C temperierte Lösung von 69.8 g (150 mmol) 10-Mesityl-spiro[acridin-9(10H),9'-xanthen] in 1000 ml Dichlormethan wird portionsweise mit 55.2 g (310 mmol) NBS versetzt und dann 16 h unter Rückfluss gerührt. Nach Erkalten entfernt man das Dichlormethan im Vakuum, nimmt den breiigen Rückstand in 500 ml Methanol auf, rührt 1 h bei 45 °C nach, saugt vom kristallinen Feststoff ab, wäscht diesen zweimal mit je 100 ml Methanol und trocknet im Vakuum. Ausbeute: 87.0 g (140 mmol), 93 %; Reinheit nach NMR ca. 99.5 %.

Analog können folgende Verbindungen erhalten werden:

| Bsp. | Edukt | Produkt | Ausbeute |
|---|---|---|---|
| 12 | 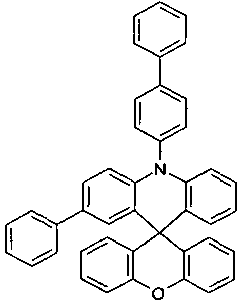 | 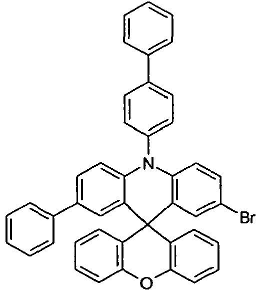 | 86% |
| | | Es werden nur 1.05 Äquivalente NBS eingesetzt | |
| 13 | 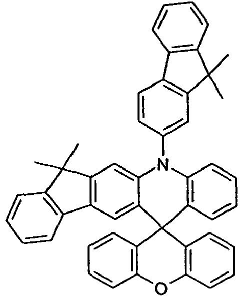 | 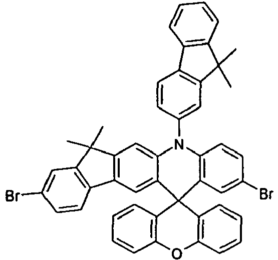 | 73 % |
| | | Zweimalige Umkristallisation des Rohprodukts aus Dioxan | |
| 38 | 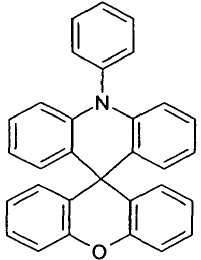 | 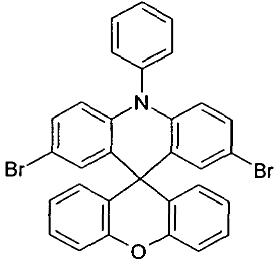 | 79 % |
| 39 | 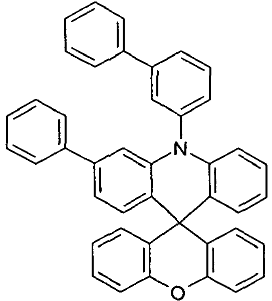 | 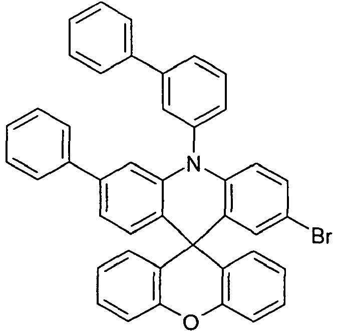 | 76 % |
| | | Es werden nur 1.05 Äquivalente NBS eingesetzt | |
| 40 | 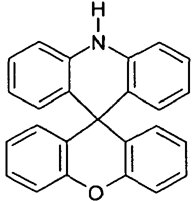 | 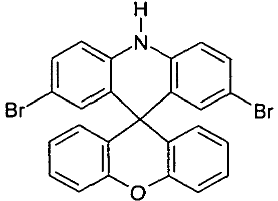 | 70% |
| 41 | 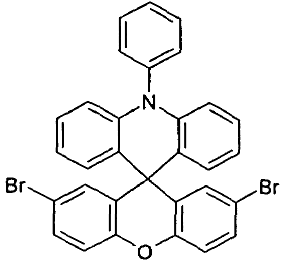 | 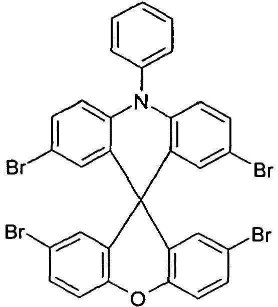 | 74 % |
| 42 | 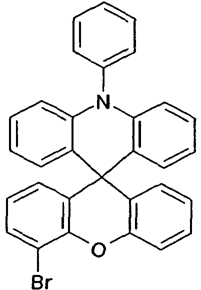 | 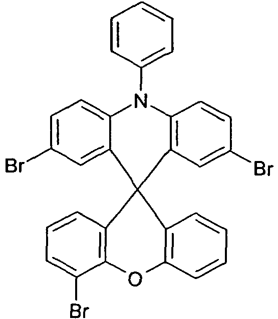 | 68 % |
| 43 | 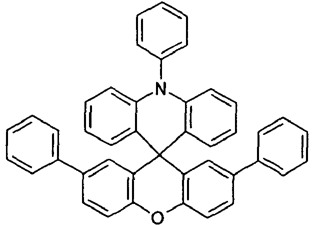 | 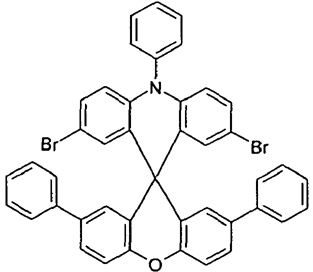 | 76 % |
| 44 | 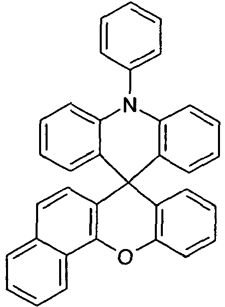 | 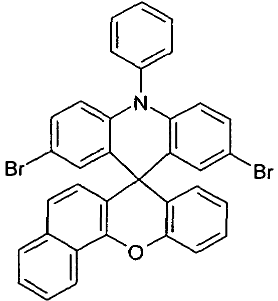 | 73 % |
| 45 | 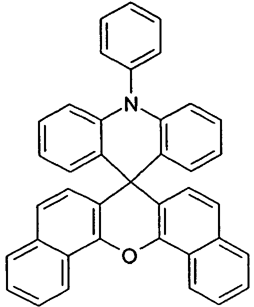 | 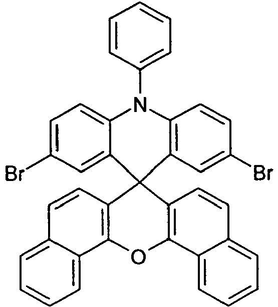 | 75 % |
| 46 | 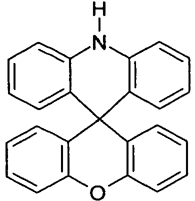 | 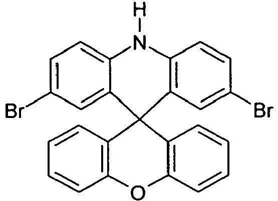 | 70 % |
| 47 | 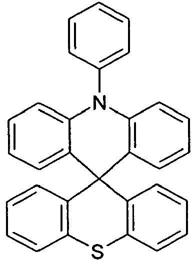 | 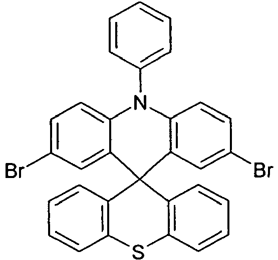 | 76 % |
| 48 | 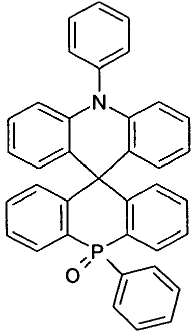 | 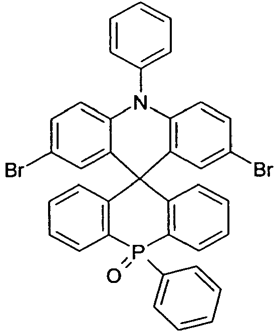 | 68 % |
| 49 | 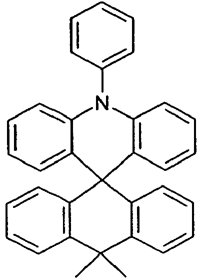 | 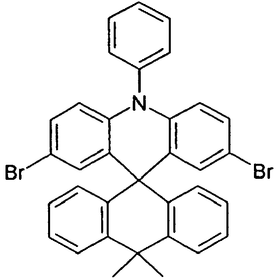 | 80 % |
| 50 | 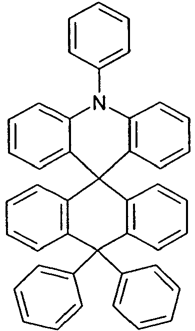 | 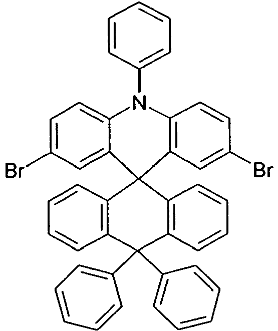 | 75 % |
| 51 | 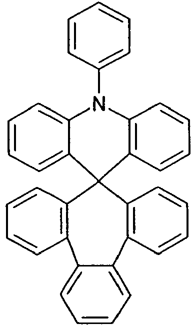 | 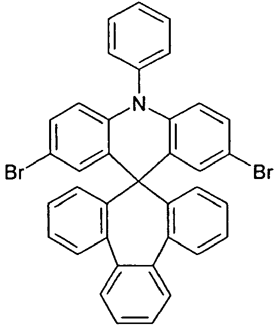 | 69 % |

### Beispiel 14: 3,6-Dipheny-10-mesityl-spiro[acridin-9(10H),9'-xanthen]

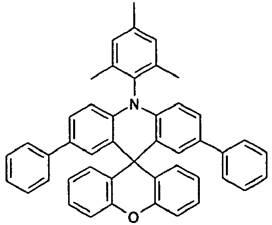

Ein Gemisch aus 62.3 g (100 mmol) 3,6-Dibrom-10-mesityl-spiro[acridin-9(10H),9'-xanthen], 30.5 g (250 mmol) Phenylboronsäure, 63.7 g (300 mmol) Trikaliumphosphat, 1.8 g (6 mmol) Tri-o-tolylphosphin, 225 mg (1 mmol) Palladium(II)acetat, 400 ml Toluol, 200 ml Dioxan und 400 ml Wasser wird 16 h unter Rückfluss erhitzt. Nach Erkalten wird die organische Phase abgetrennt, einmal mit 500 ml Wasser und einmal mit 500 ml gesättigter Kochsalzlösung gewaschen und dann über Magnesiumsulfat getrocknet. Man filtriert über ein Celite-Bett vom Trockenmittel ab, wäscht dieses mit Toluol nach, entfernt das Toluol im Vakuum und kristallisiert den Rückstand fünfmal aus Dioxan (3 ml/g) unter Zusatz von Ethanol (1-2 ml/g) in der Siedehitze um. Die weitere Reinigung erfolgt durch zweimalige fraktionierte Sublimation (p ca. 10⁻⁶ mbar, T = 300 - 320 °C). Ausbeute: 20.4 g (33 mmol), 33 %; Reinheit: > 99.9 % n. HPLC.

Analog können folgende Verbindungen erhalten werden:

| Bsp. | Edukte Bromid / Boronsäure | Produkt | Ausbeute |
|---|---|---|---|
| 15 | 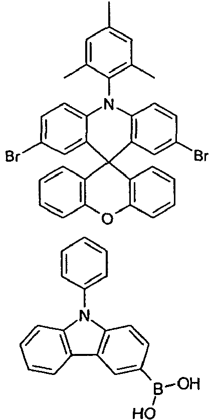 | 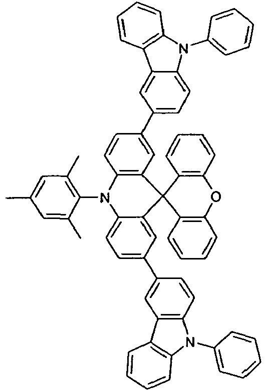 | 28 % |
| | 854952-58-2 | | |
| 16 | 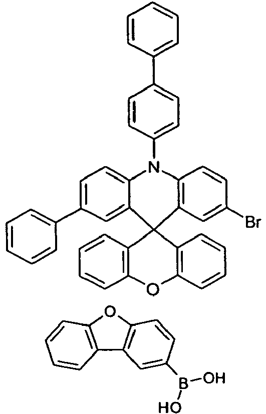 | 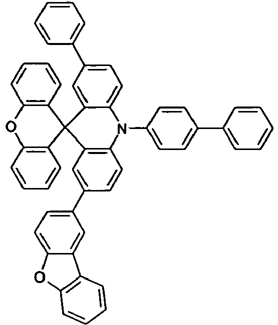 | 32 %% |
| | [402936-15-6] | | |
| | 125 mmol Boronsäure | | |
| 17 | 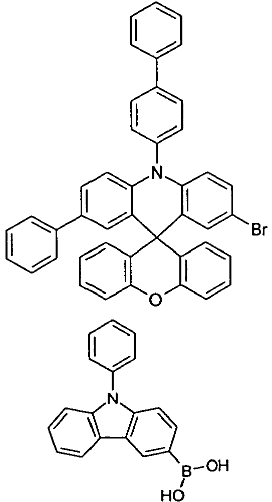 | 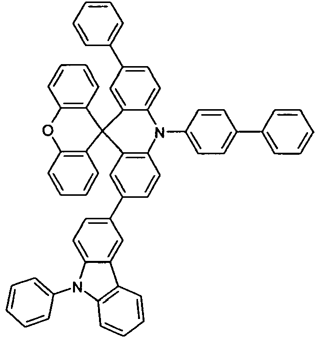 | 34 % |
| | [854952-58-2] | | |
| | 125 mmol Boronsäure | | |
| 18 | 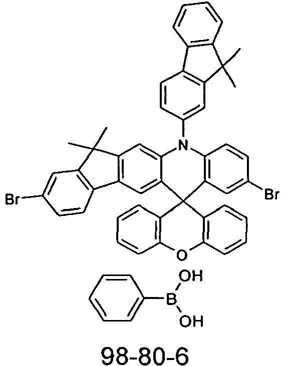 | 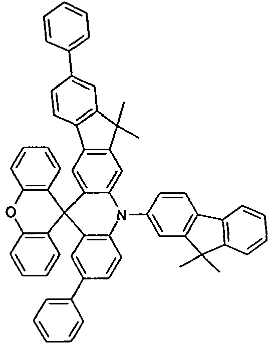 | 30 % |
| | | | |
| 52 | 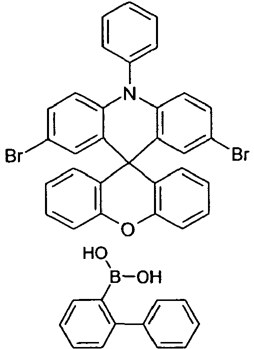 | 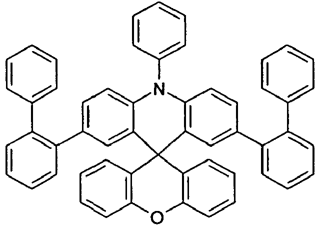 | 33 % |
| | 4688-76-0 | | |
| 53 | 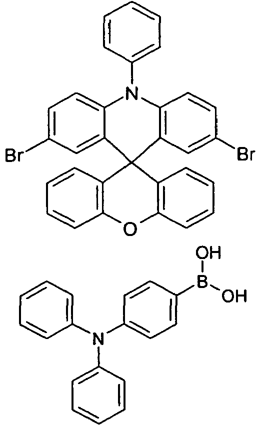 | 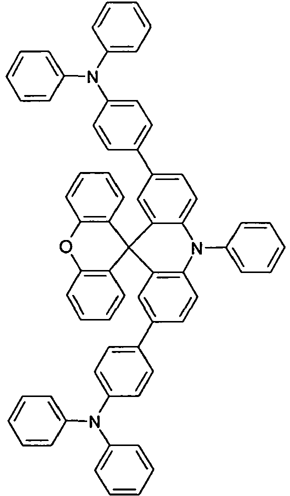 | 29 % |
| | 201802-67-7 | | |
| 54 | 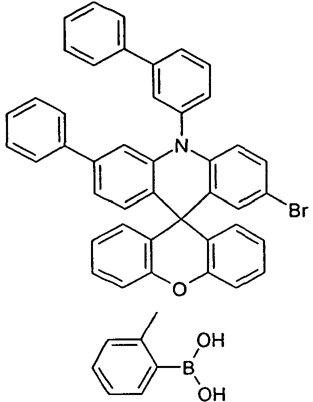 | 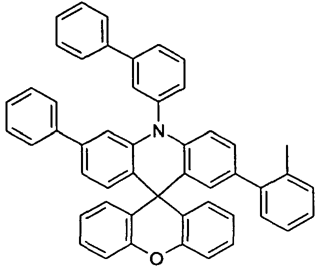 | 27 % |
| | 16419-60-6 | | |
| | 125 mmol Boronsäure | | |
| 55 | 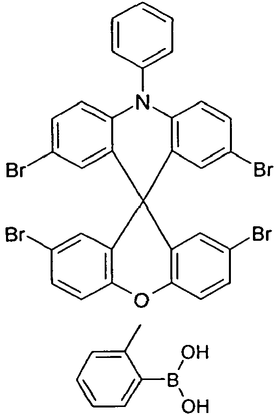 | 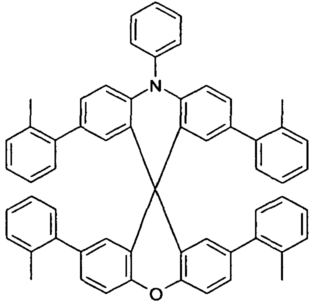 | 43 % |
| | 16419-60-6 | | |
| | 500 mmol Boronsäure | | |
| 56 | 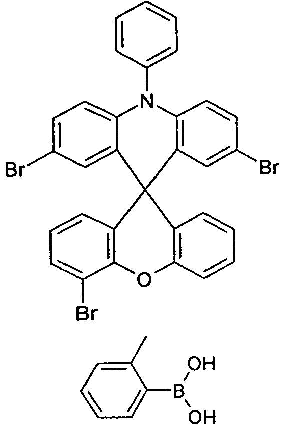 | 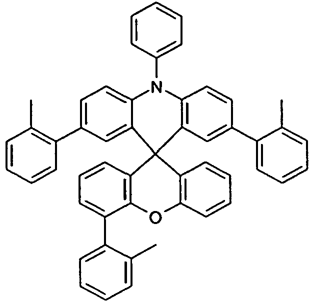 | 30 % |
| | 16419-60-6 | | |
| | 375 mmol Boronsäure | | |
| 57 | 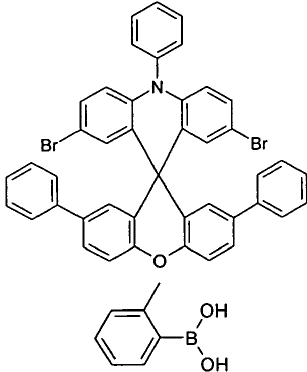 | 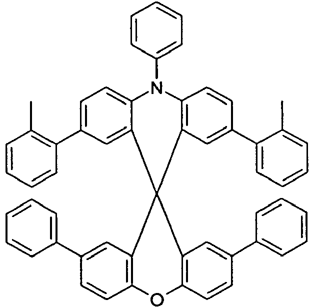 | 41 % |
| | 16419-60-6 | | |
| 58 | 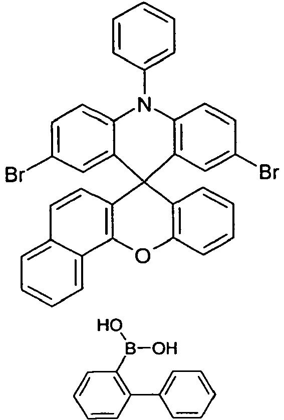 | 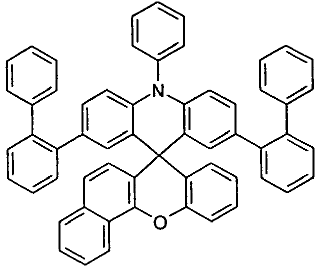 | 32 % |
| | 4688-76-0 | | |
| 59 | 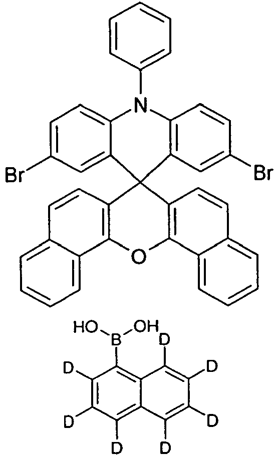 | 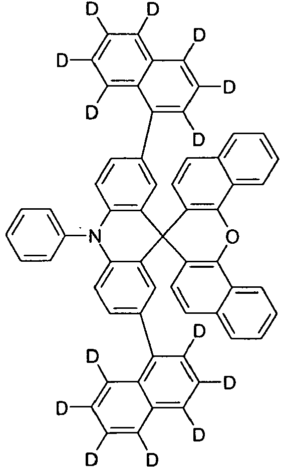 | 41 % |
| | 1000869-26-0 | | |
| 60 | 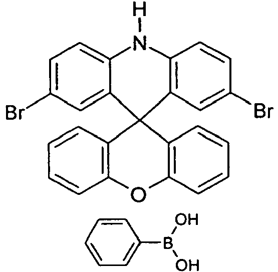 | 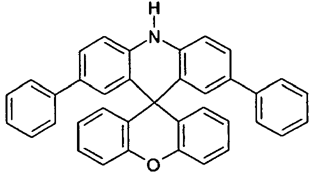 | 74% |
| | 98-80-6 | Keine Sublimation, Baustein wird weiter umgesetzt! | |
| 61 | 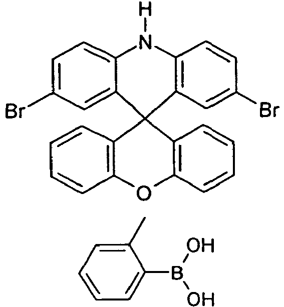 | 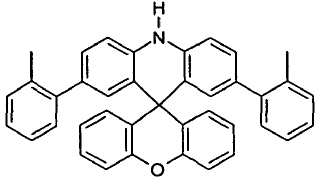 | 70 % |
| | 16419-60-6 | Keine Sublimation, Baustein wird weiter umgesetzt! | |
| 62 | 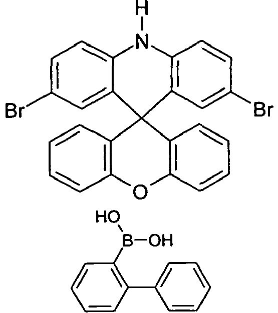 | 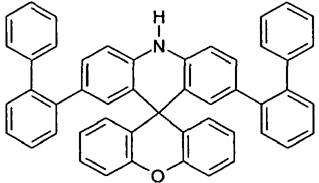 | 66 % |
| | 4688-76-0 | Keine Sublimation, Baustein wird weiter umgesetzt! | |
| 63 | 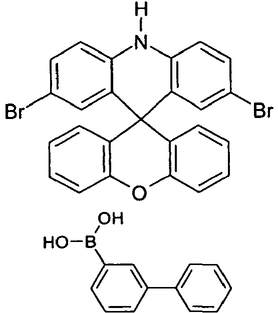 | 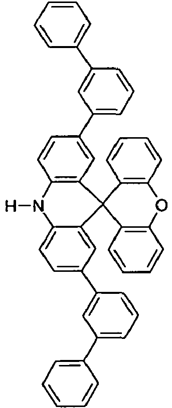 | 63 % |
| | 5122-95-2 | Keine Sublimation, Baustein wird weiter umgesetzt! | |
| 64 | 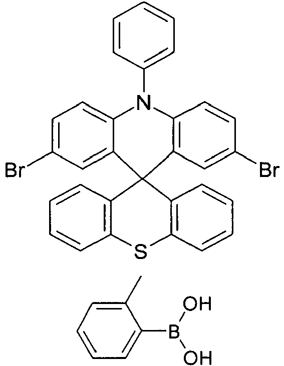 | 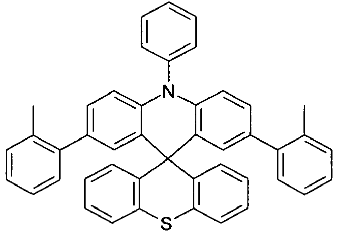 | 40 % |
| | 16419-60-6 | | |
| 65 | 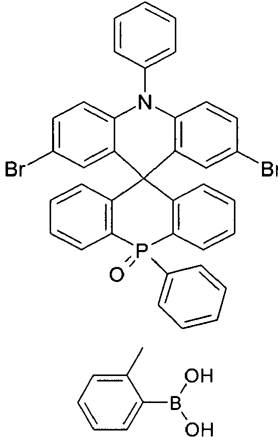 | 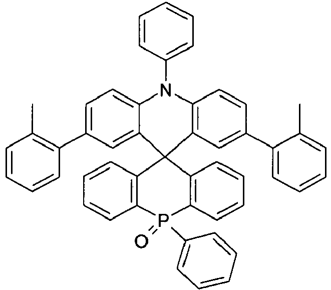 | 36 % |
| | 16419-60-6 | nicht anspruchsgemäß | |
| 66 | 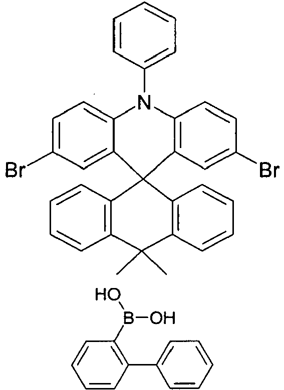 | 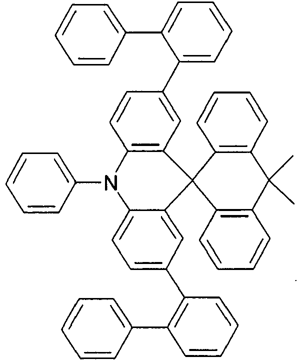 | 31 % |
| | 4688-76-0 | nicht anspruchsgemäß | |
| 67 | 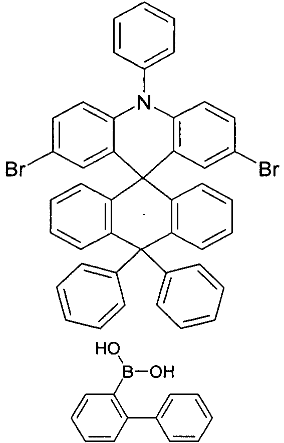 | 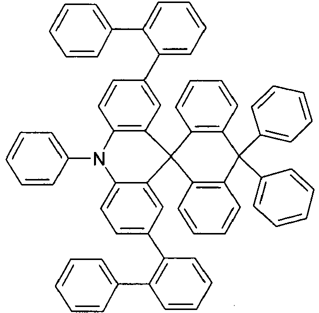 | 27 % |
| | 4688-76-0 | nicht anspruchsgemäß | |
| 68 | 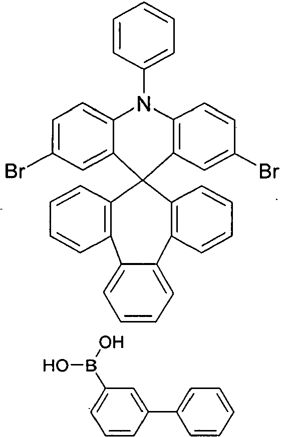 | 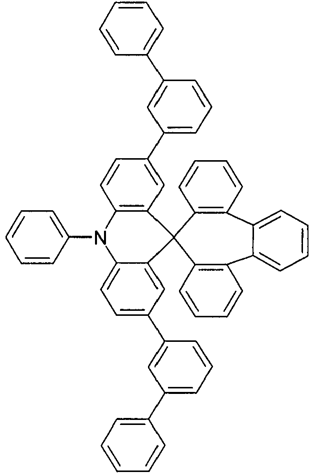 | 33 % |
| | 5122-95-2 | nicht anspruchsgemäß | |
| 69 | 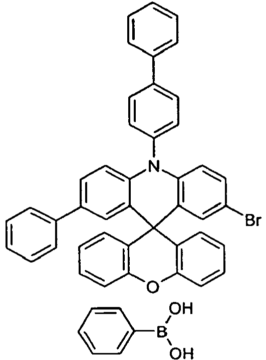 | 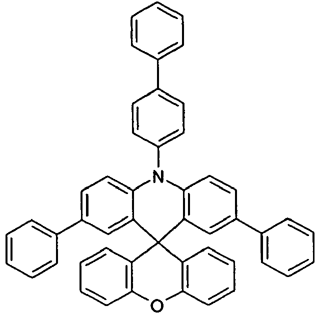 | 56 % |
| | 98-80-6 | | |
| | 125 mmol Boronsäure | | |
| 70 | 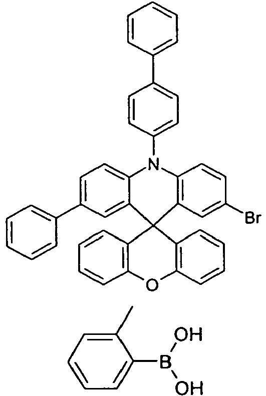 | 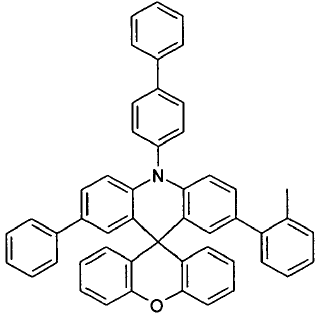 | 48 % |
| | 16419-60-6 | | |
| | 125 mmol Boronsäure | | |
| 71 | 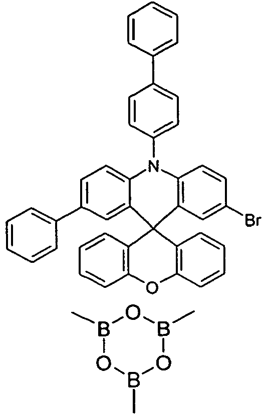 | 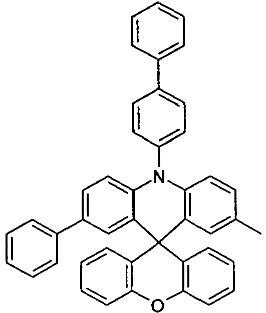 | 47 % |
| | 823-96-1 | | |
| 72 | 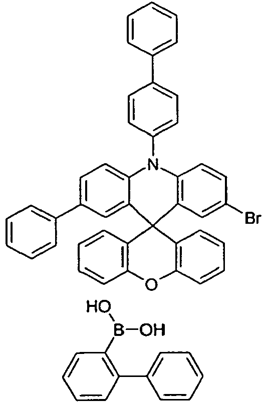 | 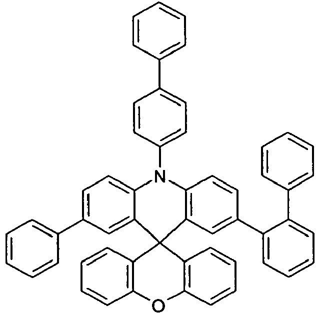 | 38 % |
| | 4688-76-0 | | |
| | 125 mmol Boronsäure | | |
| 73 | 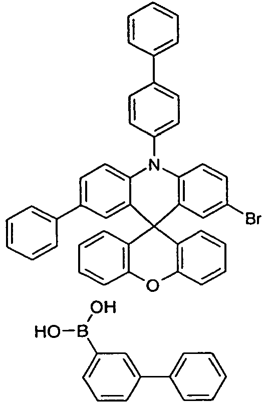 | 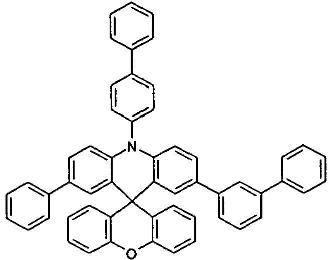 | 40 % |
| | 5122-95-2 | | |
| | 125 mmol Boronsäure | | |
| 74 | 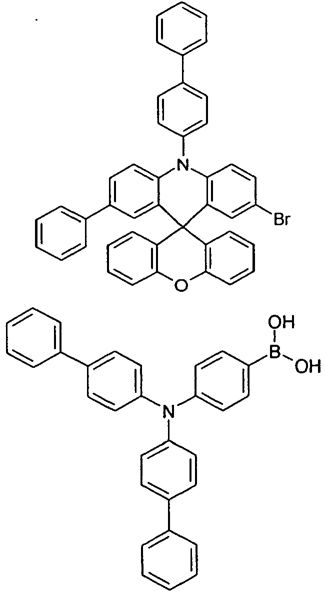 | 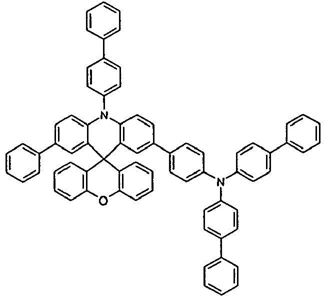 | 45 % |
| | 943836-24-6 | | |
| | 125 mmol Boronsäure | | |
| 75 | 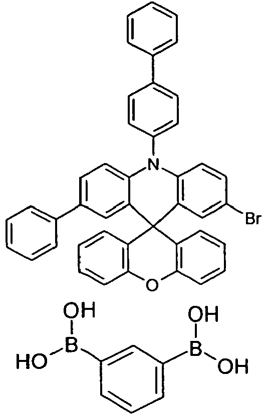 | 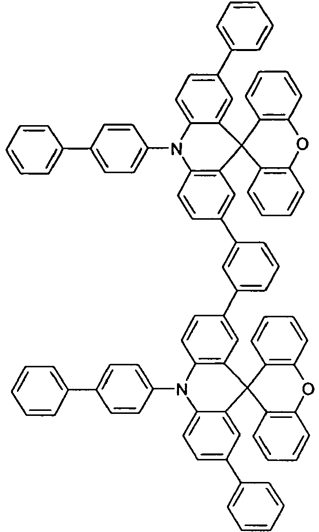 | 32 % |
| | 4612-28-6 | | |
| | 45 mmol Boronsäure | | |
| 76 | 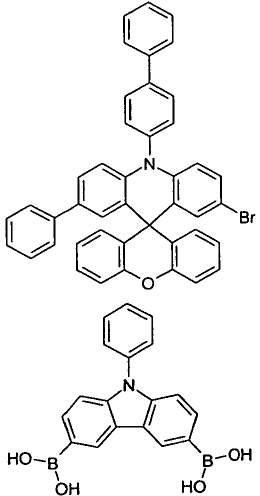 | 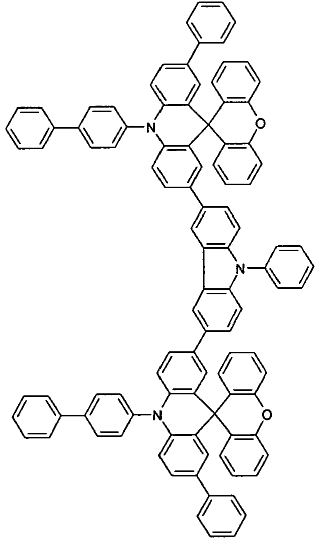 | 33 % |
| | 1135916-40-3 | | |
| | 45 mmol Boronsäure | | |
| 77 | 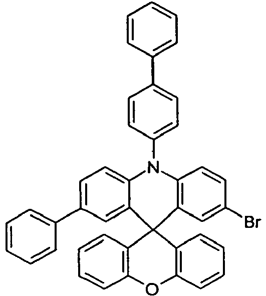 | 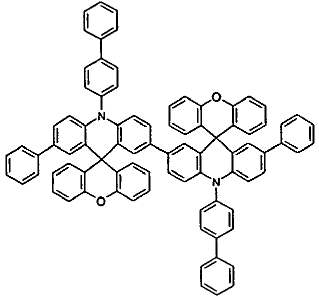 | 41 % |
| | 100 mmol | | |
| | 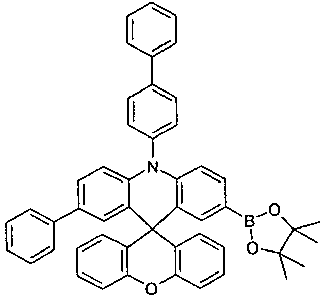 | | |
| | 110 mmol | | |
| 78 | 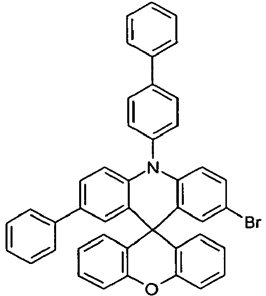 | 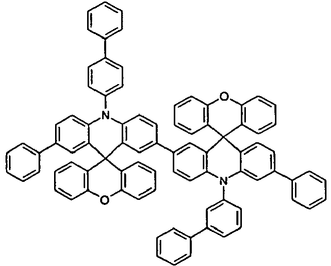 | 39 % |
| | 100 mmol | | |
| | 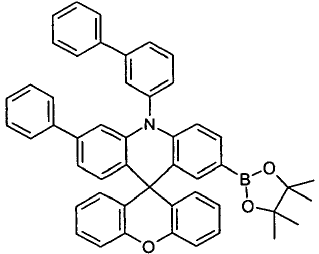 | | |
| | 110 mmol | | |
| 79 | 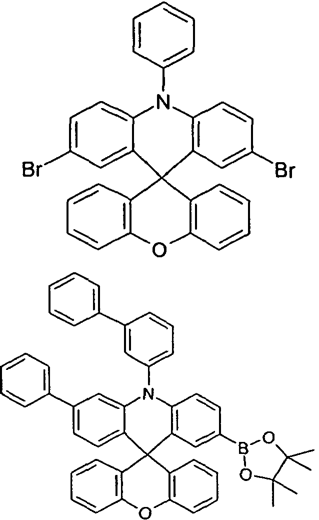 | 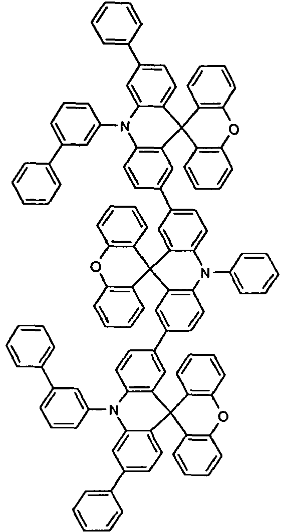 | 33 % |
| | | Verb. wird im Hochvakuum getempert, um Lösungsmittelreste zu entfernen | |
| 80 | 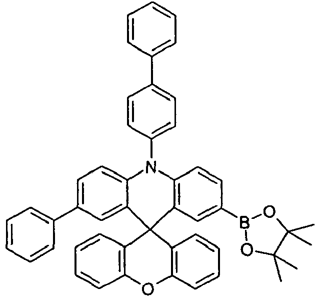 | 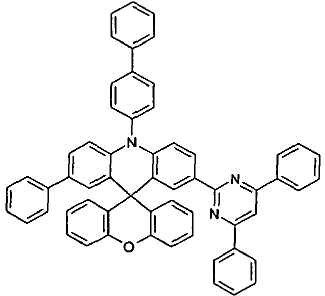 | 43 % |
| | 100 mmol | | |
| | 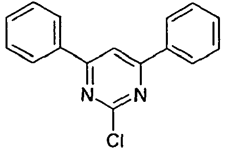 | | |
| | 2915-16-4 | | |
| | 130 mmol | | |
| 81 | 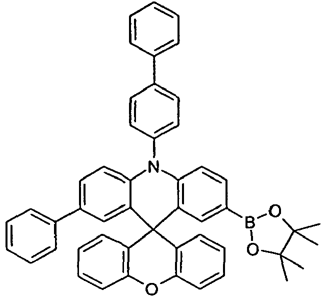 | 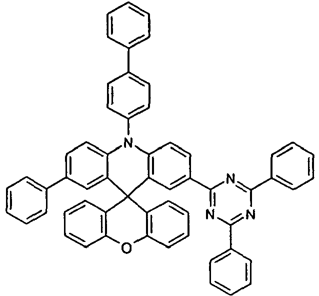 | 40 % |
| | 100 mmol | | |
| | 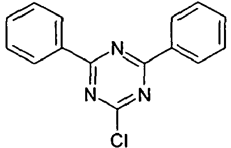 | | |
| | 3842-55-5 | | |
| | 130 mmol | | |
| 82 | 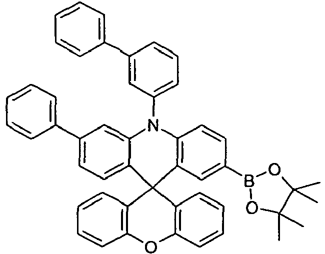 | 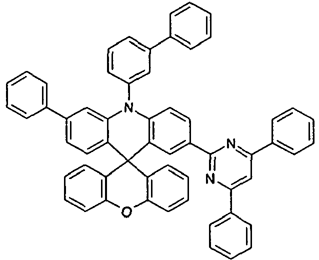 | 36 % |
| | 100 mmol | | |
| | 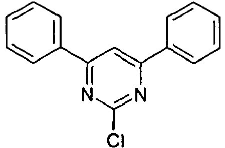 | | |
| | 2915-16-4 | | |
| | 130 mmol | | |
| 83 | 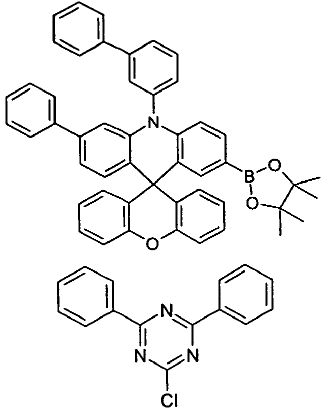 | 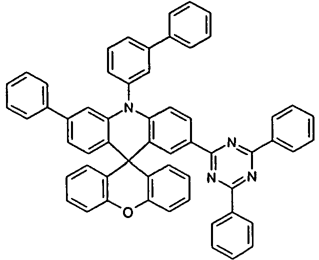 | 33 % |
| | 3842-55-5 | | |
| | 130 mmol | | |
| 84 | 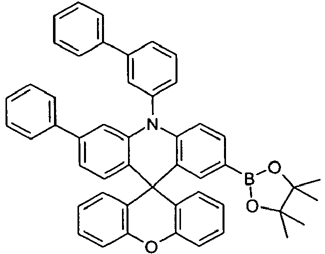 | 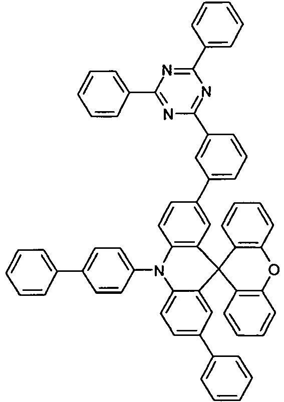 | 34 % |
| | 100 mmol | | |
| | 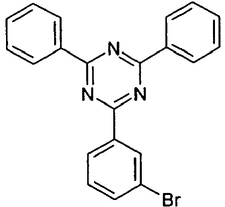 | | |
| | 864377-31-1 | | |
| | 130 mmol | | |
| 85 | 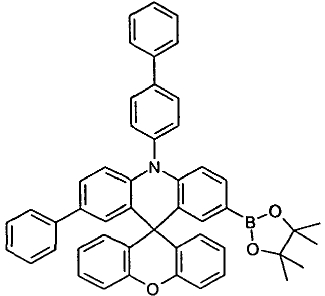 | 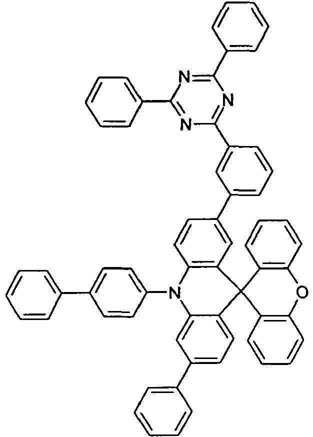 | 29 % |
| | 100 mmol | | |
| | 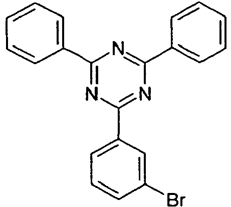 | | |
| | 864377-31-1 | | |
| | 130 mmol | | |

### Beispiel 86: 3,6-Diphenyl-10-m-biphenyl-spiro-[acridin-9(10H),9'-xanthen]

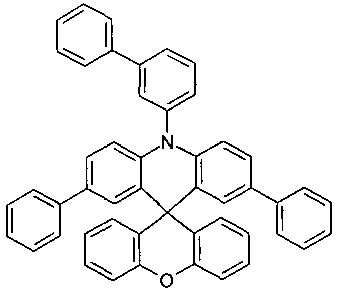

Ein Gemisch aus 50.0 g (100 mmol) 3,6-Diphenyl-spiro[acridin-9(10H),9'-xanthen], Bsp. 60, 30.3 g (130 mmol) 3-Brom-1,1'-biphenyl [2113-57-7], 14.4 g (150 mmol) Natrium-tert-butylat, 526 mg (2.6 mmol) Tri-tert-butylphosphin, 449 mg (2 mmol) Palladium(II)acetat und 500 ml o-Xylol wird 8 h unter Rückfluss erhitzt, bis das Amin verbraucht ist. Nach Abkühlen auf 60 °C wird mit 500 ml Wasser versetzt, die organische Phase wird abgetrennt, einmal mit 500 ml Wasser und einmal mit 500 ml gesättigter Kochsalzlösung gewaschen und dann über Magnesiumsulfat getrocknet. Man filtriert über ein Celite-Bett vom Trockenmittel ab, wäscht dieses mit o-Xylol nach, entfernt das o-Xylol im Vakuum und kristallisiert den Rückstand fünfmal aus Dioxan (3 ml/g) unter Zusatz von Ethanol (1-2 ml/g) in der Siedehitze um. Die weitere Reinigung erfolgt durch zweimalige fraktionierte Sublimation (p ca. 10⁻⁶ mbar, T = 300 - 320 °C). Ausbeute: 26.7 g (41 mmol), 41 %; Reinheit: > 99.9 % n. HPLC.

Analog können folgende Verbindungen erhalten werden:

| Bsp. | Edukte Amin / Bromid | Produkt | Ausbeute |
|---|---|---|---|
| 87 | 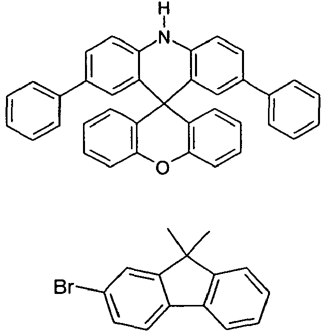 | 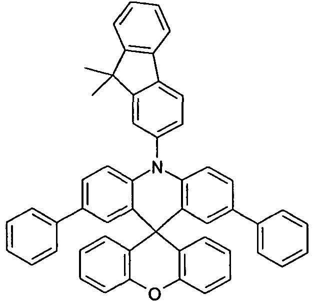 | 48 % |
| | 28320-31-2 | | |
| 88 | 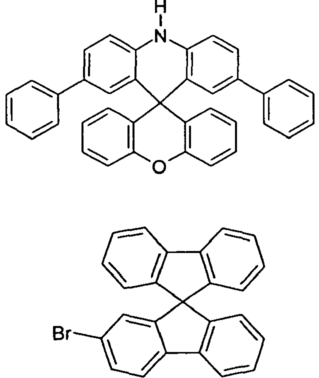 | 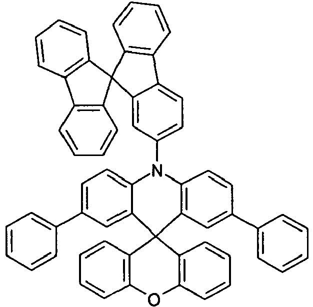 | 45 % |
| | 171408-76-7 | | |
| 89 | 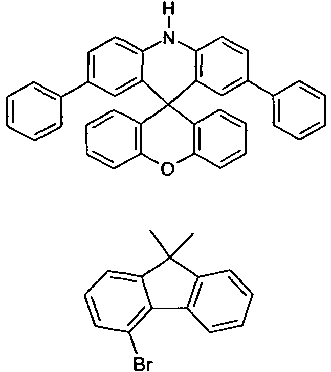 | 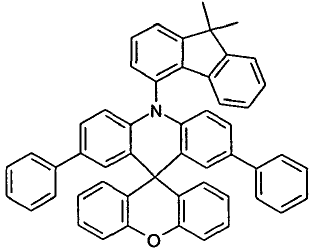 | 41 % |
| | 942615-32-9 | | |
| 90 | 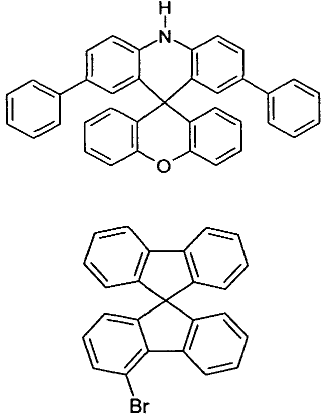 | 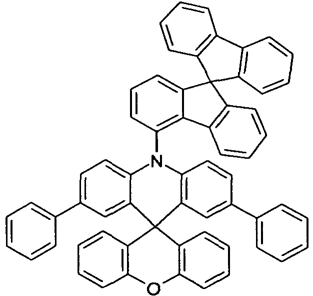 | 43 % |
| | 1161009-88-6 | | |
| 91 | 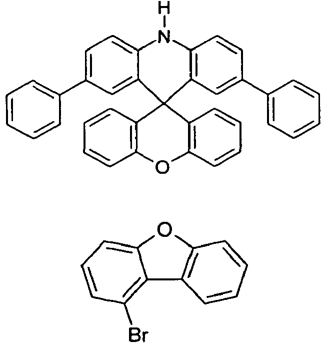 | 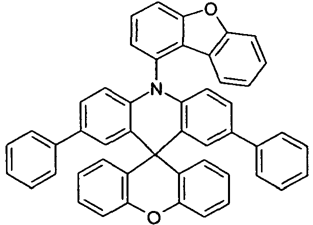 | 45 % |
| | 50548-45-3 | | |
| 92 | 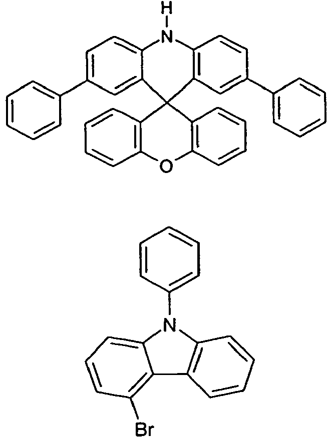 | 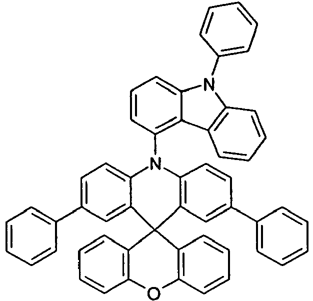 | 38 % |
| | 1097884-37-1 | | |
| 93 | 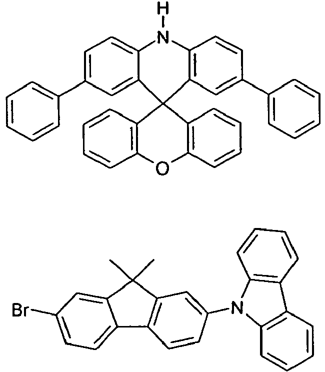 | 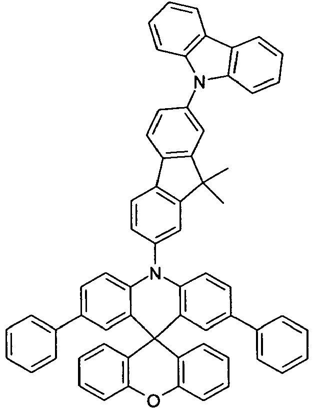 | 39 % |
| | 868549-07-9 | | |
| 94 | 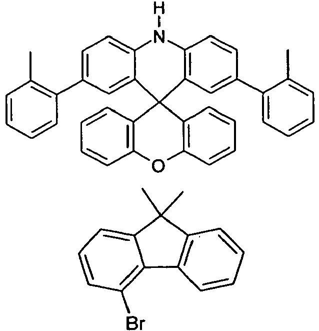 | 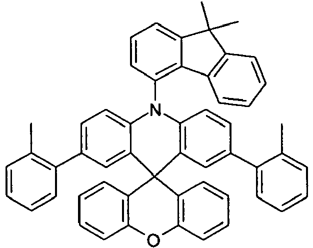 | 36 % |
| | 942615-32-9 | | |
| 95 | 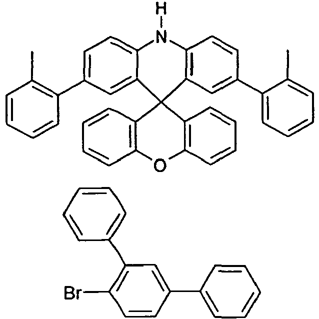 | 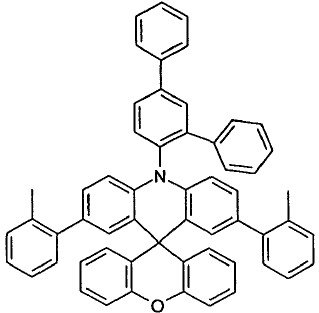 | 37 % |
| | 60631-83-6 | | |
| 96 | 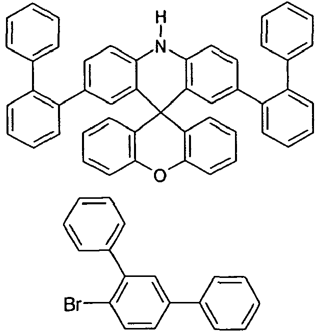 | 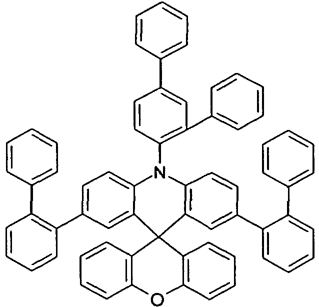 | 28 % |
| | 60631-83-6 | | |
| 97 | 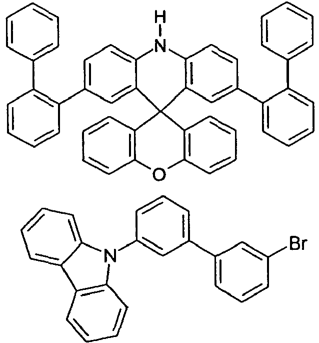 | 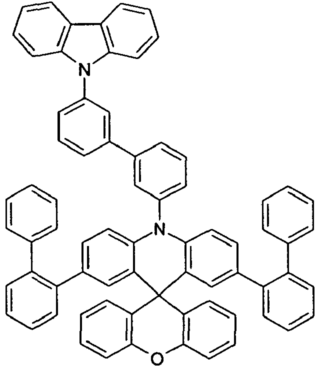 | 28 % |
| | 854952-41-3 | Verb. wird im Hochvakuum getempert, um Lösungsmittelreste zu entfernen | |
| 98 | 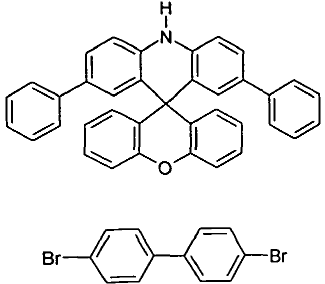 | 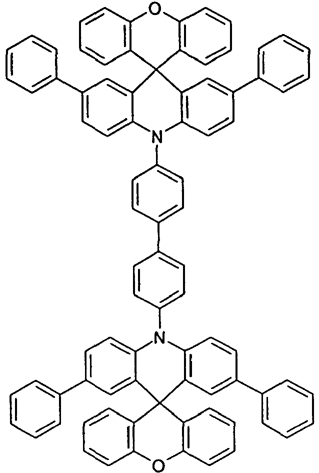 | 33 % |
| | 92-86-4 45 mmol | Verb. wird im Hochvakuum getempert, um Lösungsmittelreste zu entfernen | |
| 99 | 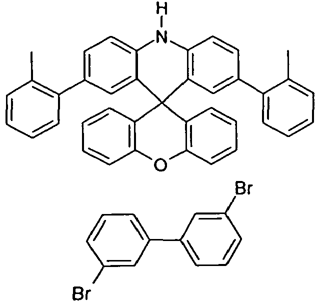 | 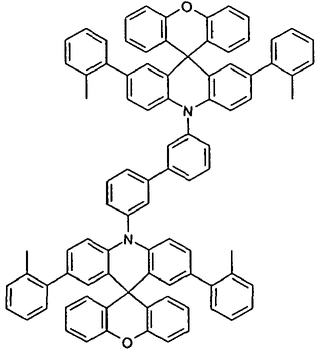 | 29 % |
| | 16400-51-4 45 mmol | Verb. wird im Hochvakuum getempert, um Lösungsmittelreste zu entfernen | |
| 100 | 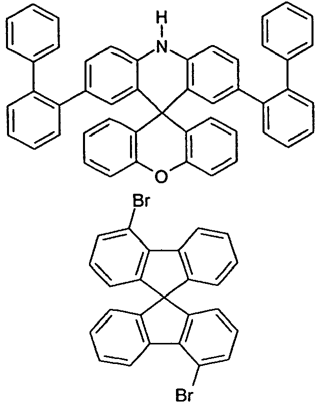 | 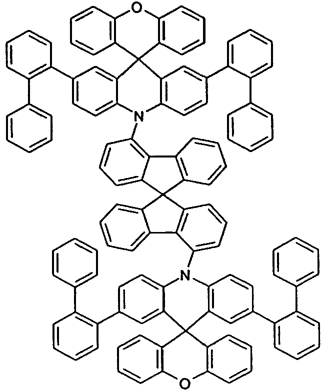 | 23 % |
| | 1257321-41-7 45 mmol | Verb. wird im Hochvakuum getempert, um Lösungsmittelreste zu entfernen | |
| 101 | 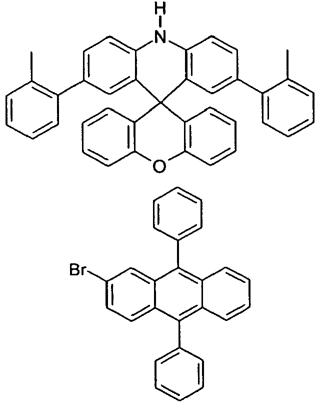 | 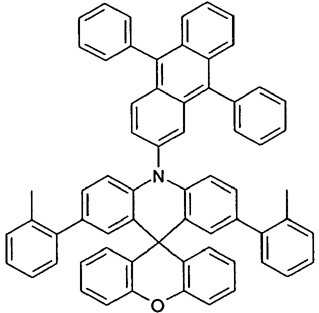 | 34 % |
| | 201731-79-5 90 mmol | | |
| 102 | 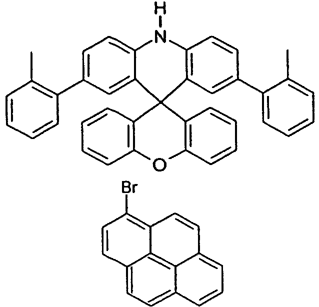 | 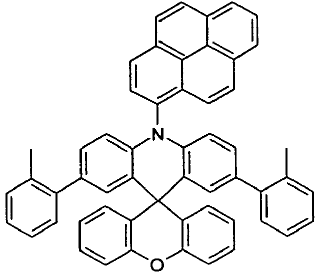 | 38 % |
| | 1714-29-0 90 mmol | | |
| 103 | 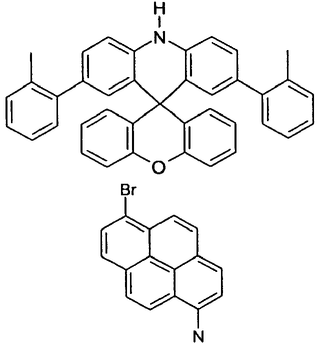 | 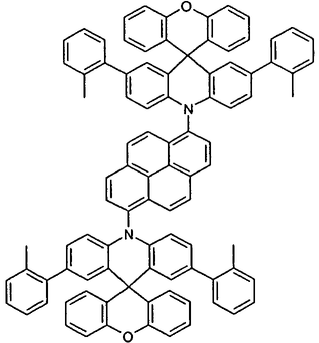 | 26% |
| | 27973-29-1 45 mmol | | |
| 104 | 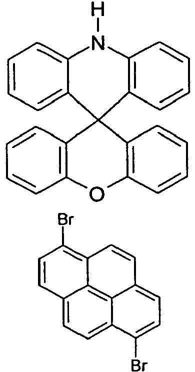 | 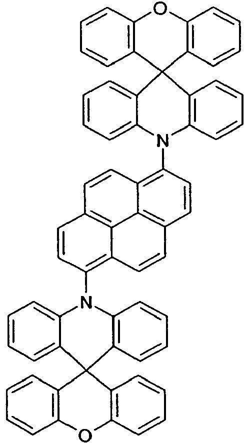 | 31 % |
| | 27973-29-1 45 mmol | | |
| 105 | 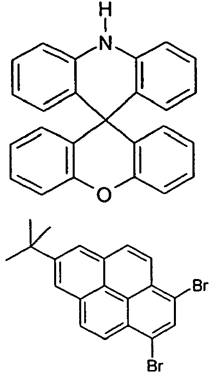 | 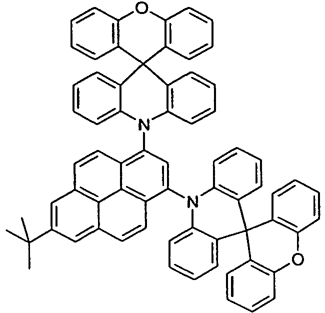 | 33 % |
| | 1005771-04-9 45 mmol | | |
| 106 | 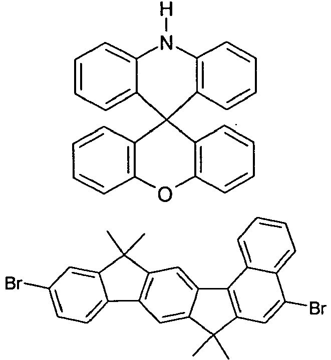 | 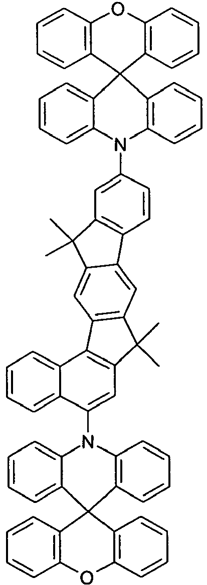 | 30 % |
| | 1001911-20-1 45 mmol | | |
| 107 | 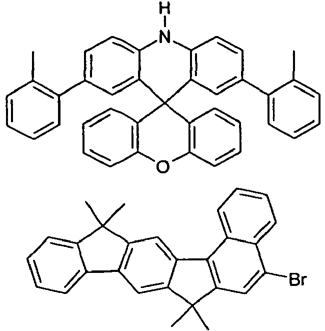 | 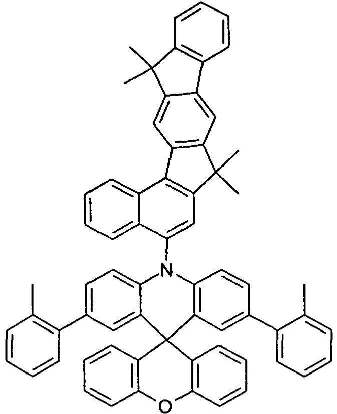 | 29 % |
| | 1001911-28-9 | | |

### Beispiel 108: 3-(4,4,5,5-Tetramethyl-[1,3,2]dioxaborolan-2-yl)-6-phenyl- 10-p-biphenyl-spiro- [acridin-9(10H),9'-xanthen]

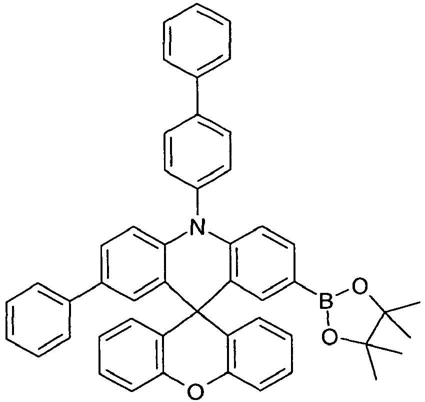

Ein Gemisch von 65.4 g (100 mmol) 3-Brom-6-phenyl-10-p-biphenyl-spiro-[acridin-9(10H),9'-xanthen], Bsp. 12, 27.9 g (110 mmol) Bis(pinacolato)-diboran [73183-34-3], 29.5 g (300 mmol) Kaliumacetat, 200 g Glaskugeln (Durchmesser 3 mm), 561 mg (2 mmol) Tricyclohexylphosphin, 249 mg (1 mmol) Palladium(II)acetat und 1000 ml Dioxan wird 16 h bei 80 °C gerührt, bis das Bromid verbraucht ist. Nach Erkalten und Entfernen des Lösungsmittels im Vakuum wird der Rückstand in 1000 ml Ethylacetat aufgenommen, über ein Celite-Bett filtriert, das Filtrat wird bis zur beginnenden Kristallisation im Vakuum eingeengt und abschießend noch tropfenweise mit ca. 100 ml Methanol versetzt, um die Kristallisation zu vervollständigen. Ausbeute: 61.0 g (87 mmol), 87 %; Reinheit: ca. 97 % n. ¹H-NMR.

Analog kann aus 3-Brom-7-phenyl-10-m-biphenyl-spiro- [acridin-9(10H),9'-xanthen], Bsp. 39, der entsprechende Boronsäureester, **Bsp. 109** dargestellt werden.

### Beispiel 110: 3,6-Diphenyl- 10-(4,6-diphenyl-pyrimidin-2-yl)-spiro-[acridin-9(10H),9'-xanthen]

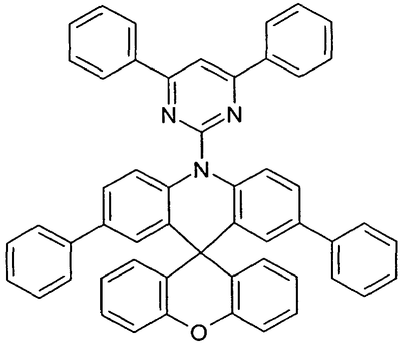

Eine Suspension von 50.0 g (100 mmol) 3,6-Diphenyl-spiro[acridin-9(10H),9'-xanthen], Bsp. 60, in 500 ml DMF wird portionsweise mit 4.8 g (120 mmol) Natriumhydrid, Dispersion in Mineralöl, 60 Gew.-% ig versetzt, und 30 min. bei Raumtemperatur gerührt. Dann tropft man eine Lösung von 32.0 g (120 mmol) 2-Chlor-4,6-diphenyl-pyrimidin [2915-16-4] in 500 ml THF während 30 min. zu und rührt 16 h bei Raumtemperatur nach. Man gießt die Reaktionsmischung unter gutem Rühren auf 2000 g Eis, saugt vom ausgefallenen Feststoff ab, wäscht diesen dreimal mit je 300 ml Methanol und dann dreimal mit je 300 ml n-Heptan und trocknet im Vakuum. Das Rohprodukt wird in 1000 ml heißem Toluol aufgenommen, über ein Celite-Bett filtriert, das Filtrat wird bis zur beginnenden Kristallisation im Vakuum eingeengt und abschießend noch tropfenweise mit ca. 100 ml Methanol versetzt, um die Kristallisation zu vervollständigen. Die Umkristallisation erfolgt aus Dioxan, gefolgt von einer zweimaligen fraktionierten Sublimation (p ca. 10⁻⁶ mbar, T = 300 - 330 °C). Ausbeute: 28.5 g (39 mmol), 39 %; Reinheit: > 99.9 % n. HPLC.

Analog können folgende Verbindungen erhalten werden:

| Bsp. | Edukte Amin / Chlorid | Produkt | Ausbeute |
|---|---|---|---|
| 111 | 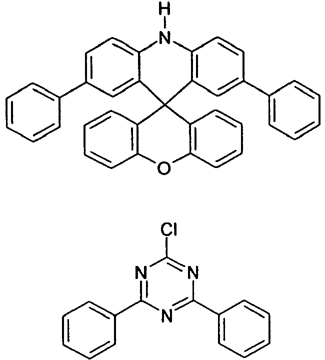 | 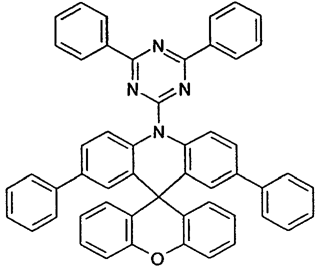 | 46 % |
| | 3842-55-5 | | |
| 112 | 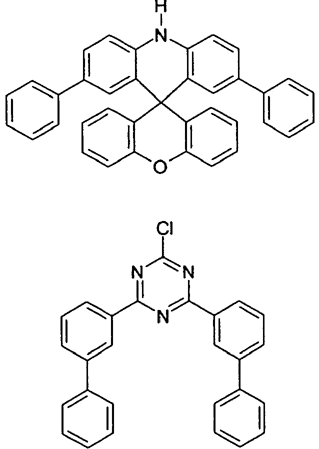 | 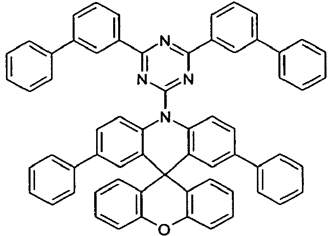 | 37 % |
| | 1205748-61-3 | | |
| 113 | 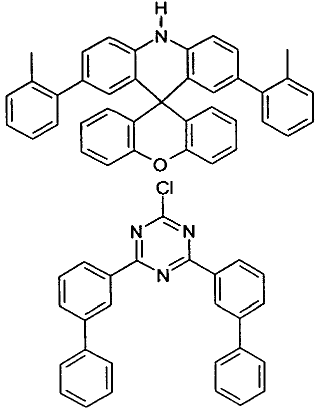 | 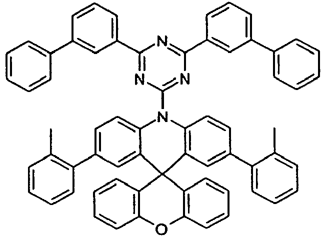 | 36 % |
| | 1205748-61-3 | | |
| 114 | 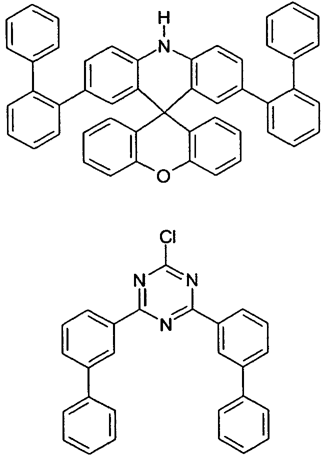 | 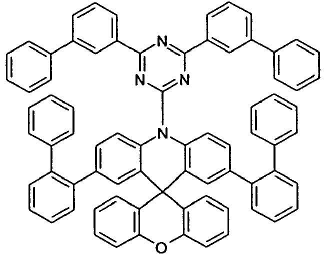 | 31 % |
| | 1205748-61-3 | | |

### Beispiel 115: 3,6-Bis(2-methylphenyl)- 10-phenyl-spiro- [acridin-9(10H),9'-(2,7-bis-diphenylphosinoyl)xanthen]

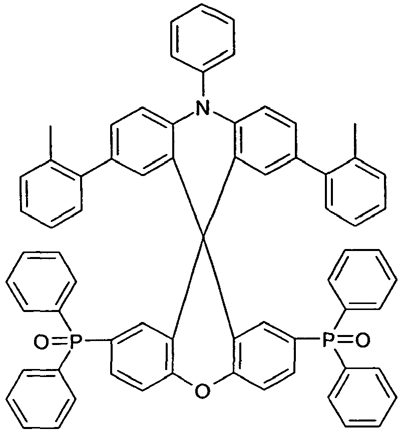

### a) 10-phenyl-spiro- [acridin-9(10H),9'-(2,7-bis-diphenylphosinoyl)-xanthen], Bsp. 116

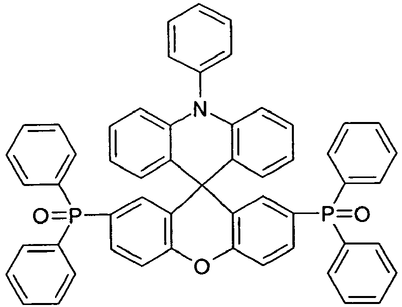

Eine auf - 78 °C gekühlte, gut gerührte Lösung von 58.1 g (100 mmol) 10-phenyl-spiro- [acridin-9(10H),9'-(2,7-dibrom)xanthen], Bsp. 28, in 1000 ml THF wird tropfenweise mit 80.0 ml (200 mmol) n-BuLi, 2.5 molar in n-Hexan versetzt und dann 1 h nachgerührt. Dann tropft man eine Mischung aus 48.5 g (220 mmol) Chlor-diphenylphosphin [1079-66-7] und 100 ml THF langsam zu, rührt 30 min. nach, lässt auf Raumtemperatur erwärmen, entfernt dann das THF im Vakuum, nimmt den Rückstand in 1000 ml Ethylacetat auf und fügt unter sehr gutem Rühren ein Gemisch aus 100 ml Wasser und 20.6 ml (200 mmol) 30 Gew.-% iges Wasserstoffperoxid zu. Nach 16 h Rühren wird die Reaktionsmischung im Vakuum auf ca. 200 ml eingeengt, dann mit 300 ml Ethanol versetzt und weiter 2 h gerührt. Man saugt vom ausgefallenen Feststoff ab, wäscht diesen mit Ethanol und trocknet im Vakuum. Der so erhaltene Feststoff wird einmal aus DMF / Ethanol umkristallisiert. Ausbeute: 48.6 g (59 mmol), 59 %; Reinheit: > 98 % n. 1H-NMR.

### b) 3,6-Dibrom-10-phenyl-spiro- [acridin-9(10H),9'-(bis-2,7-diphenylphosinoyl)xanthen]

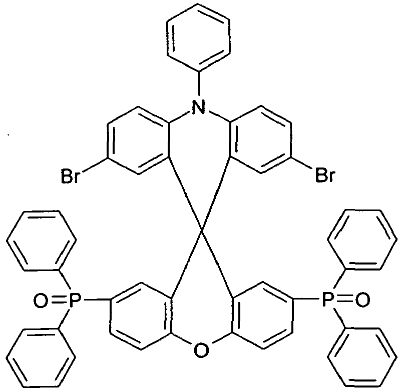

NBS-Bromierung: Durchführung analog Bsp. 11. Ansatz 41.2 g (50 mmol) 10-phenyl-spiro- [acridin-9(10H),9'-(bis-2,7-diphenylphosinoyl)xanthen], Stufe a) und 19.6 g (110 mmol) NBS. Ausbeute: 47.1 g (48 mmol), 96 %; Reinheit: > 98 % n. ¹H-NMR.

### c) 3,6-Bis(2-methylphenyl)- 10-phenyl-spiro- [acridin-9(10H),9'-(bis-2,7-diphenylphosinoyl)xanthen]

Suzuki-Kupplung: Durchführung analog Bsp. 14. Ansatz 47.1 g (48 mmol) 3,6-Dibrom-10-phenyl-spiro- [acridin-9(10H),9'-(bis-2,7-diphenylphosinoyl)-xanthen], Stufe b) und 17.0 g (125 mmol) 2-Tolylboronsäure [16419-60-6]. Ausbeute: 17.0 g (17 mmol), 35 %; Reinheit: > 99.9 % n. HPLC.

Analog können folgende Verbindungen erhalten werden:

| Bsp. | Edukte Phosphin bzw. Boran / Boronsäure | Produkt | Ausbeute |
|---|---|---|---|
| 117 | 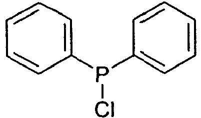 | 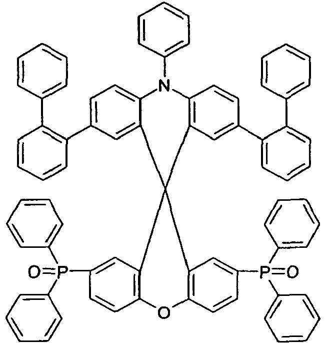 | 36 % |
| | 1079-66-9 | | |
| | 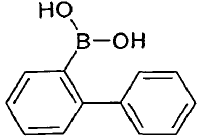 | | |
| | 4688-76-0 | | |
| 118 | 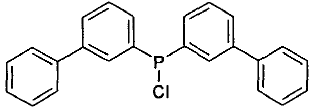 | 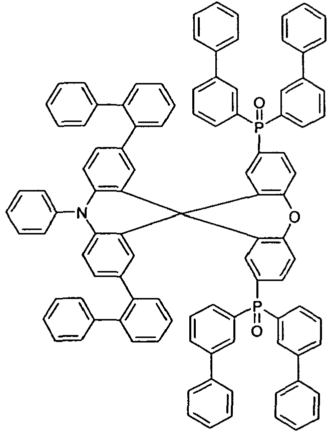 | 27 % |
| | 1190100-29-8 | | |
| | 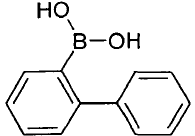 | | |
| | 4688-76-0 | Verb. wird im Hochvakuum getempert, um Lösungsmitte/reste zu entfernen | |
| 119 | 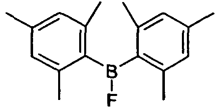 | 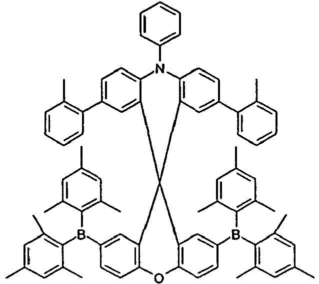 | 33 % |
| | 436-59-9 | | |
| | Auf die H₂O₂-Oxidation wird verzichtet | | |
| | 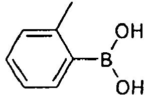 | | |
| | 16419-60-6 | | |

### Beispiel 120:

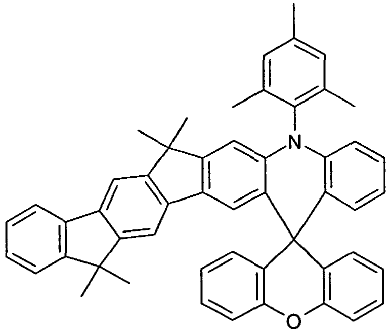

### a) (6,6,12,12-Tetramethyl-6,12-dihydro-indeno[1,2-b]fluoren-2yl)-(2,4,6-trimethylphenyl)amin

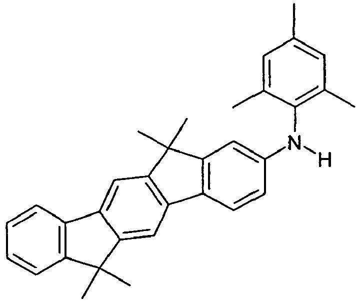

Ein Gemisch aus 38.9 g (100 mmol) 2-Brom-6,6,12,12-Tetramethyl-6,12-dihydro-indeno[1,2-b]fluoren, 13.5 g (100 mmol) 2,4,6-Trimethylphenylamin, 12.5 g (130 mmol) Natrium-tert-butylat und 500 ml Toluol wird mit 404 mg (2 mmol) Tri-tert-butyl-phosphin und dann mit 225 mg (1 mmol) Palladium(II)acetat versetzt und 16 h unter Rückfluss erhitzt. Nach Erkalten gibt man 300 ml Wasser zu, trennt die org. Phase ab, trocknet diese über Magnesiumsulfat, filtriert dann über ein Celite-Bett ab und entfernt das Toluol im Vakuum. Ausbeute: 38.7 g (87 mmol), 87 %; Reinheit: ca. 95 % n. ¹H-NMR.

### b) (2-Bromphenyl)-(6,6,12,12-tetramethyl-6,12-dihydro-indeno[1,2-b]fluoren-2yl)-(2,4,6-trimethylphenyl)amin

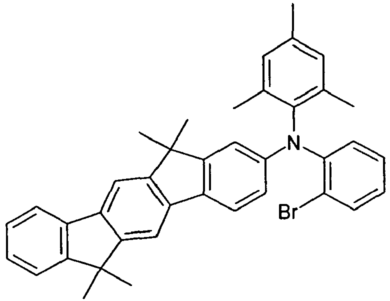

Buchwald-Kupplung: Durchführung analog Bsp. 1. Ansatz 22.2 g (50 mmol) (6,6,12,12-Tetramethyl-6,12-dihydro-indeno[1,2-b]fluoren-2yl)-(2,4,6-trimethylphenyl)amin, Stufe a). Ausbeute: 19.8 g (33 mmol), 66 %; Reinheit: > 98 % n. ¹H-NMR.

### c)

Durchführung analog Bsp. 6. Ansatz 18.0 g (30 mmol) (2-Bromphenyl)-(6,6,12,12-Tetramethyl-6,12-dihydro-indeno[1,2-b]fluoren-2yl)-(2,4,6-trimethylphenyl)-amin, Stufe b). Reinigung durch viermalige Umkristallisation aus DMF und zweimalige fraktionierte Sublimation (p ca. 10⁻⁶ mbar, T = 300 - 320 °C). Ausbeute: 6.3 g (9 mmol), 30 %, Reinheit: > 99.9 % n. HPLC.

Analog können folgende Verbindungen erhalten werden:

| Bsp. | Edukte | Produkt | Ausbeute |
|---|---|---|---|
| | Bromid - Stufe a) | | |
| | Brom-iod-aromat Stufe b) | | |
| 121 | 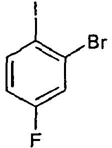 | 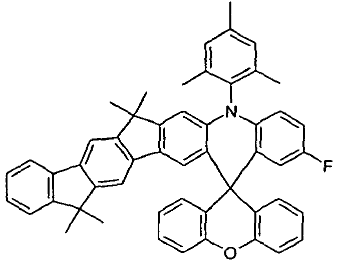 | 26 % |
| | 202865-73-4 | | |
| 122 | 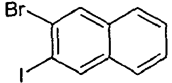 | 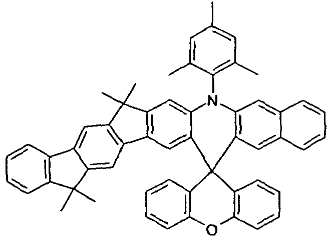 | 27 % |
| | 102153-44-6 | | |
| 123 | 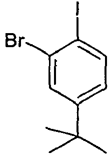 | 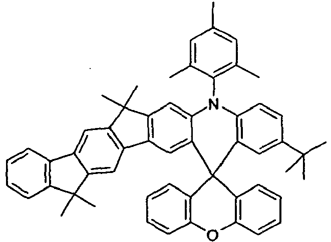 | 29 % |
| | 860435-39-8 | | |
| 124 | 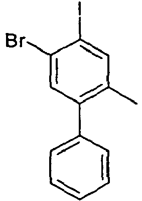 | 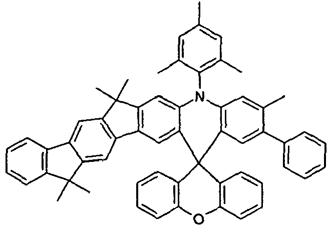 | 25 % |
| | 1238830-99-3 | | |
| 125 | 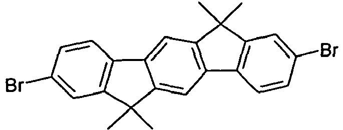 | 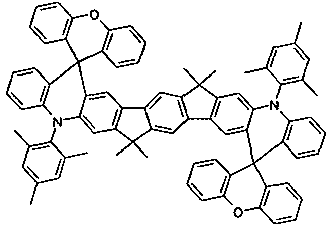 | 33 % |
| | 872705-64-1 | | |
| | 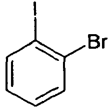 | | |
| | 583-55-1 | | |
| 126 | 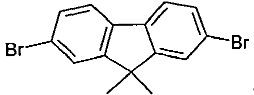 | 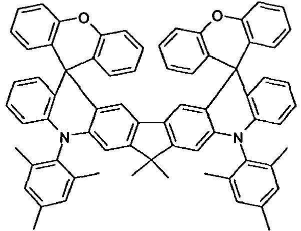 | 30 % |
| | 28320 -32-3 | | |
| | 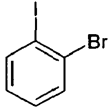 | | |
| | 583-55-1 | | |

### Herstellung der OLEDs

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen (siehe Tabellen 1 bis 11) werden der Aufbau sowie die Daten verschiedener OLEDs vorgestellt. Glasplättchen, die mit strukturiertem ITO (Indium Zinn Oxid) der Dicke 150 nm beschichtet sind, werden zur verbesserten Prozessierung mit 20 nm PEDOT beschichtet (Poly(3,4-ethylendioxy-2,5-thiophen), aus Wasser aufgeschleudert; bezogen von H. C. Starck, Goslar, Deutschland). Diese beschichteten Glasplättchen bilden die Substrate für die Versuche V1, V2 und D1-D15, solche die keine zusätzliche PEDOT-Schicht aufweisen, werden in den Beispielen D16-D119 und in D-W1 verwendet. Auf ihnen werden die OLEDs aufgebracht.

Alle Materialien werden in einer Vakuumkammer thermisch aufgedampft. Die zur Herstellung der OLEDs benötigten Materialien sind in Tabelle 12 gezeigt. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1(95%):SEB(5%) bedeutet hierbei, dass das Material H1 in einem Volumenanteil von 95% und SEB1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U @ 1000 cd/m² in Tabelle 2 und 4 bezeichnet die Spannung, die für eine Leuchtdichte von 1000 cd/m² benötigt wird. EQE @ 1000 cd/m² schließlich bezeichnet die externe Quanteneffizienz bei einer Betriebsleuchtdichte von 1000 cd/m². LD80 @ 6000 cd/m2 ist die Lebensdauer bis das OLED bei einer Helligkeit von 6000 cd/m² auf 80 % der Anfangsintensität, also auf 4800 cd/m² abgefallen ist.

### Verwendung der erfindungsgemäßen Verbindungen in fluoreszierenden und phosphoreszierenden OLEDs

Die OLEDs haben prinzipiell folgenden Schichtaufbau: Substrat / optionale Lochinjektionsschicht (IL) / Lochtransportschicht (HTL) / Zwischenschicht (IL) / Lochtransportschicht (HTL2) / Elektronenblockerschicht (EBL) / Emissionsschicht (EML) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist den Tabellen zu entnehmen.

Die erfindungsgemäßen Verbindungen eignen sich insbesondere als HTM (Lochtransportmaterial) oder EBM (Elektronenblockiermaterial) in OLEDs. Sie eignen sich zur Verwendung in einer Einzelschicht, aber auch als Mixed-Komponente als HTM, EBM oder als Bestandteil der emittierenden Schicht. Verglichen mit Vergleichs-Vorrichtungen gemäß dem Stand der Technik (V1 und V2) zeigen alle Proben mit den erfindungsgemäßen Verbindungen höhere Effizienzen und/oder verbesserte Lebensdauern. Im Vergleich zum Referenzmaterial NPB zeigen die erfindungsgemäßen Verbindungen bessere Effizienzen und bessere Lebensdauern.

| **Tabelle 1: Aufbau der OLEDs** | | | | | | | |
|---|---|---|---|---|---|---|---|
| **Bsp.** | **IL** | **HTL** | **IL** | **HTL2** | **EBL** | **EML** | **ETL** |
| | Dicke / nm | Dicke / nm | Dicke / nm | Dicke / nm | Dicke / nm | Dicke / nm | Dicke / nm |
| V1 | HIL1 | HIL2 | HIL1 | NPB | NPB | H1(95%):SEB1(5%) | ETM1(50%):LiQ(50%) |
| | 5 nm | 130 nm | 5 nm | 10 nm | 20 nm | 20 nm | 30 nm |
| D1 | HIL1 | HIL2 | HIL1 | NPB | Bsp.15 | H1(95%):SEB1(5%) | ETM1(50%):LiQ(50%) |
| | 5 nm | 130 nm | 5 nm | 10 nm | 20 nm | 20 nm | 30 nm |
| D2 | HIL1 | HIL2 | HIL1 | - | Bsp. 15 | H1(95%):SEB1(5%) | ETM1(50%):LiQ(50%) |
| | 5 nm | 130 nm | 5 nm | | 30 nm | 20 nm | 30 nm |
| D3 | HIL1 | HIL2 | HIL1 | NPB | Bsp.17 | H1(95%):SEB1(5%) | ETM1(50%):LiQ(50%) |
| | 5 nm | 130 nm | 5 nm | 10 nm | 20 nm | 20 nm | 30 nm |
| D4 | HIL1 | HIL2 | HIL1 | - | Bsp.17 | H1(95%):SEB1(5%) | ETM1(50%):LiQ(50%) |
| | 5 nm | 130 nm | 5 nm | | 30 nm | 20 nm | 30 nm |

| **Tabelle 2: Daten der OLEDs** | | | | | |
|---|---|---|---|---|---|
| **Bsp.** | **U@ 1000 cd/m2** | **EQE @ 1000 cd/m2** | **LD80 @ 6000 cd/m²** | **CIE** | |
| | V | % | [h] | x | y |
| V1 | 4.7 | 4.8 | 70 | 0.14 | 0.17 |
| D1 | 4.4 | 6.2 | 70 | 0.14 | 0.16 |
| D2 | 4.3 | 6.6 | 80 | 0.14 | 0.16 |
| D3 | 4.4 | 7.4 | 90 | 0.14 | 0.16 |
| D4 | 4.4 | 7.3 | 105 | 0.14 | 0.16 |

| **Tabelle 3: Aufbau der OLEDs** | | | | | | |
|---|---|---|---|---|---|---|
| **Bsp.** | **HTL** | **IL** | **HTL2** | **EBL** | **EML** | **ETL** |
| | Dicke / nm | Dicke / nm | Dicke / nm | Dicke / nm | Dicke / nm | Dicke / nm |
| V2 | HIL2 | HIL1 | - | NPB | H2(88%):lrpy(12%) | ETM1(50%):LiQ(50%) |
| | 70 nm | 5 nm | | 90 nm | 30 nm | 40 nm |
| D5 | HIL2 | HIL1 | NPB | Bsp.15 | H2(88%):Irpy(12%) | ETM1(50%):LiQ(50%) |
| | 70 nm | 5 nm | 10 nm | 80 nm | 30 nm | 40 nm |
| D6 | HIL2 | HIL1 | NPB | Bsp.17 | H2(88%):Irpy(12%) | ETMI(50%):LiO(50%) |
| | 70 nm | 5 nm | 10 nm | 80 nm | 30 nm | 40 nm |
| D7 | HIL2 | HIL1 | - | Bsp.15 | H2(70%): Bsp.7(25%) :Irpy(5%) | ETM1(50%):LiQ(50%) |
| | 70 nm | 5 nm | | 90 nm | 30 nm | 40 nm |
| D8 | HIL2 | HIL1 | - | Bsp.15 | H2(70%): Bsp.8(25%) :Irpy(5%) | ETM1(50%):LiQ(50%) |
| | 70 nm | 5nm | | 90 nm | 30 nm | 40 nm |
| D9 | HIL2 | HIL1 | - | Bsp.15 | H2(70%): Bsp.9(25%) :Irpy(5%) | ETM1(50%):LiQ(50%) |
| | 70 nm | 5 nm | | 90 nm | 30 nm | 40 nm |
| D10 | HIL2 | HIL1 | - | Bsp.15 | H2(75%): Bsp.10(20%) :Irpy(5%) | ETM1(50%):LiQ(50%) |
| | 70 nm | 5 nm | | 90 nm | 30 nm | 40 nm |
| D11 | HIL2 | HIL1 | - | Bsp.15 | H2(50%): Bsp14(40%) | ETM1(50%):LiQ(50%) |
| | 70 nm | 5 nm | | 90 nm | :Irpy(10%) 30 nm | 40 nm |
| D12 | HIL2 | HIL1 | - | Bsp.15 | H2(70%): Bsp15(25%) :Irpy(5%) | ETM1(50%):LiQ(50%) |
| | 70 nm | 5 nm | | 90 nm | 30 nm | 40 nm |
| D13 | HIL2 | HIL1 | - | Bsp.15 | H2(60%): Bsp16(35%) :Irpy(5%) | ETM1(50%):LiQ(50%) |
| | 70 nm | 5 nm | | 90 nm | 30 nm | 40 nm |
| D14 | HIL2 | HIL1 | - | Bsp.15 | H2(75%): Bsp17(20%) | ETM1(50%):LiQ(50%) |
| | 70 nm | 5 nm | | 90 nm | :Irpy(5%) 30 nm | 40 nm |
| D15 | HIL2 | HIL1 | - | Bsp.15 | H2(45%): Bsp18(50%) :Irpy(15%) | ETM1(50%):LiQ(50%) |
| | 70 nm | 5 nm | | 90 nm | 30 nm | 40 nm |

| **Tabelle 4: Daten der OLEDs** | | | | | |
|---|---|---|---|---|---|
| **Bsp.** | **U @ 1000 cd/m2** | **EQE @ 1000 cd/m2** | **LD80 @ 8000 cd/m²** | **CIE** | |
| | V | % | [h] | x | y |
| V2 | 3.6 | 14.4 | 85 | 0.32 | 0.63 |
| D5 | 3.2 | 17.9 | 120 | 0.33 | 0.63 |
| D6 | 3.4 | 18.4 | 110 | 0.33 | 0.63 |
| D7 | 3.2 | 18.8 | 140 | 0.33 | 0.64 |
| D8 | 3.3 | 19.0 | 130 | 0.33 | 0.63 |
| D9 | 3.2 | 18.0 | 110 | 0.33 | 0.64 |
| D10 | 3.2 | 18.2 | 115 | 0.33 | 0.63 |
| D11 | 3.4 | 18.7 | 100 | 0.33 | 0.63 |
| D12 | 3.2 | 16.5 | 145 | 0.33 | 0.64 |
| D13 | 3.3 | 18.8 | 125 | 0.33 | 0.64 |
| D14 | 3.2 | 17.0 | 160 | 0.33 | 0.63 |
| D15 | 3.3 | 18.2 | 120 | 0.33 | 0.63 |

### Verwendung der erfindungsgemäßen Verbindungen in fluoreszierenden und phosphoreszierenden OLEDs mit p-dotiertem Lochtransportschicht-aufbau

OLEDs mit p-dotierten Lochtransportschichten haben folgenden Aufbau: Substrat / Lochinjektionsschicht (HTL1, 20 nm) / Lochtransportschicht (HIL3, 140 nm für Devices mit blauem Singulett-Emitter bzw. 190 nm für Devices mit grünem oder gelben Triplet-Emitter) / Lochtransportschicht (erfindungsgemäßes HTL, dotiert mit NPD-9 (3 %) der Fa. Novaled, 20 nm) / Lochtransportschicht (erfindungsgemäßes HTL, 20 nm) / Emissionsschicht (EML, 20 nm bzw. 40 nm, s. Tabellen) / Lochblockierschicht (HBL, bei blauen Triplet Devices, 10 nm, s. Tabellen) Elektronentransportschicht (ETL, 30 nm) / optionale Elektroneninjektionsschicht (EIL) und abschließend eine Kathode. Die Kathode wird durch eine 100 nm dicke Aluminiumschicht gebildet. Der genaue Aufbau der OLEDs und die Ergebnisse ist den Tabellen 5 bis 8 zu entnehmen. Die zur Herstellung der OLEDs benötigten Materialien sind in Tabelle 12 gezeigt.

| **Tabelle 5: Aufbau der OLEDs** | | | | |
|---|---|---|---|---|
| **Bsp.** | **HTL 3 % NDP-9** | **HTL** | **EML** | **ETL** |
| | 20 nm | 20 nm | 20 nm | 30 nm |
| D16 | Bsp.7 | Bsp.7 | H1(95%):SEB1(5%) | ETM1(50%):LiQ(50%) |
| D17 | Bsp.8 | Bsp.8 | H1(95%):SEB1(5%) | ETM1(50%):LiQ(50%) |
| D18 | Bsp.9 | Bsp.9 | H1(95%):SEB1(5%) | ETM1(50%):LiQ(50%) |
| D19 | Bsp.10 | Bsp.10 | H1(95%):SEB1(5%) | ETM1(50%):LiQ(50%) |
| D20 | Bsp.17 | Bsp.17 | H1(95%):SEB1(5%) | ETM1(50%):LiQ(50%) |
| D21 | Bsp.18 | Bsp.18 | H1(95%):SEB1(5%) | ETM1(50%):LiQ(50%) |
| D22 | Bsp.24 | Bsp.25 | H1(95%):SEB1(5%) | ETM1(50%):LiQ(50%) |
| D23 | Bsp.25 | Bsp.25 | H1(95%):SEB1(5%) | ETM1(50%):LiQ(50%) |
| D24 | Bsp.52 | Bsp.52 | H1(95%):SEB1(5%) | ETM1(50%):LiQ(50%) |
| D25 | Bsp.53 | Bsp.53 | H1(95%):SEB1(5%) | ETM1(50%):LiQ(50%) |
| D26 | Bsp.54 | Bsp.54 | H1(95%):SEB1(5%) | ETM1(50%):LiQ(50%) |
| D27 | Bsp.55 | Bsp.55 | H1(95%):SEB1(5%) | ETM1(50%):LiQ(50%) |
| D28 | Bsp.58 | Bsp.58 | H1(95%):SEB1(5%) | ETM1(50%):LiQ(50%) |
| D29 | Bsp.59 | Bsp.59 | H1(95%):SEB1(5%) | ETM1(50%):LiQ(50%) |
| D30* | Bsp.66 | Bsp.66 | H1(95%):SEB1(5%) | ETM1(50%):LiQ(50%) |
| D31* | Bsp.67 | Bsp.67 | H1(95%):SEB1(5%) | ETM1(50%):LiQ(50%) |
| D32* | Bsp.68 | Bsp.68 | H1(95%):SEB1(5%) | ETM1(50%):LiQ(50%) |
| D33 | Bsp.69 | Bsp.69 | H1(95%):SEB1(5%) | ETM1(50%):LiQ(50%) |
| D34 | Bsp.71 | Bsp.71 | H1(95%):SEB1(5%) | ETM1(50%):LiQ(50%) |
| D35 | Bsp.72 | Bsp.72 | H1(95%):SEB1(5%) | ETM1(50%):LiQ(50%) |
| D36 | Bsp.73 | Bsp.73 | H1(95%):SEB1(5%) | ETM1(50%):LiQ(50%) |
| D37 | Bsp.74 | Bsp.74 | H1(95%):SEB1(5%) | ETM1(50%):LiQ(50%) |
| D38 | Bsp.77 | Bsp.77 | H1(95%):SEB1(5%) | ETM1(50%):LiQ(50%) |
| D39 | Bsp.86 | Bsp.86 | H1(95%):SEB1(5%) | ETM1(50%):LiQ(50%) |
| D40 | Bsp.87 | Bsp.87 | H1(95%):SEB1(5%) | ETM1(50%):LiQ(50%) |
| D41 | Bsp.88 | Bsp.88 | H1(95%):SEB1(5%) | ETM1(50%):LiQ(50%) |
| D42 | Bsp.89 | Bsp.89 | H1(95%):SEB1(5%) | ETM1(50%):LiQ(50%) |
| D43 | Bsp.90 | Bsp.90 | H1(95%):SEB1(5%) | ETM1(50%):LiQ(50%) |
| D45 | Bsp.91 | Bsp.81 | H1(95%):SEB1(5%) | ETM1(50%):LiQ(50%) |
| D46 | Bsp.92 | Bsp.92 | H1(95%):SEB1(5%) | ETM1(50%):LiQ(50%) |
| D47 | Bsp.93 | Bsp.93 | H1(95%):SEB1(5%) | ETM1(50%):LiQ(50%) |
| D48 | Bsp.94 | Bsp.94 | H1(95%):SEB1(5%) | ETM1(50%):LiQ(50%) |
| D49 | Bsp.95 | Bsp.95 | H1(95%):SEB1(5%) | ETM1(50%):LiQ(50%) |
| D50 | Bsp.96 | Bsp.96 | H1(95%):SEB1(5%) | ETM1(50%):LiQ(50%) |
| D51 | Bsp.69 | Bsp.69 | H1(95%):Bsp.101(5%) | ETM1(50%):LiQ(50%) |
| D52 | Bsp.69 | Bsp.69 | H1(95%):Bsp.102(5%) | ETM1(50%):LiQ(50%) |
| D53 | Bsp.72 | Bsp.72 | H1(95%):Bsp.103(5%) | ETM1(50%):LiQ(50%) |
| D54 | Bsp.88 | Bsp.88 | H1(95%):Bsp.104(5%) | ETM1(50%):LiQ(50%) |
| D55 | Bsp.69 | Bsp.69 | H1(95%):Bsp.105(5%) | ETM1(50%):LiQ(50%) |
| D56 | Bsp.90 | Bsp.90 | H1(95%):Bsp.106(5%) | ETM1(50%):LiQ(50%) |
| D57 | Bsp.69 | Bsp.69 | H1(95%):Bsp.107(5%) | ETM1(50%):LiQ(50%) |
| D58 | Bsp.90 | Bsp.90 | H1(95%):Bsp.120(5%) | ETM1(50%):LiQ(50%) |
| D59 | Bsp.90 | Bsp.90 | H1(95%):Bsp.121(5%) | ETM1(50%):LiQ(50%) |
| D60 | Bsp.90 | Bsp.90 | H1(95%):Bsp.122(5%) | ETM1(50%):LiQ(50%) |
| D61 | Bsp.69 | Bsp.69 | H1(95%):Bsp.123(5%) | ETM1(50%):LiQ(50%) |
| D62 | Bsp.69 | Bsp.69 | H1(95%):Bsp.124(5%) | ETM1(50%):LiQ(50%) |
| D63 | Bsp.90 | Bsp.90 | H1(95%):Bsp.125(5%) | ETM1(50%):LiQ(50%) |
| D64 | Bsp.90 | Bsp.90 | H1(95%):Bsp.126(5%) | ETM1(50%):LiQ(50%) |

| **Tabelle 6: Daten der OLEDs** | | | | | |
|---|---|---|---|---|---|
| **Bsp.** | **U @ 1000 cd/m2** | **EQE @ 1000 cd/m2** | **LD80 @ 1000 cd/m²** | **CIE** | |
| | V | % | [h] | x | y |
| D16 | 3.9 | 8.2 | 3300 | 0.14 | 0.14 |
| D17 | 4.0 | 8.3 | 3200 | 0.14 | 0.14 |
| D18 | 3.8 | 8.4 | 3900 | 0.14 | 0.13 |
| D19 | 3.9 | 7.6 | 2700 | 0.14 | 0.14 |
| D20 | 4.0 | 8.0 | 4600 | 0.14 | 0.14 |
| D21 | 3.9 | 8.1 | 4400 | 0.14 | 0.15 |
| D22 | 3.9 | 8.0 | 3100 | 0.14 | 0.14 |
| D23 | 3.8 | 8.1 | 4500 | 0.14 | 0.14 |
| D24 | 3.9 | 7.4 | 4000 | 0.14 | 0.14 |
| D25 | 3.8 | 7.5 | 2000 | 0.14 | 0.16 |
| D26 | 4.2 | 7.8 | 3800 | 0.14 | 0.14 |
| D27 | 4.3 | 7.9 | 3400 | 0.14 | 0.14 |
| D28 | 3.8 | 7.4 | 4100 | 0.14 | 0.14 |
| D29 | 3.9 | 7.1 | 4300 | 0.14 | 0.14 |
| D30_* | 4.0 | 8.2 | 4100 | 0.14 | 0.14 |
| D31_* | 4.2 | 8.3 | 4200 | 0.14 | 0.14 |
| D32_* | 4.4 | 7,7 | 3600 | 0.14 | 0.14 |
| D33 | 3.9 | 7.6 | 4200 | 0.14 | 0.14 |
| D34 | 4.0 | 7.0 | 2200 | 0.14 | 0.14 |
| D35 | 3.9 | 7.5 | 4200 | 0.14 | 0.14 |
| D36 | 3.8 | 7.7 | 4100 | 0.14 | 0.14 |
| D37 | 3.8 | 7.8 | 4000 | 0.14 | 0.14 |
| D38 | 4.0 | 7.8 | 4500 | 0.14 | 0.14 |
| D39 | 3.9 | 7.8 | 4200 | 0.14 | 0.14 |
| D40 | 3.8 | 8.1 | 4400 | 0.14 | 0.13 |
| D41 | 4.1 | 8.3 | 4600 | 0.14 | 0.14 |
| D42 | 3.8 | 8.1 | 4900 | 0.14 | 0.13 |
| D43 | 3.9 | 8.0 | 5100 | 0.14 | 0.14 |
| D45 | 4.0 | 7.8 | 3900 | 0.14 | 0.14 |
| D46 | 4.0 | 7.9 | 3700 | 0.14 | 0.14 |
| D47 | 4.0 | 7.6 | 3000 | 0.14 | 0.14 |
| D48 | 3.9 | 8.2 | 4700 | 0.14 | 0.14 |
| D49 | 3.9 | 8.0 | 4000 | 0.14 | 0.14 |
| D50 | 4.2 | 8.0 | 4100 | 0.14 | 0.14 |
| D51 | 4.0 | 5.3 | 6100 | 0.17 | 0.33 |
| D52 | 3.9 | 6.7 | 1600 | 0.14 | 0.11 |
| D53 | 3.9 | 8.0 | 4600 | 0.14 | 0.15 |
| D54 | 3.9 | 8.1 | 4000 | 0.14 | 0.15 |
| D55 | 3.9 | 8.3 | 3800 | 0.14 | 0.13 |
| D56 | 4.2 | 7.7 | 4700 | 0.14 | 0.16 |
| D57 | 3.9 | 8.0 | 3900 | 0.14 | 0.13 |
| D58 | 4.2 | 8.1 | 3900 | 0.14 | 0.13 |
| D59 | 4.3 | 7.8 | 3900 | 0.14 | 0.13 |
| D60 | 4.2 | 8.4 | 4400 | 0.14 | 0.15 |
| D61 | 3.9 | 8.1 | 3700 | 0.14 | 0.14 |
| D62 | 3.9 | 8.0 | 4600 | 0.14 | 0.14 |
| D63 | 4.2 | 7.7 | 4000 | 0.15 | 0.17 |
| D64 | 4.3 | 8.0 | 3400 | 0.14 | 0.12 |

| | | | | | |
|---|---|---|---|---|---|
| * : nicht anspruchsgemäß | | | | | |

| **Tabelle 7: Aufbau der OLEDs** | | | | |
|---|---|---|---|---|
| **Bsp.** | **HTL 3 % NDP-9** | **HTL** | **EML** | **ETL** |
| | | | **HBL** | |
| | 20 nm | 20 nm | 40 nm | 30 nm |
| | | | 10 nm | |
| D65 | Bsp.7 | Bsp.7 | H3(50%):H4(45%):Irpy(5%) | ETM1(50%):LiQ(50%) |
| D66 | Bsp.8 | Bsp.8 | H3(50%):H4(45%):Irpy(5%) | ETM1(50%):LiQ(50%) |
| D67 | Bsp.16 | Bsp16. | H3(50%):H4(45%):Irpy(5%) | ETM1(50%):LiQ(50%) |
| D68 | Bsp.17 | Bsp.17 | H2(90%):Irpy(10%) | ETM1(50%):LiQ(50%) |
| D69 | Bsp.18 | Bsp.18 | H3(50%):H4(45%):Irpy(5%) | ETM1(50%):LiQ(50%) |
| D70 | Bsp.54 | Bsp.54 | H3(50%):H4(45%):Irpy(5%) | ETM1(50%):LiQ(50%) |
| D71 | Bsp.64 | Bsp.64 | H3(50%):H4(45%):Irpy(5%) | ETM1(50%):LiQ(50%) |
| D72_* | Bsp.66 | Bsp.66 | H3(50%):H4(45%):Irpy(5%) | ETM1(50%):LiQ(50%) |
| D73_* | Bsp.67 | Bsp.67 | H3(50%):H4(45%):Irpy(5%) | ETM1(50%):LiQ(50%) |
| D74 | Bsp.69 | Bsp.69 | H3(50%):H4(45%):Irpy(5%) | ETM1(50%):LiQ(50%) |
| D75 | Bsp.70 | Bsp.70 | H3(50%):H4(45%):Irpy(5%) | ETM1(50%):LiQ(50%) |
| D76 | Bsp.72 | Bsp.72 | H3(50%):H4(45%):Irpy(5%) | ETM1(50%):LiQ(50%) |
| D77 | Bsp.73 | Bsp.73 | H3(50%):H4(45%):Irpy(5%) | ETM1(50%):LiQ(50%) |
| D78 | Bsp.78 | Bsp.78 | H3(50%):H4(45%):Irpy(5%) | ETM1(50%):LiQ(50%) |
| D79 | Bsp.86 | Bsp.86 | H3(50%):H4(45%):Irpy(5%) | ETM1(50%):LiQ(50%) |
| D80 | Bsp.87 | Bsp.87 | H3(50%):H4(45%):Irpy(5%) | ETM1(50%):LiQ(50%) |
| D81 | Bsp.88 | Bsp.88 | H3(50%):H4(45%):Irpy(5%) | ETM1(50%):LiQ(50%) |
| D82 | Bsp.89 | Bsp.89 | H3(50%):H4(45%):Irpy(5%) | ETM1(50%):LiQ(50%) |
| D83 | Bsp.90 | Bsp.90 | H3(50%):H4(45%):Irpy(5%) | ETM1(50%):LiQ(50%) |
| D84 | Bsp.91 | Bsp.91 | H3(50%):H4(45%):Irpy(5%) | ETM1(50%):LiQ(50%) |
| D85 | Bsp.96 | Bsp.96 | H3(50%):H4(45%):Irpy(5%) | ETM1(50%):LiQ(50%) |
| D86 | Bsp.59 | Bsp.59 | H3(45%):H4(45%):Irppy(10%) | ETM1(50%):LiQ(50%) |
| D87 | Bsp.86 | Bsp.86 | H3(45%):H4(45%):Irppy(10%) | ETM1(50%):LiQ(50%) |
| D88 | Bsp.87 | Bsp.87 | H3(45%):H4(45%):Irppy(10%) | ETM1(50%):LiQ(50%) |
| D89 | Bsp.88 | Bsp.88 | H3(45%):H4(45%):Irppy(10%) | ETM1(50%):LiQ(50%) |
| D90 | Bsp.89 | Bsp.89 | H3(45%):H4(45%):Irppy(10%) | ETM1(50%):LiQ(50%) |
| D91 | Bsp.90 | Bsp.90 | H3(45%):H4(45%):Irppy(10%) | ETM1(50%):LiQ(50%) |
| D92 | Bsp.30 | Bsp.30 | H5(30%):H6(65%):Irbic(10%) | ETM1(50%):LiQ(50%) |
| | | | HBL | |
| D93 | Bsp.36 | Bsp.36 | H5(30%):H6(65%):Irbic(10%) | ETM1(50%):LiQ(50%) |
| | | | HBL | |
| D94 | Bsp.37 | Bsp.37 | H5(30%):H6(65%):Irbic(10%) | ETM1(50%):LiQ(50%) |
| | | | HBL | |
| D95 | Bsp.52 | Bsp.52 | H5(30%):H6(65%):Irbic(10%) | ETM1(50%):LiQ(50%) |
| | | | HBL | |
| D96 | Bsp.55 | Bsp.55 | H5(30%):H6(65%):Irbic(10%) | ETM1(50%):LiQ(50%) |
| | | | HBL | |
| D97 | Bsp.56 | Bsp.56 | H5(30%):H6(65%):Irbic(10%) | ETM1(50%):LiQ(50%) |
| | | | HBL | |
| D98 | Bsp.57 | Bsp.57 | H5(30%):H6(65%):Irbic(10%) | ETM1(50%):LiQ(50%) |
| | | | HBL | |
| D99 | Bsp.94 | Bsp.94 | H5(30%):H6(65%):Irbic(10%) | ETM1(50%):LiQ(50%) |
| | | | HBL | |
| D100 | Bsp.95 | Bsp.95 | H5(30%):H6(65%):Irbic(10%) | ETM1(50%):LiQ(50%) |
| | | | HBL | |
| D101 | Bsp.96 | Bsp.96 | H5(30%):H6(65%):Irbic(10%) | ETM1(50%):LiQ(50%) |
| | | | HBL | |
| D102 | Bsp.69 | Bsp.69 | Bsp.80(50%):H4(45%):Irpy(5%) | ETM1(50%):LiQ(50%) |
| D103 | Bsp.69 | Bsp.69 | Bsp.81(50%):H4(45%):Irpy(5%) | ETM1(50%):LiQ(50%) |
| D104 | Bsp.69 | Bsp.69 | Bsp.82(50%):H4(45%):Irpy(5%) | ETM1(50%):LiQ(50%) |
| D105 | Bsp.69 | Bsp.69 | Bsp.83(50%):H4(45%):Irpy(5%) | ETM1(50%):LiQ(50%) |
| D106 | Bsp.69 | Bsp.69 | Bsp.84(90%)Irpy(10%) | ETM1(50%):LiQ(50%) |
| D107 | Bsp.69 | Bsp.69 | Bsp.85(50%):H4(45%):Irpy(5%) | ETM1(50%):LiQ(50%) |
| D108 | Bsp.69 | Bsp.69 | Bsp.111(10%):Irpy(10%) | ETM1(50%):LiQ(50%) |
| D109 | Bsp.69 | Bsp.69 | Bsp.112(50%):H4(45%):Irpy(5%) | ETM1(50%):LiQ(50%) |
| D110 | Bsp.69 | Bsp.69 | Bsp.113(50%):H4(45%):Irpy(5%) | ETM1(50%):LiQ(50%) |
| D111 | Bsp.69 | Bsp.69 | Bsp.114(50%):H4(45%):Irpy(5%) | ETM1(50%):LiQ(50%) |
| D112 | Bsp.57 | Bsp.57 | Bsp.65(10%):Irbic(10%) | ETM1(50%):LiQ(50%) |
| | | | HBL | |
| D113 | Bsp.57 | Bsp.57 | Bsp.85(10%):Irbic(10%) | ETM1(50%):LiQ(50%) |
| | | | HBL | |
| D114 | Bsp.57 | Bsp.57 | H5(30%):Bsp97(65%):Irbic(5%) HBL | ETM1(50%):LiQ(50%) |
| | | | | |
| D115 | Bsp.57 | Bsp.57 | Bsp.113(30%):Bsp.97(65%):Irbic(5%) | ETM1(50%):LiQ(50%) |
| | | | Bsp.113 | |
| D116 | Bsp.57 | Bsp.57 | Bsp.115(10%):Irbic(10%) | ETM1(50%):LiQ(50%) |
| | | | HBL | |
| D117 | Bsp.57 | Bsp.57 | Bsp.116(10%):Irbic(10%) | ETM1(50%):LiQ(50%) |
| | | | HBL | |
| D118 | Bsp.57 | Bsp.57 | Bsp.117(10%):Irbic(10%) | ETM1(50%):LiQ(50%) |
| | | | HBL | |
| D119 | Bsp.57 | Bsp.57 | Bsp.119(10%):Irbic(10%) | ETM1(50%):LiQ(50%) |
| | | | HBL | |

| | | | | |
|---|---|---|---|---|
| * : nicht anspruchsgemäß | | | | |

| **Tabelle 8: Daten der OLEDs** | | | | | |
|---|---|---|---|---|---|
| **Bsp.** | **U @ 1000 cd/m²** | **EQE @ 1000 cd/m²** | **LD80 @ 1000 cd/m²** | **CIE** | |
| | V | % | [h] | x | y |
| D65 | 3.1 | 20.3 | 16000 | 0.35 | 0.62 |
| D66 | 3.3 | 20.4 | 17000 | 0.35 | 0.62 |
| D67 | 3.1 | 19.5 | 60000 | 0.33 | 0.63 |
| D68 | 3.0 | 20.0 | 2300 | 0.36 | 0.61 |
| D69 | 3.1 | 19.4 | 54000 | 0.33 | 0.63 |
| D70 | 3.2 | 19.8 | 58000 | 0.33 | 0.64 |
| D71 | 3.2 | 18.8 | 41000 | 0.33 | 0.64 |
| D72 * | 3.1 | 19.3 | 48000 | 0.33 | 0.63 |
| D73 * | 3.3 | 19.7 | 51000 | 0.34 | 0.63 |
| D74 | 3.0 | 20.4 | 61000 | 0.34 | 0.63 |
| D75 | 3.1 | 19.7 | 63000 | 0.34 | 0.63 |
| D76 | 3.0 | 20.5 | 59000 | 0.34 | 0.63 |
| D77 | 3.0 | 20.1 | 60000 | 0.34 | 0.63 |
| D78 | 3.0 | 18.4 | 33000 | 0.34 | 0.65 |
| D79 | 3.1 | 19.7 | 62000 | 0.34 | 0.63 |
| D80 | 3.0 | 20.7 | 66000 | 0.34 | 0.63 |
| D81 | 3.2 | 21.1 | 65000 | 0.33 | 0.63 |
| D82 | 3.0 | 21.3 | 67000 | 0.34 | 0.63 |
| D83 | 3.2 | 20.8 | 66000 | 0.33 | 0.63 |
| D84 | 3.0 | 21.4 | 62000 | 0.34 | 0.63 |
| D85 | 3.0 | 21.0 | 58000 | 0.34 | 0.63 |
| D86 | 3.0 | 23.5 | 46000 | 0.44 | 0.55 |
| D87 | 2.9 | 23.6 | 60000 | 0.43 | 0.55 |
| D88 | 2.9 | 23.5 | 61000 | 0.44 | 0.55 |
| D89 | 3.1 | 24.0 | 58000 | 0.44 | 0.55 |
| D90 | 2.9 | 23.7 | 67000 | 0.44 | 0.55 |
| D91 | 3.2 | 23.9 | 65000 | 0.43 | 0.55 |
| D92 | 4.7 | 13.4 | 180 | 0.15 | 0.30 |
| D93 | 4.8 | 13.6 | 160 | 0.15 | 0.30 |
| D94 | 4.6 | 12.9 | 150 | 0.15 | 0.30 |
| D95 | 4.7 | 13.1 | 180 | 0.15 | 0.31 |
| D96 | 4.7 | 13.2 | 210 | 0.15 | 0.30 |
| D97 | 4.8 | 13.3 | 200 | 0.15 | 0.29 |
| D98 | 4.8 | 13.4 | 230 | 0.15 | 0.30 |
| D99 | 4.7 | 12.8 | 220 | 0.15 | 0.30 |
| D100 | 5.0 | 13.6 | 200 | 0.15 | 0.30 |
| D101 | 4.8 | 13.7 | 200 | 0.15 | 0.30 |
| D102 | 3.0 | 22.4 | 66000 | 0.34 | 0.63 |
| D103 | 2.9 | 22.0 | 65000 | 0.34 | 0.63 |
| D104 | 3.0 | 21.9 | 68000 | 0.34 | 0.64 |
| D105 | 2.9 | 22.2 | 69000 | 0.34 | 0.63 |
| D106 | 3.3 | 21.5 | 48000 | 0.34 | 0.63 |
| D107 | 2.9 | 22.5 | 63000 | 0.34 | 0.63 |
| D108 | 3.3 | 21.9 | 45000 | 0.34 | 0.63 |
| D109 | 2.9 | 21.5 | 60000 | 0.34 | 0.63 |
| D110 | 2.9 | 22.2 | 64000 | 0.35 | 0.63 |
| D111 | 3.1 | 22.1 | 62000 | 0.34 | 0.63 |
| D112 | 4.8 | 13.0 | 200 | 0.15 | 0.30 |
| D113 | 5.2 | 13.9 | 170 | 0.15 | 0.29 |
| D114 | 4.5 | 13.6 | 230 | 0.15 | 0.30 |
| D115 | 4.6 | 13.8 | 210 | 0.15 | 0.29 |
| D116 | 4.5 | 13.6 | 180 | 0.15 | 0.30 |
| D117 | 4.5 | 13.4 | 150 | 0.15 | 0.30 |
| D118 | 4.5 | 13.7 | 200 | 0.15 | 0.30 |
| D119 | 4.8 | 13.4 | 160 | 0.15 | 0.30 |

| | | | | | |
|---|---|---|---|---|---|
| * : nicht anspruchsgemäß | | | | | |

### Verwendung der erfindungsgemäßen Verbindungen in weißen OLEDs

Gemäß den allgemeinen Verfahren wird eine weiß emittierende OLED mit folgendem Schichtaufbau hergestellt: Substrat / Lochinjektionsschicht (HIL1, 20 nm) / Lochtransportschicht (HIL3, 40 nm) / Lochtransportschicht (erfindungsgemäßes HTL, dotiert mit NPD-9 (3 %) der Fa. Novaled, 20 nm) / Lochtransportschicht (erfindungsgemäßes HTL, 200 nm) / Emissionsschichten (EML, s. Tabelle) / Lochblockierschicht (HBL, 10 nm, s. Tabelle) Elektronentransportschicht (ETL, 30 nm) / optionale Elektroneninjektionsschicht (EIL) und abschließend eine Kathode. Die Kathode wird durch eine 100 nm dicke Aluminiumschicht gebildet. Der genaue Aufbau der OLEDs und die Ergebnisse ist den Tabellen 9 und 10 zu entnehmen. Die zur Herstellung der OLEDs benötigten Materialien sind in Tabelle 12 gezeigt.

**Tabelle 9: Aufbau der weißen OLEDs**

| **Bsp.** | **HTL** | **EML Rot** | **EML Blau** | **EML Grün** | **HBL** | **ETL** |
|---|---|---|---|---|---|---|
| | **Dicke** | **Dicke** | **Dicke** | **Dicke** | **Dicke** | **Dicke** |
| D-W1 | Bsp. 88 | Bsp.88:Ir-R (97%:3%) | H6:HBL:Irbic₃ (45%:50%:5%) | Bsp.110:Irpy (90%:10%) | HBL | ETM1(50%): LiQ(50%) |
| | 200 nm | 9 nm | 8 nm | 7 nm | 10 nm | 30 nm |

**Tabelle 10: Deviceergebnisse**

| **Bsp.** | **EQE (%)** | **Spannung (V)** | **CIE x/y 1000 cd/m²** | **LD50 (h)** |
|---|---|---|---|---|
| | **1000 cd/m²** | **1000 cd/m²** | **CRI** | **1000 cd/m²** |
| D-W1 | 14.1 | 6.3 | 0.45/0.43 | 2500 |
| | | | 80 | |

### Verwendung der erfindungsgemäßen Verbindungen aus Lösung prozessierten OLEDs

Die erfindungsgemäßen Verbindungen können auch aus Lösung verarbeitet werden und führen dort zu prozesstechnisch wesentlich einfacheren OLEDs, im Vergleich zu den vakuumprozessierten OLEDs, mit dennoch guten Eigenschaften. Die Herstellung solcher Bauteile lehnt sich an die Herstellung polymerer Leuchtdioden (PLEDs) an, die in der Literatur bereits vielfach beschrieben ist (z.B. in der WO 2004/037887).

Der Aufbau setzt sich aus Substrat / ITO / PEDOT (80 nm) / Interlayer (80 nm) / Emissionsschicht (80 nm) / Kathode zusammen. Dazu werden Substrate der Firma Technoprint (Sodalimeglas) verwendet, auf welche die ITO-Struktur (Indium-Zinn-Oxid, eine transparente, leitfähige Anode) aufgebracht wird. Die Substrate werden im Reinraum mit DI Wasser und einem Detergens (Deconex 15 PF) gereinigt und dann durch eine UV/Ozon-Plasmabehandlung aktiviert. Danach wird ebenfalls im Reinraum als Pufferschicht eine 80 nm Schicht PEDOT (PEDOT ist ein Polythiophen-Derivat (Baytron P VAI 4083sp.) von H. C. Starck, Goslar, das als wässrige Dispersion geliefert wird) durch Spin-Coating aufgebracht. Die benötigte Spinrate hängt vom Verdünnungsgrad und der spezifischen Spin-Coater-Geometrie ab (typisch für 80 nm: 4500 rpm). Um Restwasser aus der Schicht zu entfernen, werden die Substrate für 10 Minuten bei 180 °C auf einer Heizplatte ausgeheizt. Die verwendete Interlayer dient der Lochinjektion, in diesem Fall wird HIL-012 von Merck verwendet. Die Interlayer kann alternativ auch durch eine oder mehrere Schichten ersetzt werden, die lediglich die Bedingung erfüllen müssen, durch den nachgelagerten Prozessierungsschritt der EML-Abscheidung aus Lösung nicht wieder abgelöst zu werden. Zur Herstellung der Emissionsschicht werden die erfindungsgemäßen Emitter zusammen mit den Matrixmaterialien in Toluol gelöst. Der typische Feststoffgehalt solcher Lösungen liegt zwischen 16 und 25 g/L, wenn, wie hier, die für eine Device typische Schichtdicke von 80 nm mittels Spincoating erzielt werden soll. Die lösungsprozessierten Devices enthalten eine Emissionsschicht (EML) aus (Polystyrol):Bsp.:H7:IrL (D-L1-D-L6) bzw. (Polystyrol):Bsp.:Bsp.:IrL (D-L7) (15%:25%:50%:10%). Die Emissionsschicht wird in einer Inertgasatmosphäre, im vorliegenden Fall Argon, aufgeschleudert und 30 min bei 130 °C ausgeheizt. Zuletzt wird eine Kathode aus Barium (5 nm) und dann Aluminium (100 nm) (hochreine Metalle von Aldrich, besonders Barium 99.99 % (Best-Nr. 474711); Aufdampfanlagen von Lesker o.a., typischer Aufdampfdruck 5 x 10⁻⁶ mbar) aufgedampft. Optional kann zunächst eine Lockblockierschicht und dann eine Eletronentransportschicht und dann erst die Kathode (z.B. Al oder LiF/Al) im Vakuum aufgedampft werden. Um das Device vor Luft und Luftfeuchtigkeit zu schützen, wird die Vorrichtung abschließend verkapselt und dann charakterisiert. Die genannten OLED-Beispiele sind noch nicht optimiert, Tabelle 11 fasst die erhaltenen Daten zusammen.

| **Tabelle 11: Daten der OLEDs** | | | | | | |
|---|---|---|---|---|---|---|
| **Bsp.** | **EML Bsp.** | **U @ 1000 cd/m2** | **EQE @ 1000 cd/m2** | **LD80 @ 1000 cd/m²** | **CIE** | |
| | | V | % | [h] | x | y |
| D-L1 | 75 | 3.6 | 17.9 | 16000 | 0.32 | 0.63 |
| D-L2 | 76 | 3.8 | 18.2 | 18000 | 0.33 | 0.63 |
| D-L3 | 79 | 3.7 | 18.0 | 17000 | 0.32 | 0.64 |
| D-L4 | 98 | 3.7 | 16.4 | 19000 | 0.32 | 0.64 |
| D-L5 | 99 | 3.8 | 19.2 | 20000 | 0.33 | 0.63 |
| D-L6 | 10 | 4.0 | 19.7 | 24000 | 0.33 | 0.63 |
| D-L7 | 100 + 118 | 3.7 | 17.2 | 14000 | 0.33 | 0.63 |

**Tabelle 12:**

| | | |
|---|---|---|
| 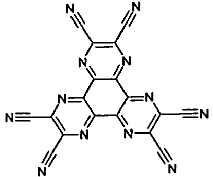 | 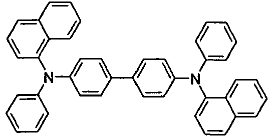 | 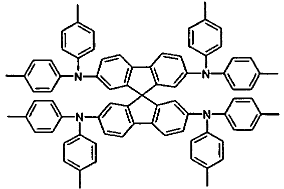 |
| HIL1 | NPB | HIL2 |
| 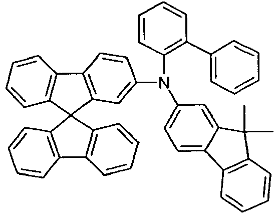 | 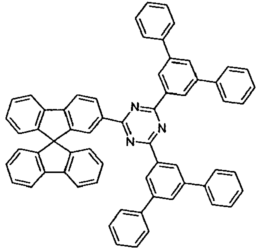 | 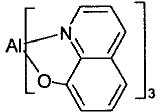 |
| HIL3 | ETM1 | Alq3 |
| 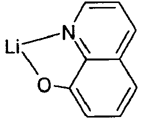 | 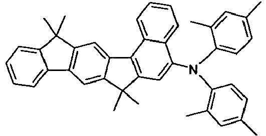 | 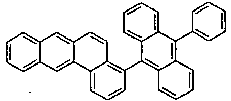 |
| LiQ | SEB1 | H1 |
| 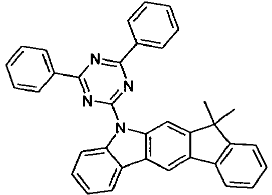 | 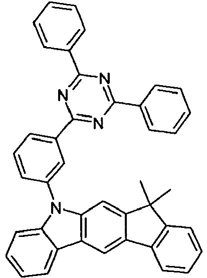 | 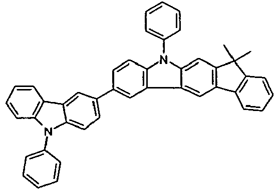 |
| H2 | H3 | H4 |
| 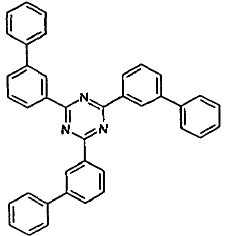 | 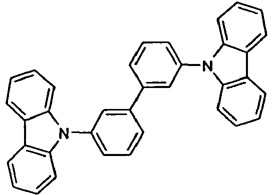 | 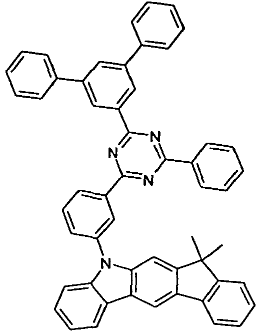 |
| H5 | H6 | H7 |
| 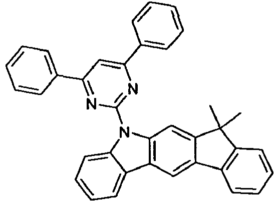 | 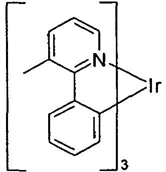 | 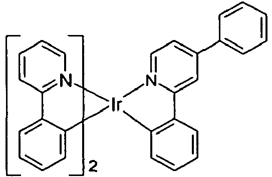 |
| HBL | Irpy | Irppy |
| 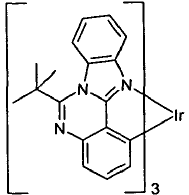 | 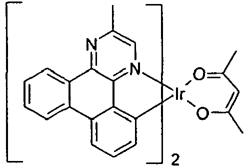 | 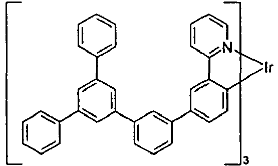 |
| Irbic | IrR | IrL |

## Patentansprüche

1. Verbindung gemäß Formel (1), (2) oder (3) 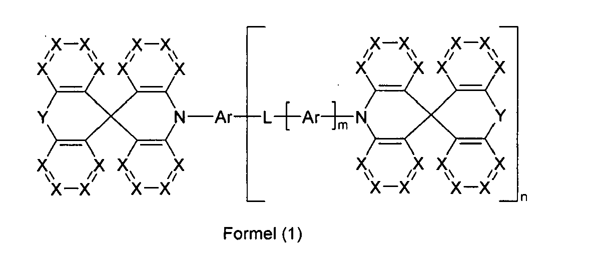 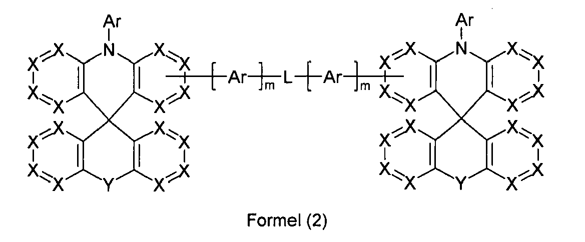 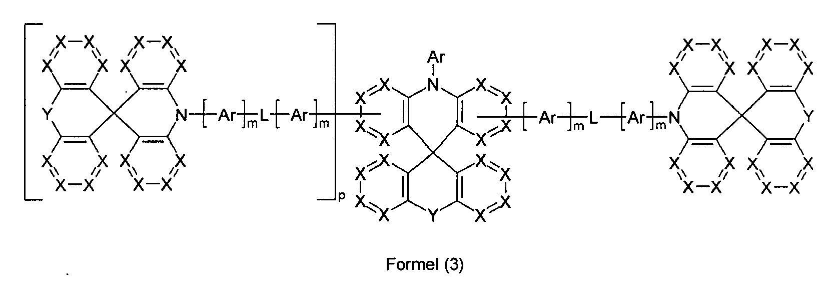 wobei für die verwendeten Symbole und Indizes gilt:
Y ist bei jedem Auftreten gleich oder verschieden O oder S;
X ist bei jedem Auftreten gleich oder verschieden CR² oder N; oder zwei benachbarte X stehen für S, O oder NR², so dass ein Fünfring entsteht; oder zwei benachbarte X stehen für eine Gruppe der folgenden Formel (4) oder (5), 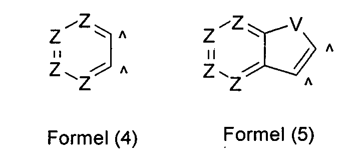 wobei ^ die entsprechenden benachbarten Gruppen X in der Formel (1), (2) oder (3) andeutet;
dabei steht X für C, wenn an diese Gruppe X in Formel (2) bzw. (3) eine Gruppe Ar bzw. L gebunden ist;
V ist bei jedem Auftreten gleich oder verschieden C(R²)₂, NR², O oder S;
Z ist bei jedem Auftreten gleich oder verschieden CR² oder N;
Ar ist bei jedem Auftreten gleich oder verschieden ein aromatisches oder heteroaromatisches Ringsystem mit 5 bis 40 aromatischen Ringatomen, das mit einem oder mehreren Resten R³ substituiert sein kann; dabei können die Gruppe Ar und die benachbarte Gruppe X, die in diesem Fall für C steht, auch durch eine Einfachbindung oder eine bivalente Gruppe, ausgewählt aus C(R³)₂, NR³, O oder S, miteinander verbrückt sein;
L ist bei jedem Auftreten gleich oder verschieden eine Einfachbindung oder eine bivalente Gruppe;
R¹, R², R³ ist bei jedem Auftreten gleich oder verschieden ausgewählt aus der Gruppe bestehend aus H, D, F, Cl, Br, I, CN, NO₂, N(Ar¹)₂, N(R⁴)₂, C(=O)Ar¹, C(=O)R⁴, P(=O)(Ar¹)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R⁴ substituiert sein kann, wobei eine oder mehrere nicht-benachbarte CH₂-Gruppen durch R⁴C=CR⁴, C=C, Si(R⁴)₂, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60, aromatischen Ringatomen, das jeweils mit einem oder mehreren Resten R⁴ substituiert sein kann, einer Aryloxy- oder Heteroaryl-oxygruppe mit 5 bis 60 aromatischen Ringatomen, die mit einem oder mehreren Resten R⁴ substituiert sein kann, wobei optional zwei oder mehr benachbarte Substituenten R¹ bzw. R³ ein mono-cyclisches oder polycyclisches, aliphatisches Ringsystem bzw. R² ein monocyclisches oder polycyclisches, aliphatisches, aromatisches oder heteroaromatisches Ringsystem bilden können, das mit einem oder mehreren Resten R⁴ substituiert sein kann;
R⁴ ist bei jedem Auftreten gleich oder verschieden ausgewählt aus der Gruppe bestehend aus H, D, F, Cl, Br, I, CN, NO₂, N(Ar¹)₂, N(R⁵)₂, C(=O)Ar¹, C(=O)R⁵, P(=O)(Ar¹)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R⁵ substituiert sein kann, wobei eine oder mehrere nicht-benachbarte CH₂-Gruppen durch R⁵C=CR⁵, C=C, Si(R⁵)₂, C=O, C=S, C=NR⁵, P(=O)(R⁵), SO, SO₂, NR⁵, O, S oder CONR⁵ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R⁵ substituiert sein kann, einer Aryloxy- oder Heteroaryloxygruppe mit 5 bis 60 aromatischen Ringatomen, die mit einem oder mehreren Resten R⁵ substituiert sein kann, oder einer Kombination dieser Systeme, wobei optional zwei oder mehr benachbarte Substituenten R⁴ ein monocyclisches oder polycyclisches, aliphatisches Ringsystem bilden können, das mit einem oder mehreren Resten R⁵ substituiert sein kann;
Ar¹ ist bei jedem Auftreten gleich oder verschieden ein aromatisches oder heteroaromatisches Ringsystem mit 5-30 aromatischen Ringatomen, das mit einem oder mehreren nicht-aromatischen Resten R⁵ substituiert sein kann; dabei können zwei Reste Ar¹, welche an dasselbe N-Atom oder P-Atom binden, auch durch eine Einfachbindung oder eine Brücke, ausgewählt aus N(R⁵), C(R⁵)₂, O oder S, miteinander verbrückt sein;
R⁵ ist bei jedem Auftreten gleich oder verschieden ausgewählt aus der Gruppe bestehend aus H, D, F, CN, einem aliphatischen Kohlenwasserstoffrest mit 1 bis 20 C-Atomen, einem aromatischen oder heteroaromatischen Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R³ miteinander ein mono- oder polycyclisches, aliphatisches Ringsystem bilden können;
m ist bei jedem Auftreten gleich oder verschieden 0 oder 1;
n ist 0, 1, 2, 3, 4 oder 5;
p ist 0 oder 1.

2. Verbindung nach Anspruch 1, **dadurch gekennzeichnet, dass** Y gleich O ist.

3. Verbindung nach Anspruch 1 oder 2, **dadurch gekennzeichnet, dass** X gleich oder verschieden bei jedem Auftreten für CR² oder N, wobei maximal eine Gruppe X pro Cyclus für N steht; oder zwei benachbarte Gruppen X stehen für eine Gruppe der Formel (4) oder (5), insbesondere Formel (5), wobei Z gleich oder verschieden bei jedem Auftreten für CR² steht und V gleich oder verschieden bei jedem Auftreten für NR² oder C(R²)₂steht.

4. Verbindung nach einem oder mehreren der Ansprüche 1 bis 3, **dadurch gekennzeichnet, dass** L gleich oder verschieden bei jedem Auftreten für eine geradkettige Alkylen-, Alkyliden-, Alkylenoxy- oder Thioalkylenoxygruppe mit 1 bis 10 C-Atomen oder eine verzweigte oder cyclische Alkylen-, Alkyliden-, Alkylenoxy- oder Thioalkylenoxygruppe mit 3 bis 40 C-Atomen oder eine Alkenylen- oder Alkinylengruppe mit 2 bis 40 C-Atomen, die mit jeweils einem oder mehreren Resten R⁴ substituiert sein kann, wobei eine oder mehrere nicht benachbarte CH₂-Gruppen durch -R⁴C=CR⁴-, -C=C-, Si(R⁴)₂, C=O, C=NR⁴, P(=O)R⁴, S=O, SO₂, -O-, -S- oder -CONR⁴- ersetzt sein können und wobei ein oder mehrere H-Atome durch D, F, Cl, Br, I, CN oder NO₂ ersetzt sein können oder P(R⁴), P(=O)(R⁴), N(Ar¹) steht; oder L ist eine Einfachbindung.

5. Verbindung nach einem oder mehreren der Ansprüche 1 bis 4, ausgewählt aus den Verbindungen der Formeln (6) bis (12), 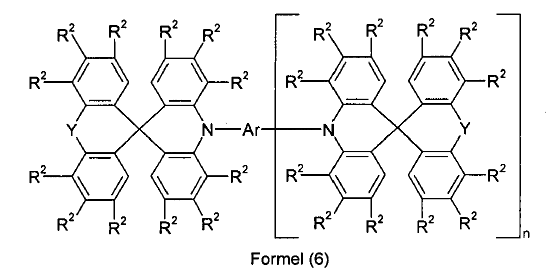 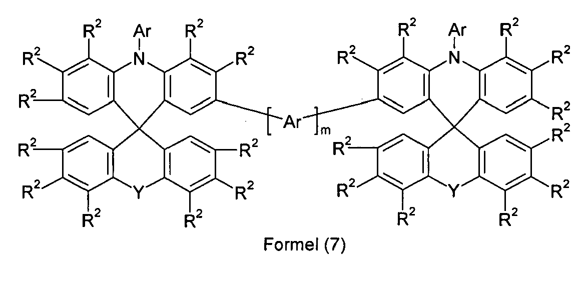 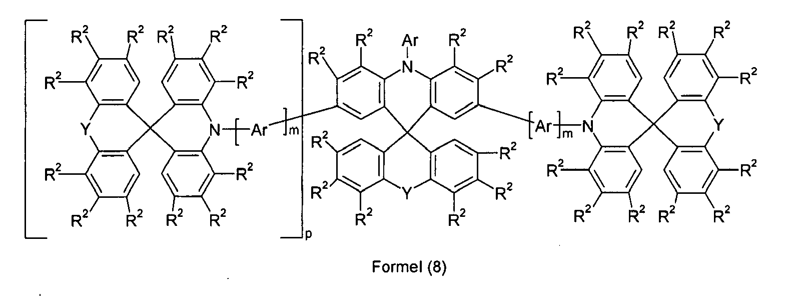 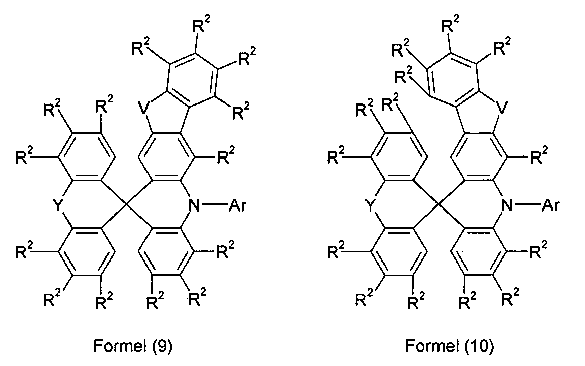 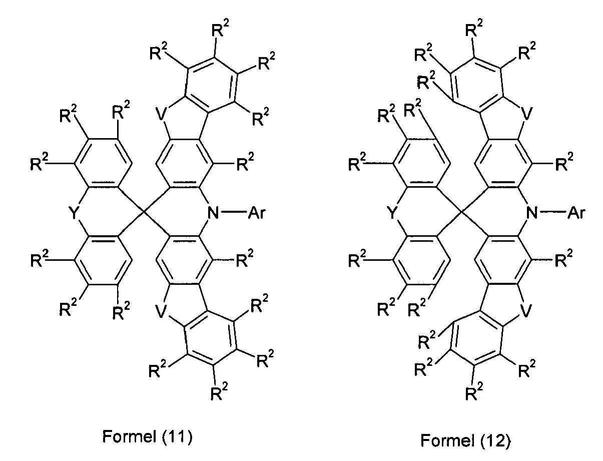 wobei die verwendeten Symbole und Indizes die in Anspruch 1 genannten Bedeutungen aufweisen und Y bevorzugt für O steht.

6. Verbindung nach einem oder mehreren der Ansprüche 1 bis 5, ausgewählt aus den Verbindungen der Formeln (6a) bis (12a), 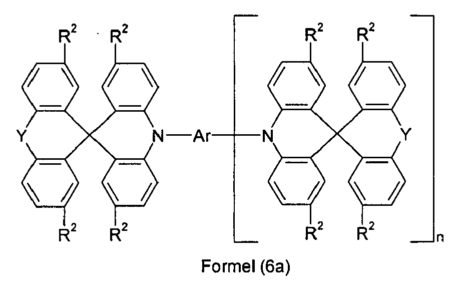 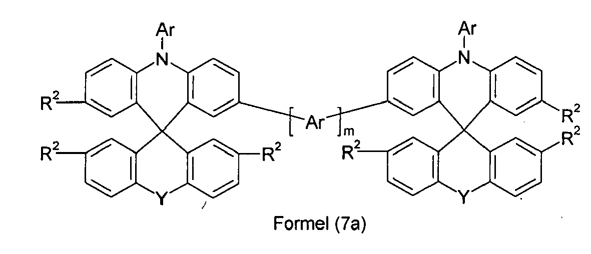 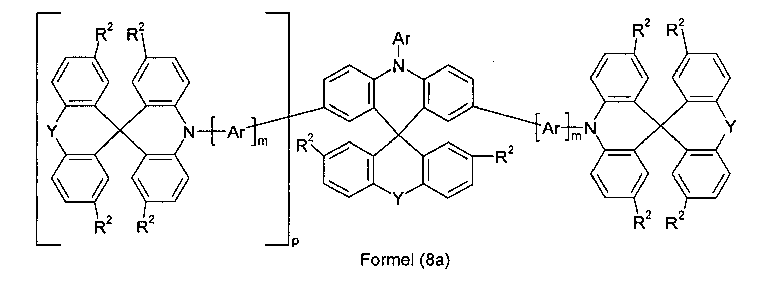 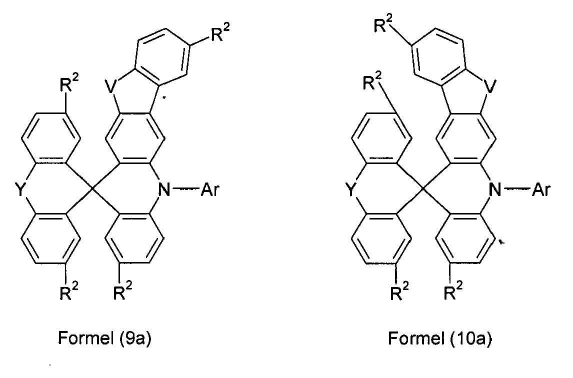 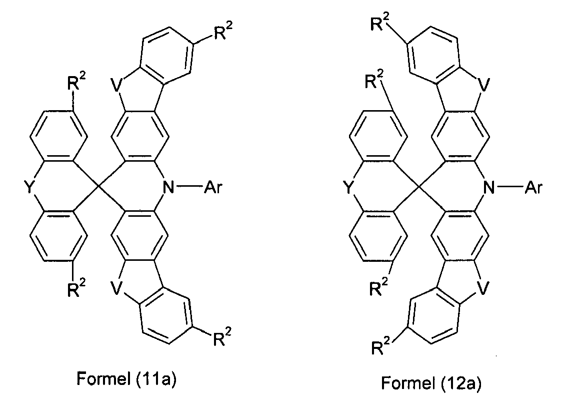 wobei die verwendeten Symbole und Indizes die in Anspruch 1 genannten Bedeutungen aufweisen und Y bevorzugt für O steht.

7. Verbindung nach einem oder mehreren der Ansprüche 1 bis 6, **dadurch gekennzeichnet, dass** Ar ausgewählt ist aus der Gruppe bestehend aus aromatischen oder heteroaromatischen Ringsystemen mit 5 bis 24 aromatischen Ringatomen, welche jeweils durch einen oder mehrere Reste R³ substituiert sein können, insbesondere Benzol, ortho-, meta- oder para-Biphenyl, ortho-, meta-, para- oder verzweigtes Terphenyl, ortho-, meta- para- oder verzweigtes Quaterphenyl, 1- oder 2-Naphthyl, Pyrrol, Furan, Thiophen, Indol, Benzothiophen, Benzofuran, Carbazol, Dibenzofuran, Dibenzothiophen, Pyridin, Pyrimidin, Pyrazin, Pyridazin, Triazin, Anthracen, Phenanthren, Pyren, Benzanthracen oder Kombinationen aus zwei oder drei dieser Gruppen, welche jeweils mit einem oder mehreren Resten R³ substituiert sein können.

8. Verbindung nach einem oder mehreren der Ansprüche 1 bis 7, **dadurch gekennzeichnet, dass** mindestens ein Substituent R¹, R² und/oder R³ oder eine monovalente Gruppe Ar ausgewählt ist aus Strukturen gemäß den Formeln (13) bis (16) für R¹ bis R³ oder den Formeln (13), (15) oder (16) für Ar, 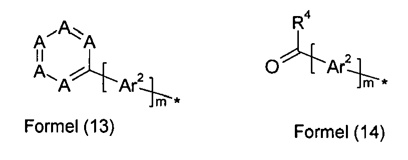 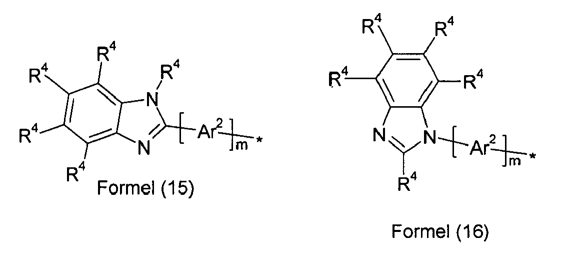 und/oder mindestens eine bivalente oder trivalente Gruppe Ar ausgewählt ist aus den Gruppen der Formeln (17) bis (19), 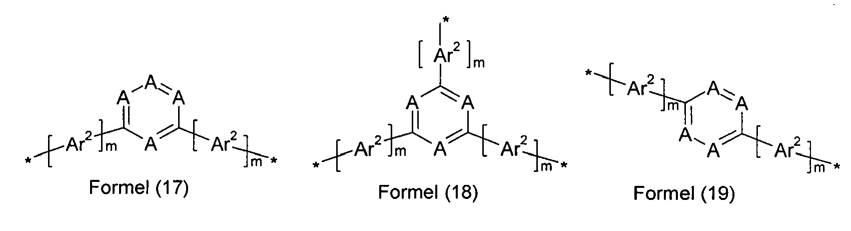 wobei R³, R⁴ und m die in Anspruch 1 genannte Bedeutung haben, * die Position der Bindung der Gruppe gemäß Formel (13) bis (19) andeutet und weiterhin gilt:
A ist bei jedem Auftreten gleich oder verschieden CR⁴ oder N, mit der Maßgabe, dass eine Gruppe A, zwei Gruppen A oder drei Gruppen A für N stehen;
Ar² ist gleich oder verschieden bei jedem Auftreten ein bivalentes aromatisches oder heteroaromatisches Ringsystem mit 5 bis 16 C-Atomen, welches durch einen oder mehrere Reste R⁴ substituiert sein kann;
und/oder dass mindestens einer der Reste R¹, R², R³ oder Ar ausgewählt ist aus den Gruppen der Formeln (20) bis (26), und/oder mindestens einer der Reste Ar ausgewählt ist aus Gruppen der Formeln (27) bis (34), 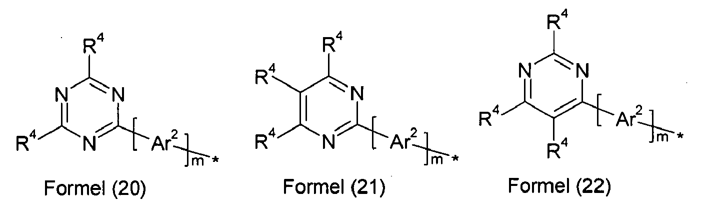 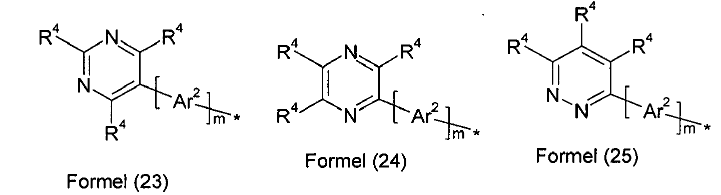 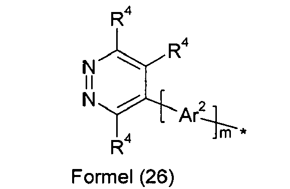 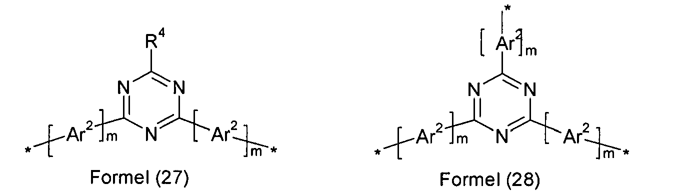 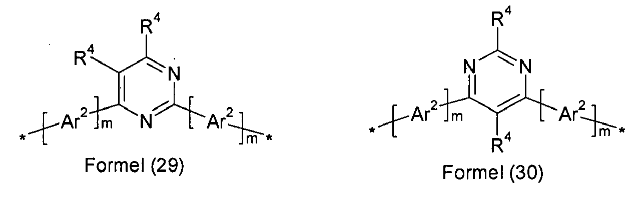 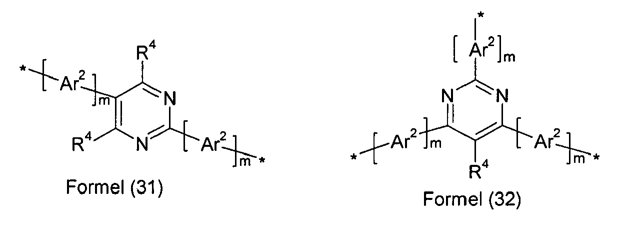 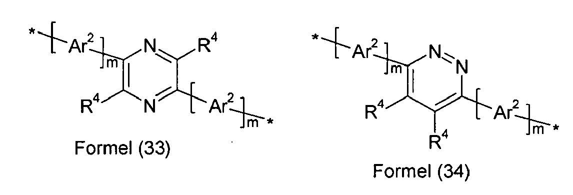 wobei die verwendeten Symbole und Indizes die in Anspruch 1 und oben genannten Bedeutungen aufweisen;
und/oder dass mindestens einer der Reste R¹, R², R³ oder Ar ausgewählt ist aus den Gruppen der Formeln (35) bis (49), 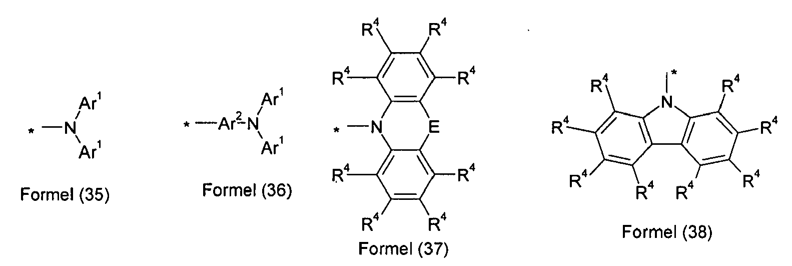 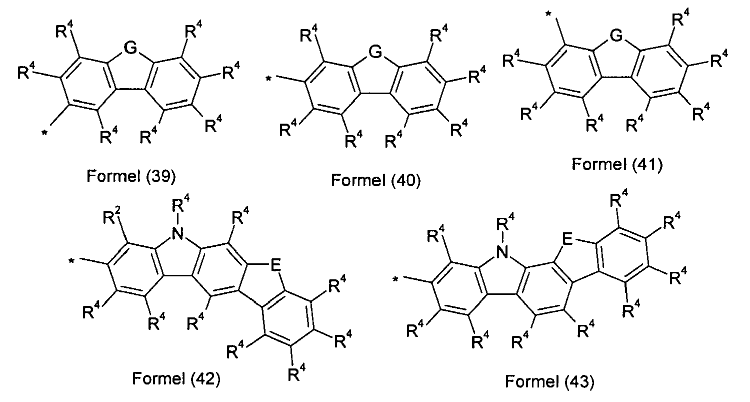 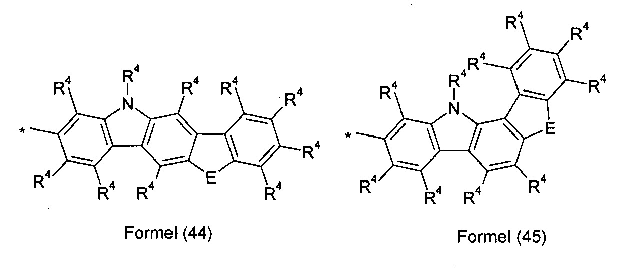 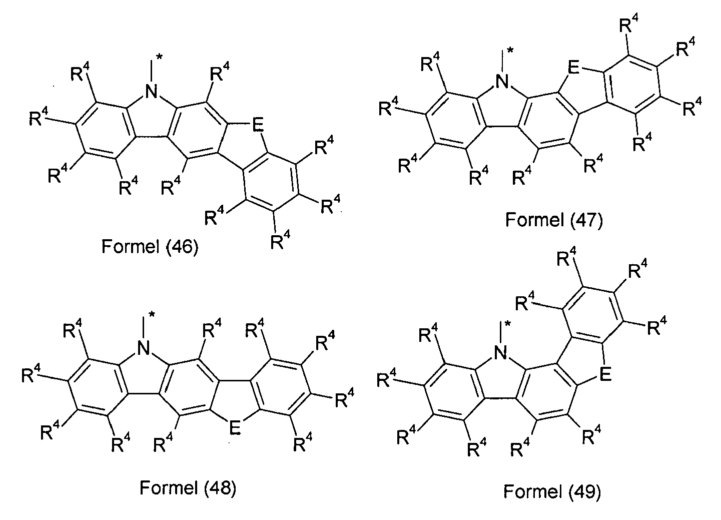 wobei die verwendeten Symbole die in Anspruch 1 genannten Bedeutungen aufweisen und weiterhin gilt:
E ist ausgewählt aus der Gruppe bestehend aus C(R⁴)₂, NR⁴, O oder S;
G ist ausgewählt aus der Gruppe bestehend aus NR⁴, O oder S;
wobei R⁴ durch R³ zu ersetzen ist, wenn die Gruppen der Formeln (13), (15), (16), (20) bis (26) und (35) bis (49) für Ar stehen, sowie in den Fällen der Formeln (17) bis (19) und (27) bis (34).

9. Verfahren zur Herstellung einer Verbindung nach einem oder mehreren der Ansprüche 1 bis 8, umfassend die Reaktionsschritte:
a) Aufbau des halogenierten Grundkörpers; und
b) Einführung von Substituenten über eine übergangsmetallkatalysierte Kupplungsreaktion.

10. Oligomer, Polymer oder Dendrimer enthaltend eine oder mehrere Verbindungen nach einem oder mehreren der Ansprüche 1 bis 8, wobei eine oder mehrere Bindungen der Verbindung zum Polymer, Oligomer oder Dendrimer vorhanden sind.

11. Verwendung einer Verbindung nach einem oder mehreren der Ansprüche 1 bis 8 oder 10 in einer elektronischen Vorrichtung, insbesondere in einer organischen Elektrolumineszenzvorrichtung.

12. Elektronische Vorrichtung enthaltend mindestens eine Verbindung nach einem oder mehreren der Ansprüche 1 bis 8 oder 10, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Devices, lichtemittierenden elektrochemischen Zellen, organischen Laserdioden und "organic plasmon emitting devices".

13. Elektronische Vorrichtung nach Anspruch 12, wobei es sich um eine organische Elektrolumineszenzvorrichtung handelt, **dadurch gekennzeichnet, dass** die Verbindung nach einem oder mehreren der Ansprüche 1 bis 8 oder 10 als Matrixmaterial für fluoreszierende oder phosphoreszierende Emitter und/oder als fluoreszierender Emitter und/oder in einer elektronenblockierenden bzw. exzitonenblockierenden Schicht und/oder in einer Lochtransportschicht und/oder in einer Lochblockierschicht und/oder in einer Elektronentransportschicht und/oder in einer optischen Auskopplungsschicht eingesetzt wird.

## Claims

1. Compound of the formula (1), (2) or (3) 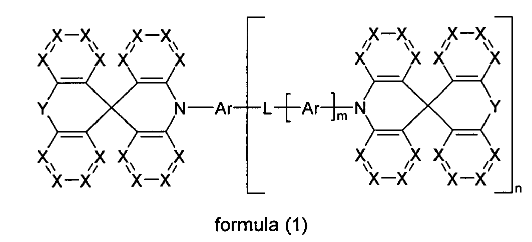 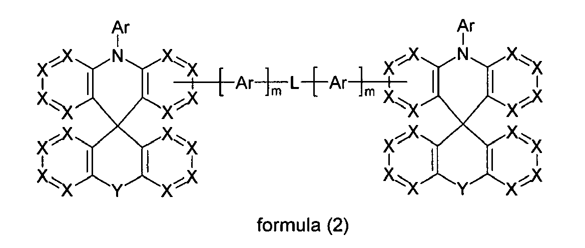 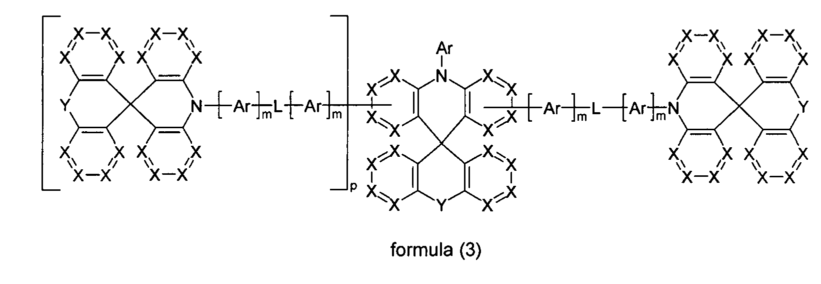 where the following applies to the symbols and indices used:
Y is on each occurrence, identically or differently, O or S;
X is on each occurrence, identically or differently, CR² or N; or two adjacent X stand for S, O or NR², so that a five-membered ring arises; or two adjacent X stand for a group of the following formula (4) or (5), 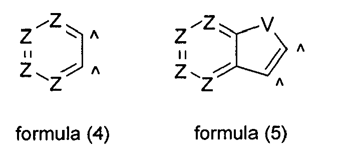 where ^ indicates the corresponding adjacent groups X in the formula (1), (2) or (3);
X here stands for C if a group Ar or L is bonded to this group X in formula (2) or (3);
V is on each occurrence, identically or differently, C(R²)₂, NR², O or S;
Z is on each occurrence, identically or differently, CR² or N;
Ar is on each occurrence, identically or differently, an aromatic or heteroaromatic ring system having 5 to 40 aromatic ring atoms, which may be substituted by one or more radicals R³; the group Ar and the adjacent group X, which in this case stands for C, may also be bridged to one another here by a single bond or a divalent group selected from C(R³)₂, NR³, O or S;
L is on each occurrence, identically or differently, a single bond or a divalent group;
R¹, R², R³ are selected on each occurrence, identically or differently, from the group consisting of H, D, F, Cl, Br, I, CN, NO₂, N(Ar¹)2, N(R⁴)₂, C(=O)Ar¹, C(=O)R⁴, P(=O)(Ar¹)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, each of which may be substituted by one or more radicals R⁴, where one or more non-adjacent CH₂ groups may be replaced by R⁴C=CR⁴, C≡C, Si(R⁴)₂, C=O, C=S, C=NR⁴, P(=O)(R⁴), SO, SO₂, NR⁴, O, S or CONR⁴ and where one or more H atoms may be replaced by D, F, Cl, Br, I, CN or NO₂, an aromatic or heteroaromatic ring system having 5 to 60 aromatic ring atoms, which may in each case be substituted by one or more radicals R⁴, an aryloxy or heteroaryloxy group having 5 to 60 aromatic ring atoms, which may be substituted by one or more radicals R⁴, where two or more adjacent substituents R¹ or R³ may optionally form a monocyclic or polycyclic, aliphatic ring system or R² may form a monocyclic or polycyclic, aliphatic, aromatic or heteroaromatic ring system, which may be substituted by one or more radicals R⁴;
R⁴ is selected on each occurrence, identically or differently, from the group consisting of H, D, F, Cl, Br, I, CN, NO₂, N(Ar¹)₂, N(R⁵)₂, C(=O)Ar¹, C(=O)R⁵, P(=O)(Ar¹)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, each of which may be substituted by one or more radicals R⁵, where one or more non-adjacent CH₂ groups may be replaced by R⁵C=CR⁵, C≡C, Si(R⁵)₂, C=O, C=S, C=NR⁵, P(=O)(R⁵), SO, SO₂, NR⁵, O, S or CONR⁵ and where one or more H atoms may be replaced by D, F, Cl, Br, I, CN or NO₂, an aromatic or heteroaromatic ring system having 5 to 60 aromatic ring atoms, which may in each case be substituted by one or more radicals R⁵, an aryloxy or heteroaryloxy group having 5 to 60 aromatic ring atoms, which may be substituted by one or more radicals R⁵, or a combination of these systems, where two or more adjacent substituents R⁴ may optionally form a monocyclic or polycyclic, aliphatic ring system, which may be substituted by one or more radicals R⁵;
Ar¹ is on each occurrence, identically or differently, an aromatic or heteroaromatic ring system having 5-30 aromatic ring atoms, which may be substituted by one or more non-aromatic radicals R⁵; two radicals Ar¹ which are bonded to the same N atom or P atom may also be bridged to one another here by a single bond or a bridge selected from N(R⁵), C(R⁵)₂, O or S;
R⁵ is selected on each occurrence, identically or differently, from the group consisting of H, D, F, CN, an aliphatic hydrocarbon radical having 1 to 20 C atoms, an aromatic or heteroaromatic ring system having 5 to 30 aromatic ring atoms, in which one or more H atoms may be replaced by D, F, Cl, Br, I or CN, where two or more adjacent substituents R³ may form a mono- or polycyclic, aliphatic ring system with one another;
m is on each occurrence, identically or differently, 0 or 1;
n is 0, 1, 2, 3, 4 or 5;
p is 0 or 1.

2. Compound according to Claim 1, **characterised in that** Y is equal to O.

3. Compound according to Claim 1 or 2, **characterised in that** X stands, identically or differently on each occurrence, for CR² or N, where a maximum of one group X per ring stands for N; or two adjacent groups X stand for a group of the formula (4) or (5), in particular formula (5), where Z stands, identically or differently on each occurrence, for CR² and V stands, identically or differently on each occurrence, for NR² or C(R²)_{2.}

4. Compound according to one or more of Claims 1 to 3, **characterised in that** L stands, identically or differently on each occurrence, for a straight-chain alkylene, alkylidene, alkyleneoxy or thioalkyleneoxy group having 1 to 10 C atoms or a branched or cyclic alkylene, alkylidene, alkyleneoxy or thioalkyleneoxy group having 3 to 40 C atoms or an alkenylene or alkynylene group having 2 to 40 C atoms, which may be substituted by in each case one or more radicals R⁴, where one or more non-adjacent CH₂ groups may be replaced by -R⁴C=CR⁴-, -C≡C-, Si(R⁴)₂, C=O, C=NR⁴, P(=O)R⁴, S=O, SO₂, -O-, -S- or -CONR⁴- and where one or more H atoms may be replaced by D, F, Cl, Br, I, CN or NO₂, or for P(R⁴), P(=O)(R⁴), N(Ar¹); or L is a single bond.

5. Compound according to one or more of Claims 1 to 4, selected from the compounds of the formulae (6) to (12), 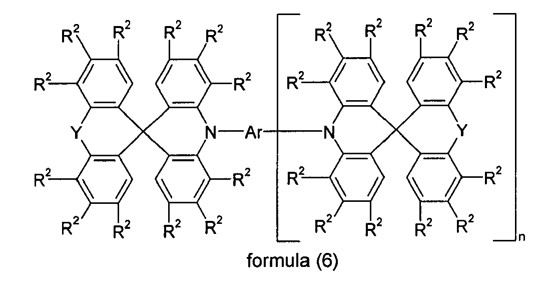 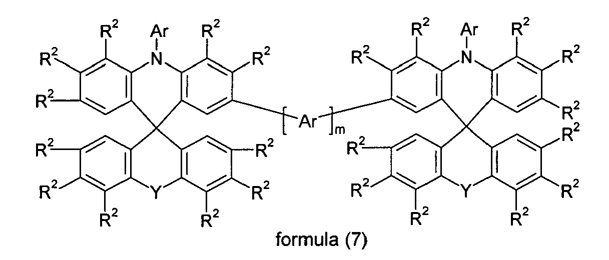 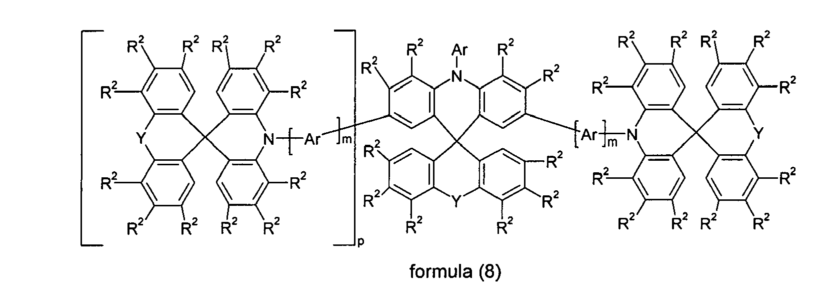 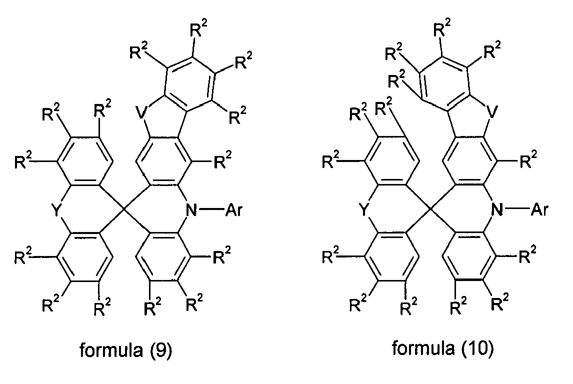 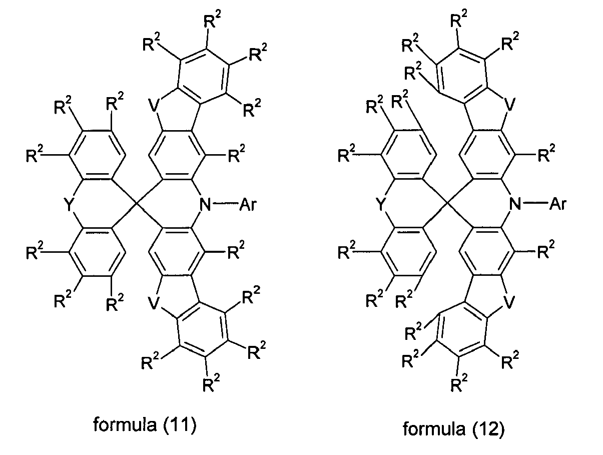 where the symbols and indices used have the meanings given in Claim 1 and Y preferably stands for O.

6. Compound according to one or more of Claims 1 to 5, selected from the compounds of the formulae (6a) to (12a), 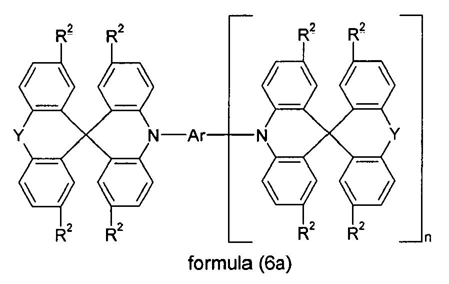 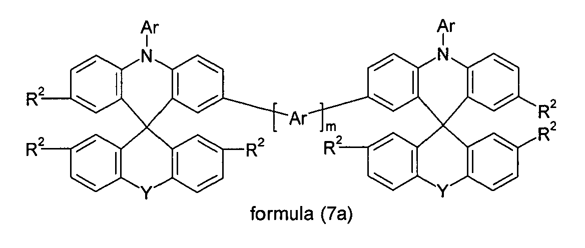 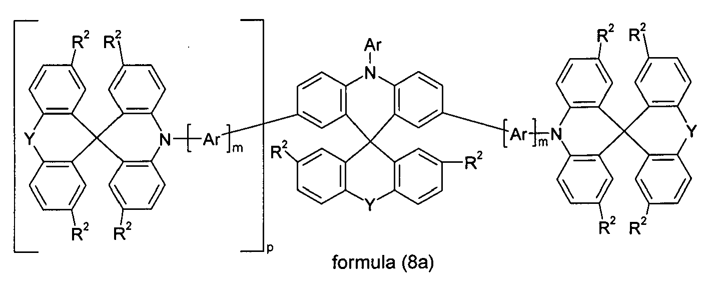 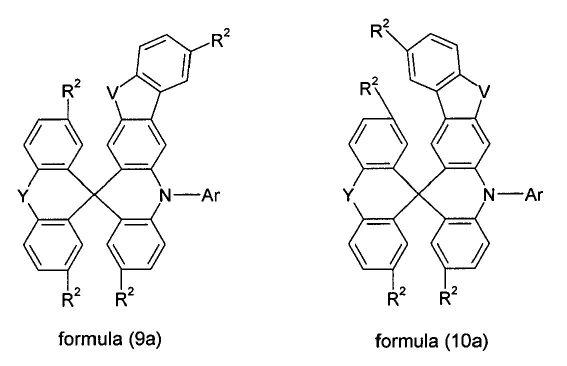 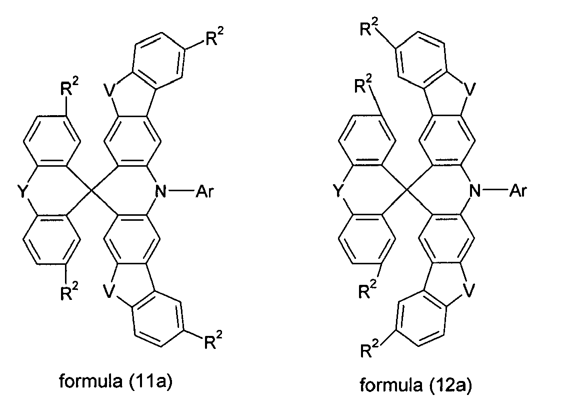 where the symbols and indices used have the meanings given in Claim 1 and Y preferably stands for O.

7. Compound according to one or more of Claims 1 to 6, **characterised in that** Ar is selected from the group consisting of aromatic or heteroaromatic ring systems having 5 to 24 aromatic ring atoms, which may in each case be substituted by one or more radicals R³, in particular benzene, ortho-, meta- or para-biphenyl, ortho-, meta-, para- or branched terphenyl, ortho-, meta-, para- or branched quaterphenyl, 1- or 2-naphthyl, pyrrole, furan, thiophene, indole, benzothiophene, benzofuran, carbazole, dibenzofuran, dibenzothiophene, pyridine, pyrimidine, pyrazine, pyridazine, triazine, anthracene, phenanthrene, pyrene, benzanthracene or combinations of two or three of these groups, each of which may be substituted by one or more radicals R³.

8. Compound according to one or more of Claims 1 to 7, **characterised in that** at least one substituent R¹, R² and/or R³ or a monovalent group Ar is selected from structures of the formulae (13) to (16) for R¹ to R³ or the formulae (13), (15) or (16) for Ar, 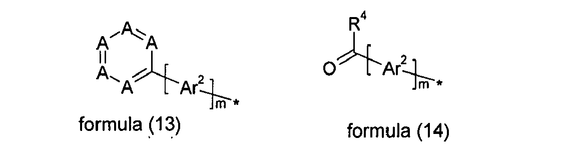 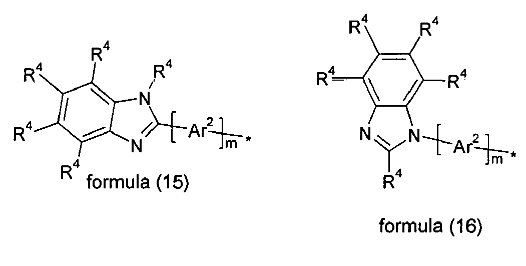 and/or at least one divalent or trivalent group Ar is selected from the groups of the formulae (17) to (19), 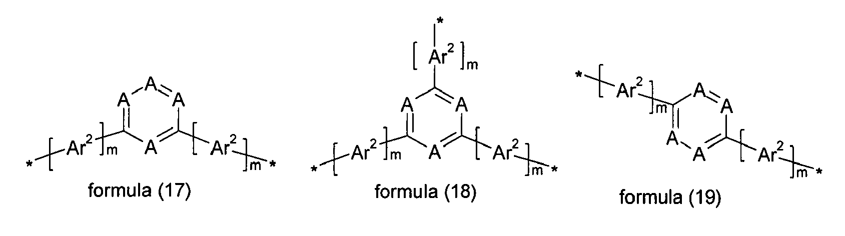 where R³, R⁴ and m have the meaning given in Claim 1, * indicates the position of the bonding of the group of the formulae (13) to (19) and furthermore:
A is on each occurrence, identically or differently, CR⁴ or N, with the proviso that one group A, two groups A or three groups A stand for N;
Ar² is, identically or differently on each occurrence, a divalent aromatic or heteroaromatic ring system having 5 to 16 C atoms, which may be substituted by one or more radicals R⁴;
and/or **in that** at least one of the radicals R¹, R², R³ or Ar is selected from the groups of the formulae (20) to (26), and/or at least one of the radicals Ar is selected from groups of the formulae (27) to (34), 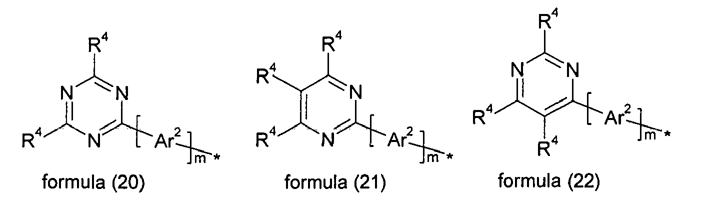 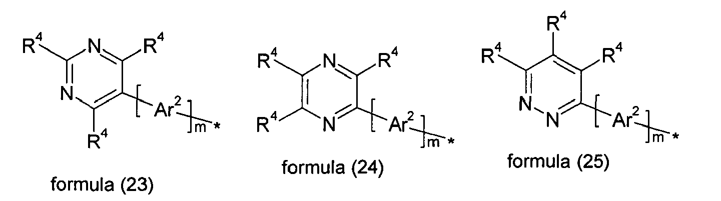 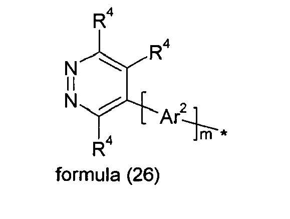 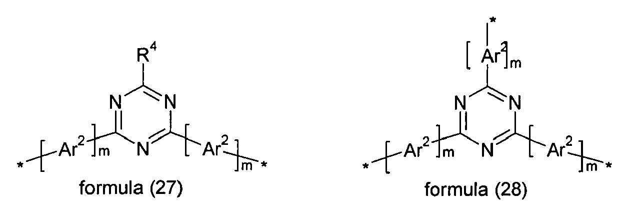 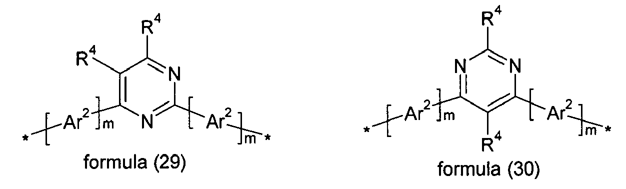 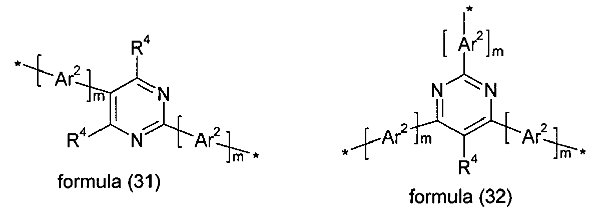 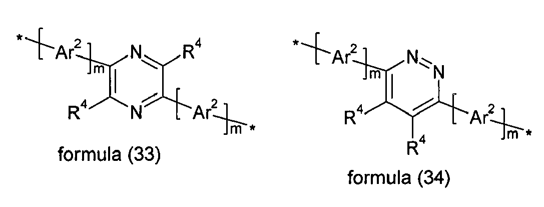 where the symbols and indices used have the meanings given in Claim 1 and above;
and/or **in that** at least one of the radicals R¹, R², R³ or Ar is selected from the groups of the formulae (35) to (49), 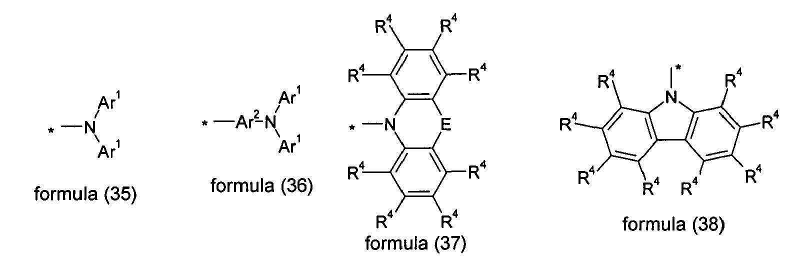 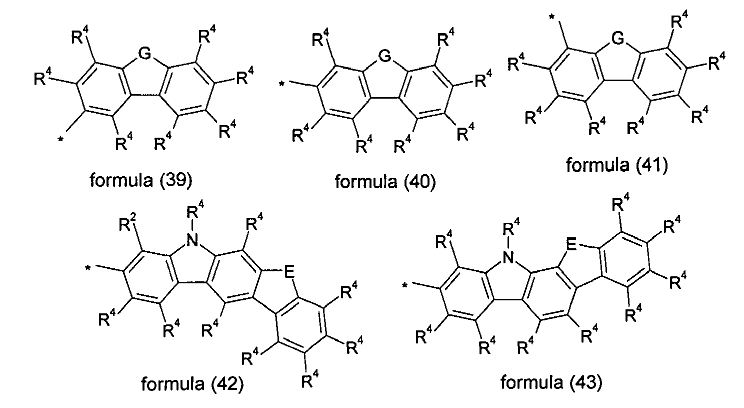 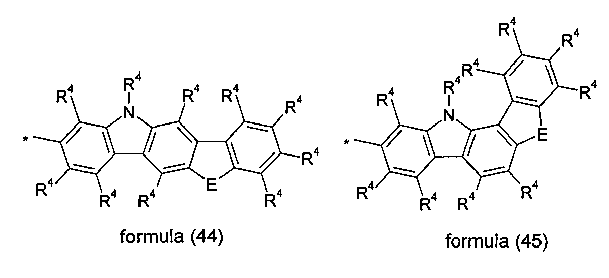 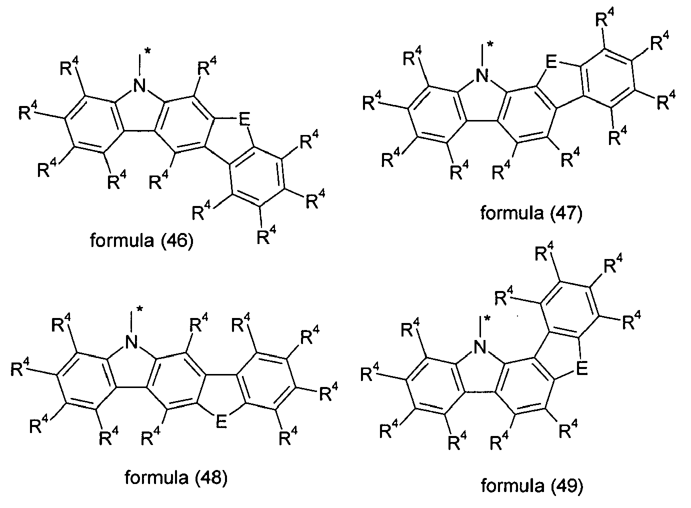 where the symbols used have the meanings given in Claim 1 and furthermore:
E is selected from the group consisting of C(R⁴)₂, NR⁴, O or S;
G is selected from the group consisting of NR⁴, O or S;
where R⁴ is to be replaced by R³ if the groups of the formulae (13), (15), (16), (20) to (26) and (35) to (49) stand for Ar and in the cases of the formulae (17) to (19) and (27) to (34).

9. Process for the preparation of a compound according to one or more of Claims 1 to 8, comprising the reaction steps:
a) build-up of the halogenated skeleton; and
b) introduction of substituents via a transition metal-catalysed coupling reaction.

10. Oligomer, polymer or dendrimer containing one or more compounds according to one or more of Claims 1 to 8, where one or more bonds are present from the compound to the polymer, oligomer or dendrimer.

11. Use of a compound according to one or more of Claims 1 to 8 or 10 in an electronic device, in particular in an organic electroluminescent device.

12. Electronic device comprising at least one compound according to one or more of Claims 1 to 8 or 10, where the electronic device is preferably selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and "organic plasmon emitting devices".

13. Electronic device according to Claim 12, where it is an organic electroluminescent device, **characterised in that** the compound according to one or more of Claims 1 to 8 or 10 is employed as matrix material for fluorescent or phosphorescent emitters and/or as fluorescent emitter and/or in an electron-blocking or exciton-blocking layer and/or in a hole-transport layer and/or in a hole-blocking layer and/or in an electron-transport layer and/or in an optical coupling-out layer.

## Revendications

1. Composé de la formule (1), (2) ou (3) : 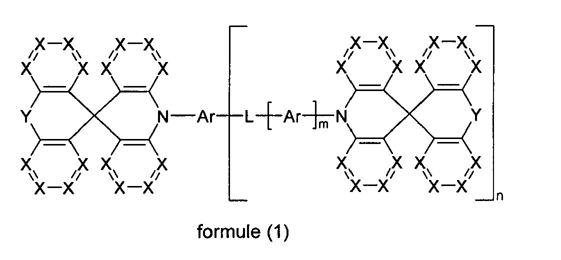 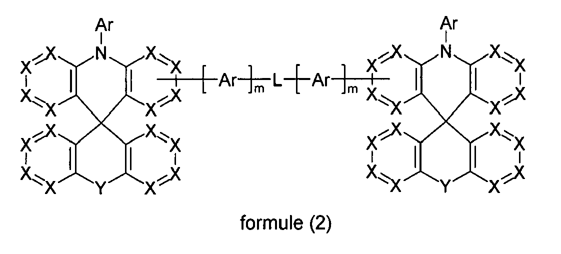 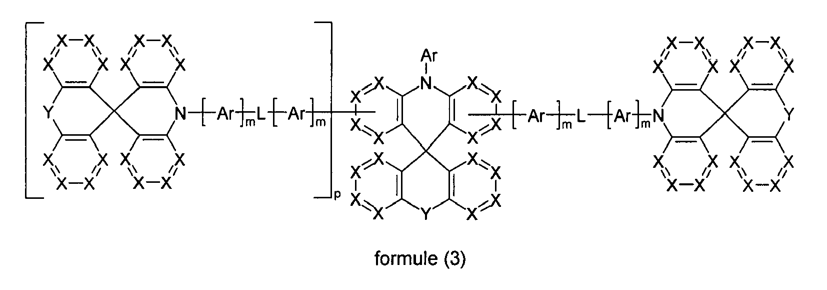 dans lesquelles ce qui suit s'applique aux symboles et indices utilisés :
Y est, pour chaque occurrence, de manière identique ou différente, O ou S;
X est, pour chaque occurrence, de manière identique ou différente, CR² ou N ; ou deux X adjacents représentent S, O ou NR², de telle sorte qu'un cycle à cinq éléments soit constitué ; ou deux X adjacents représentent un groupe de la formule (4) ou (5) qui suit : 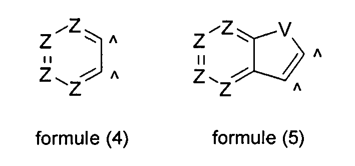 dans lesquelles ^ représente les groupes X adjacents correspondants dans la formule (1), (2) ou (3) ;
X représente ici C si un groupe Ar ou L est lié à ce groupe X dans la formule (2) ou (3) ;
V est, pour chaque occurrence, de manière identique ou différente, C(R²)₂, NR², O ou S ;
Z est, pour chaque occurrence, de manière identique ou différente, CR² ou N ;
Ar est, pour chaque occurrence, de manière identique ou différente, un système de cycle aromatique ou hétéroaromatique comportant 5 à 40 atomes de cycle aromatique, lequel peut être substitué par un radical ou plusieurs radicaux R³ ; le groupe Ar et le groupe X adjacent, lequel dans ce cas représente C, peuvent également être pontés l'un à l'autre au moyen d'une liaison simple ou d'un groupe divalent choisi parmi C(R³)₂, NR³, O ou S ;
L est, pour chaque occurrence, de manière identique ou différente, une liaison simple ou un groupe divalent;
R¹, R², R³ sont choisis, pour chaque occurrence, de manière identique ou différente, parmi le groupe constitué par H, D, F, Cl, Br, I, CN, NO₂, N(Ar¹)₂, N(R⁴)₂, C(=O)Ar¹, C(=O)R⁴, P(=O)(Ar¹)₂, un groupe alkyle, alcoxy ou thioalkyle en chaîne droite comportant 1 à 40 atome(s) de C ou un groupe alkyle, alcoxy ou thioalkyle ramifié ou cyclique comportant 3 à 40 atomes de C ou un groupe alkényle ou alkynyle comportant 2 à 40 atomes de C, dont chacun peut être substitué par un radical ou plusieurs radicaux R⁴, où un ou plusieurs groupe(s) CH₂ non adjacent(s) peut/peuvent être remplacé(s) par R⁴C=CR⁴, C≡C, Si(R⁴)₂, C=O, C=S, C=NR⁴, P(=O)(R⁴), SO, SO₂, NR⁴, O, S ou CONR⁴ et où un ou plusieurs atome(s) de H peut/peuvent être remplacé(s) par D, F, Cl, Br, I, CN ou NO₂, un système de cycle aromatique ou hétéroaromatique comportant 5 à 60 atomes de cycle aromatique, lequel peut dans chaque cas être substitué par un radical ou plusieurs radicaux R⁴, un groupe aryloxy ou hétéroaryloxy comportant 5 à 60 atomes de cycle aromatique, lequel peut être substitué par un radical ou plusieurs radicaux R⁴, où deux substituants R¹ ou R³ adjacents ou plus peuvent en option former un système de cycle aliphatique monocyclique ou polycyclique ou R² peut former un système de cycle aliphatique, aromatique ou hétéroaromatique monocyclique ou polycyclique, lequel peut être substitué par un radical ou plusieurs radicaux R⁴ ;
R⁴ est choisi, pour chaque occurrence, de manière identique ou différente, parmi le groupe constitué par H, D, F, Cl, Br, I, CN, NO₂, N(Ar¹)₂, N(R⁵)₂, C(=O)Ar¹, C(=O)R⁵, P(=O)(Ar¹)₂, un groupe alkyle, alcoxy ou thioalkyle en chaîne droite comportant 1 à 40 atome(s) de C ou un groupe alkyle, alcoxy ou thioalkyle ramifié ou cyclique comportant 3 à 40 atomes de C ou un groupe alkényle ou alkynyle comportant 2 à 40 atomes de C, dont chacun peut être substitué par un radical ou plusieurs radicaux R⁵, où un ou plusieurs groupe(s) CH₂ non adjacent(s) peut/peuvent être remplacé(s) par R⁵C=CR⁵, C≡C, Si(R⁵)₂, C=O, C=S, C=NR⁵, P(=O)(R⁵), SO, SO₂, NR⁵, O, S ou CONR⁵ et où un ou plusieurs atome(s) de H peut/ peuvent être remplacé(s) par D, F, Cl, Br, I, CN ou NO₂, un système de cycle aromatique ou hétéroaromatique comportant 5 à 60 atomes de cycle aromatique, lequel peut dans chaque cas être substitué par un radical ou plusieurs radicaux R⁵, un groupe aryloxy ou hétéroaryloxy comportant 5 à 60 atomes de cycle aromatique, lequel peut être substitué par un radical ou plusieurs radicaux R⁵, ou une combinaison de ces systèmes, où deux substituants R⁴ adjacents ou plus peuvent en option former un système de cycle aliphatique monocyclique ou polycyclique, lequel peut être substitué par un radical ou plusieurs radicaux R⁵ ;
Ar¹ est, pour chaque occurrence, de manière identique ou différente, un système de cycle aromatique ou hétéroaromatique comportant 5-30 atomes de cycle aromatique, lequel peut être substitué par un ou plusieurs radicaux non aromatiques R⁵ ; deux radicaux Ar¹ qui sont liés au même atome de N ou de P peuvent également être pontés l'un à l'autre ici au moyen d'une liaison simple ou d'un pont choisi parmi N(R⁵), C(R⁵)₂, O ou S ;
R⁵ est choisi, pour chaque occurrence, de manière identique ou différente, parmi le groupe constitué par H, D, F, CN, un radical hydrocarbone aliphatique comportant 1 à 20 atome(s) de C, un système de cycle aromatique ou hétéroaromatique comportant 5 à 30 atomes de cycle aromatique, où un ou plusieurs atome(s) de H peut/peuvent être remplacé(s) par D, F, Cl, Br, I ou CN, où deux substituants R³ adjacents ou plus peuvent former un système de cycle aliphatique monocyclique ou polycyclique l'un avec l'autre ou les uns avec les autres ;
m est, pour chaque occurrence, de manière identique ou différente, 0 ou 1 ;
n est 0, 1, 2, 3, 4 ou 5 ;
p est 0 ou 1.

2. Composé selon la revendication 1, **caractérisé en ce que** Y est égal à O.

3. Composé selon la revendication 1 ou 2, **caractérisé en ce que** X représente, de manière identique ou différente pour chaque occurrence, CR² ou N, où un maximum d'un seul groupe X par cycle représente N ; ou deux groupes X adjacents représentent un groupe de la formule (4) ou (5), en particulier de la formule (5), où Z représente, de manière identique ou différente pour chaque occurrence, CR² et V représente, de manière identique ou différente pour chaque occurrence, NR² ou C(R²)₂.

4. Composé selon une ou plusieurs des revendications 1 à 3, **caractérisé en ce que** L représente, de manière identique ou différente pour chaque occurrence, un groupe alkylène, alkylidène, alkylèneoxy ou thioalkylèneoxy en chaîne droite comportant 1 à 10 atome(s) de C ou un groupe alkylène, alkylidène, alkylèneoxy ou thioalkylèneoxy ramifié ou cyclique comportant 3 à 40 atomes de C ou un groupe alkénylène ou alkynylène comportant 2 à 40 atomes de C, lequel peut être substitué, dans chaque cas, par un radical ou plusieurs radicaux R⁴, où un ou plusieurs groupe(s) CH₂ non adjacent(s) peut/peuvent être remplacé(s) par -R⁴C=CR⁴-, -C≡C-, Si(R⁴)₂, C=O, C=NR⁴, P(=O)R⁴, S=O, SO₂, -O-, -S- ou -CONR⁴- et où un ou plusieurs atome(s) de H peut/ peuvent être remplacé(s) par D, F, Cl, Br, I, CN ou NO₂, ou P(R⁴), P(=O)(R⁴), N(Ar¹) ; ou L est une liaison simple.

5. Composé selon une ou plusieurs des revendications 1 à 4, choisi parmi les composés des formules (6) à (12) : 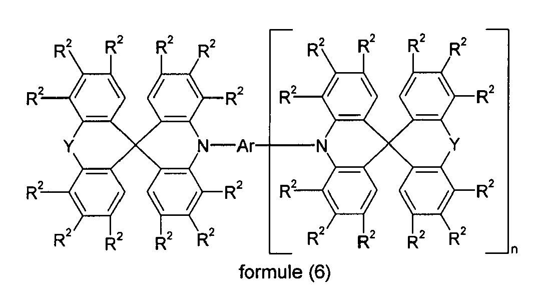 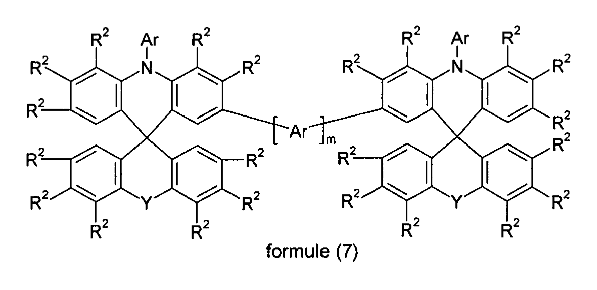 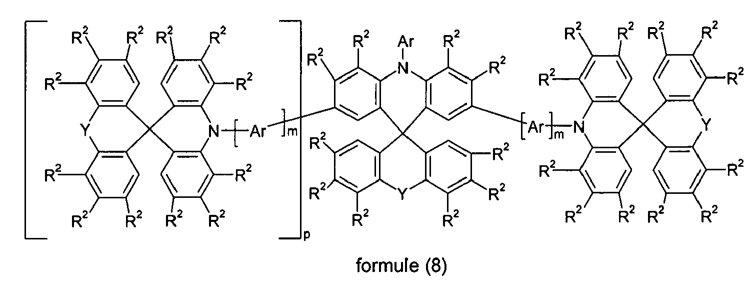 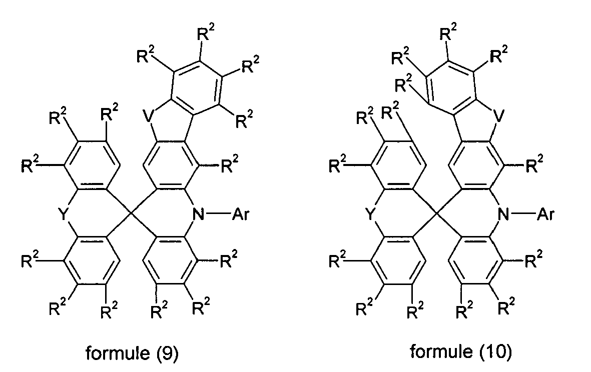 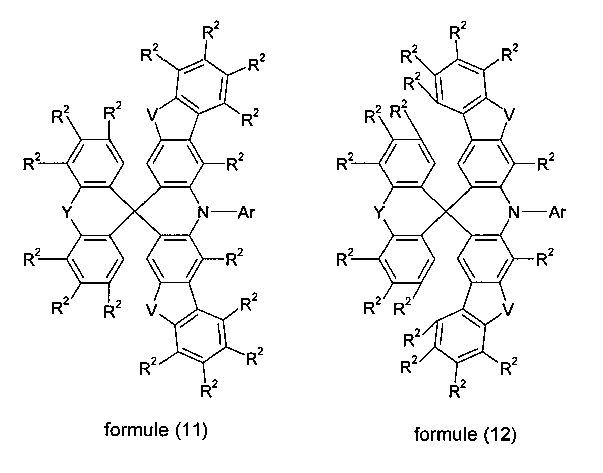 dans lesquelles les symboles et indices utilisés présentent les significations données selon la revendication 1 et Y représente de façon préférable O.

6. Composé selon une ou plusieurs des revendications 1 à 5, choisi parmi les composés des formules (6a) à (12a) : 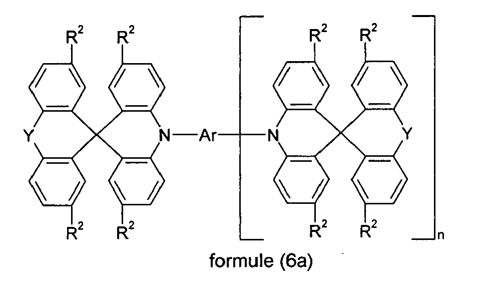 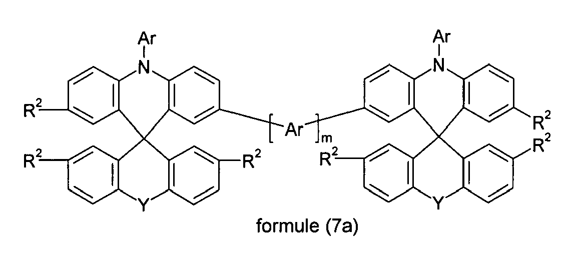 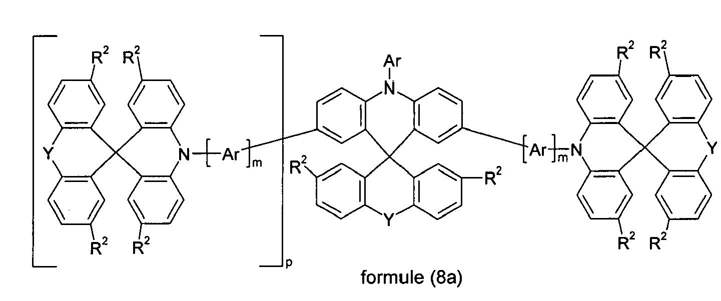 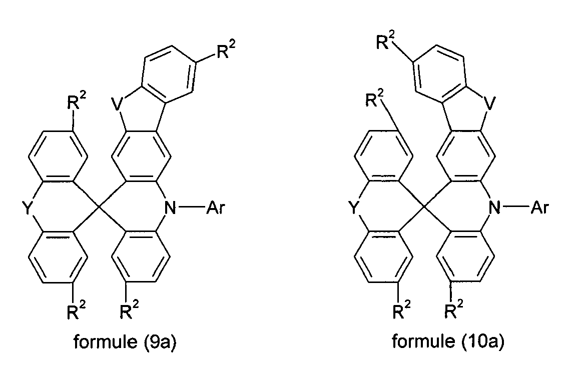 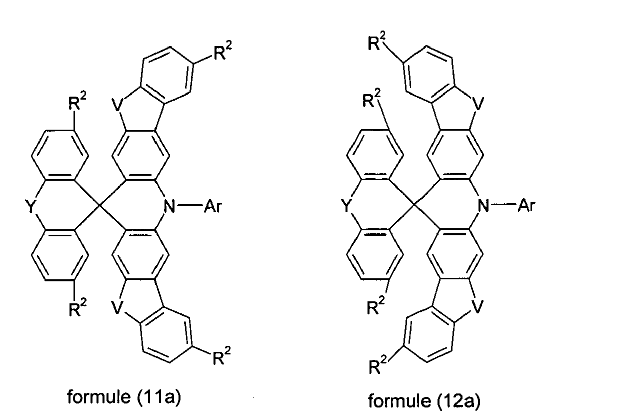 dans lesquelles les symboles et indices utilisés présentent les significations données selon la revendication 1 et Y représente de façon préférable O.

7. Composé selon une ou plusieurs des revendications 1 à 6, **caractérisé en ce que** Ar est choisi parmi le groupe constitué par des systèmes de cycle aromatique ou hétéroaromatique comportant 5 à 24 atomes de cycle aromatique, lesquels peuvent dans chaque cas être substitués par un radical ou plusieurs radicaux R³, en particulier benzène, ortho-, méta- ou para-biphényle, ortho-, méta-, para- ou terphényle ramifié, ortho-, méta-, para- ou quaterphényle ramifié, 1- ou 2-naphtyle, pyrrole, furan, thiophène, indole, benzothiophène, benzofuran, carbazole, dibenzofuran, dibenzothiophène, pyridine, pyrimidine, pyrazine, pyridazine, triazine, anthracène, phénanthrène, pyrène, benzanthracène ou des combinaisons de deux ou trois de ces groupes, dont chacun peut être substitué par un radical ou plusieurs radicaux R³.

8. Composé selon une ou plusieurs des revendications 1 à 7, **caractérisé en ce qu'**au moins un substituant R¹, R² et/ou R³ ou un groupe Ar monovalent est/sont choisi(s) parmi des structures des formules (13) à (16) pour R¹ à R³ ou des formules (13), (15) ou (16) pour Ar : 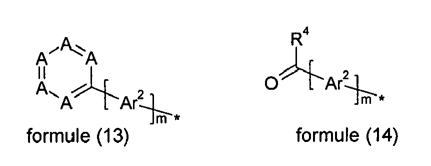 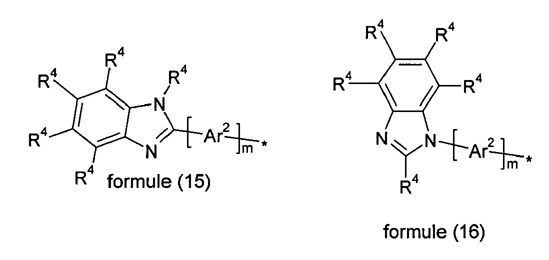 et/ou au moins un groupe divalent ou trivalent Ar est choisi parmi les groupes des formules (17) à (19) : 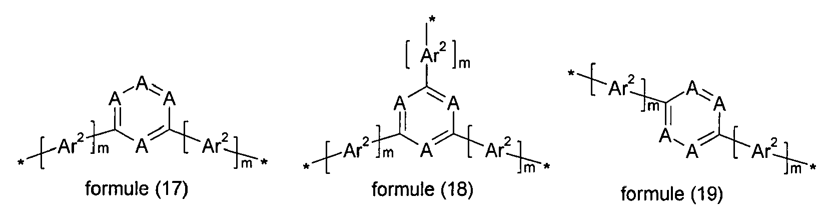 dans lesquelles R³, R⁴ et m présentent la signification donnée selon la revendication 1, * représente la position de la liaison du groupe des formules (13) à (19) et en outre :
A est, pour chaque occurrence, de manière identique ou différente, CR⁴ ou N, étant entendu qu'un seul groupe A, deux groupes A ou trois groupes A représente(nt) N ;
Ar² est, pour chaque occurrence, de manière identique ou différente, un système de cycle aromatique ou hétéroaromatique divalent comportant 5 à 16 atomes de C, lequel peut être substitué par un radical ou plusieurs radicaux R⁴ ;
et/ou **en ce qu'**au moins l'un des radicaux R¹, R², R³ ou Ar est choisi parmi les groupes des formules (20) à (26), et/ou au moins l'un des radicaux Ar est choisi parmi des groupes des formules (27) à (34) : 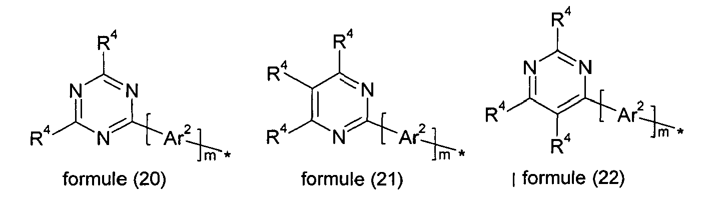 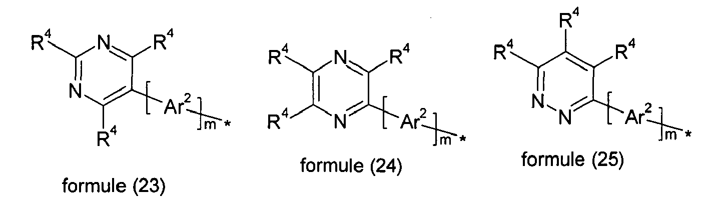 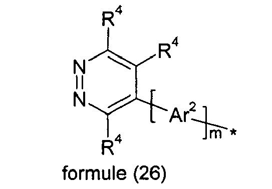 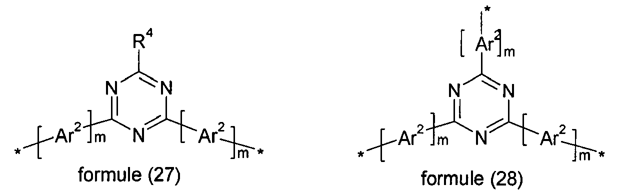 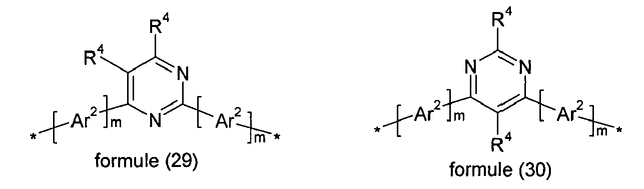 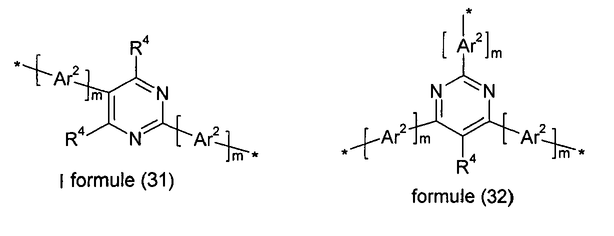 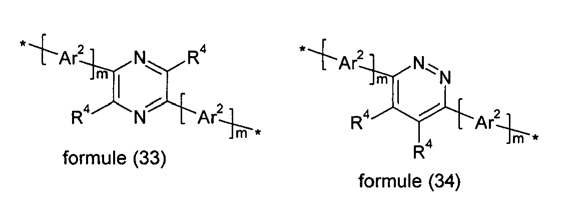 dans lesquelles les symboles et indices utilisés présentent les significations données selon la revendication 1 et ci avant ;
et/ou **en ce qu'**au moins l'un des radicaux R¹, R², R³ ou Ar est choisi parmi les groupes des formules (35) à (49) : 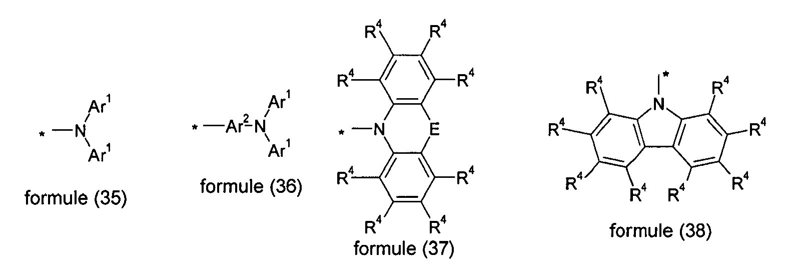 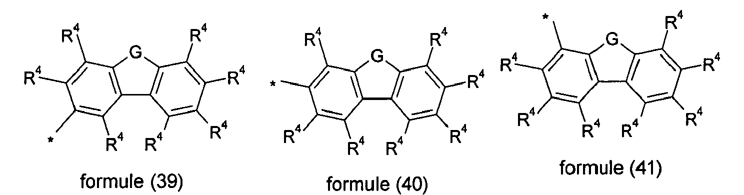 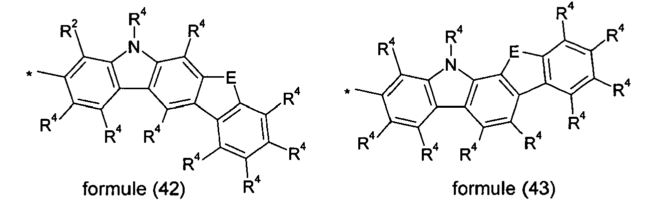 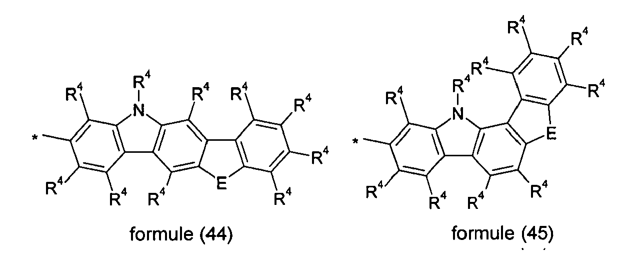 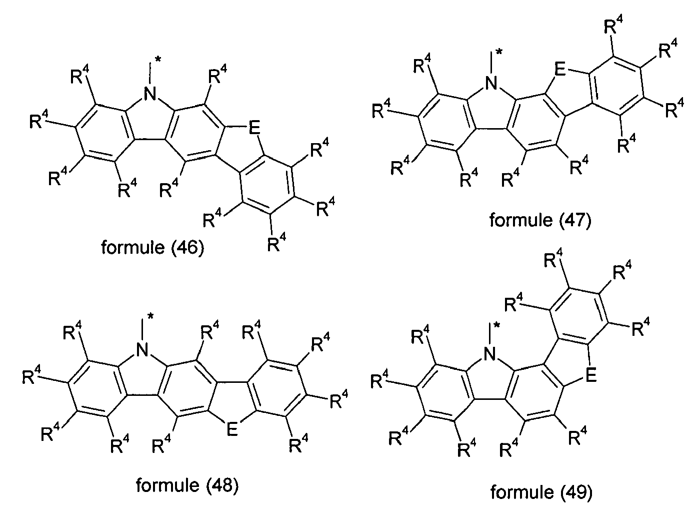 dans lesquelles les symboles utilisés présentent les significations données selon la revendication 1 et en outre :
E est choisi parmi le groupe constitué par C(R⁴)₂, NR⁴, O ou S ;
G est choisi parmi le groupe constitué par NR⁴, O ou S ;
où R⁴ doit être remplacé par R³ si les groupes des formules (13), (15), (16), (20) à (26) et (35) à (49) représentent Ar et dans les cas des formules (17) à (19) et (27) à (34).

9. Procédé pour la préparation d'un composé selon une ou plusieurs des revendications 1 à 8, comprenant les étapes de réaction qui suivent :
a) construction du squelette halogéné ; et
b) introduction de substituants via une réaction de couplage catalysée par métal de transition.

10. Oligomère, polymère ou dendrimère contenant un ou plusieurs composé(s) selon une ou plusieurs des revendications 1 à 8, dans lequel une ou plusieurs liaison(s) est/sont présente(s) depuis le composé jusqu'au polymère, jusqu'à l'oligomère ou jusqu'au dendrimère.

11. Utilisation d'un composé selon une ou plusieurs des revendications 1 à 8 ou 10 dans un dispositif électronique, en particulier dans un dispositif électroluminescent organique.

12. Dispositif électronique comprenant au moins un composé selon une ou plusieurs des revendications 1 à 8 ou 10, dans lequel le dispositif électronique est de façon préférable choisi parmi le groupe constitué par des dispositifs électroluminescents organiques, des circuits intégrés organiques, des transistors à effet de champ organiques, des transistors à film mince organiques, des transistors à émission de lumière organiques, des cellules solaires organiques, des cellules solaires organiques sensibilisées par colorant, des détecteurs optiques organiques, des photorécepteurs organiques, des dispositifs à extinction de champ organiques, des cellules électrochimiques à émission de lumière, des diodes laser organiques et des "dispositifs à émission de plasmons organiques".

13. Dispositif électronique selon la revendication 12, dans lequel il s'agit d'un dispositif électroluminescent organique, **caractérisé en ce que** le composé selon une ou plusieurs des revendications 1 à 8 ou 10 est utilisé en tant que matériau de matrice pour des émetteurs fluorescents ou phosphorescents et/ou en tant qu'émetteur fluorescent et/ou dans une couche de blocage d'électrons ou de blocage d'excitons et/ou dans une couche de transport de trous et/ou dans une couche de blocage de trous et/ou dans une couche de transport d'électrons et/ou dans une couche de couplage optique en sortie.
